# EUROPEAN PATENT APPLICATION

(11) **EP 3 991 754 A1**
(43) Date of publication of application: **04.05.2022**
(21) Application number: 20833007.6
(22) Date of filing: 05.06.2020
(51) Int. Cl.: A61K 47/68, A61P 35/00

(54) **ANTIBODY-DRUG CONJUGATE, INTERMEDIATE THEREOF, PREPARATION METHOD THEREFOR AND APPLICATION THEREOF**

(30) Priority: 28.06.2019 CN 201910577909
(71) Applicant: Shanghai Fudan-Zhangjiang Bio-Pharmaceutical Co., Ltd., Shanghai 201210 (CN)
(72) Inventor: BAO, Bin, Shanghai 201210 (CN); GUO, Qingsong, Shanghai 201210 (CN); GAO, Bei, Shanghai 201210 (CN); ZHANG, Yifan, Shanghai 201210 (CN); QIU, Xuefei, Shanghai 201210 (CN); YANG, Tong, Shanghai 201210 (CN); SHEN, Yijun, Shanghai 201210 (CN); ZHANG, Wenbo, Shanghai 201210 (CN); LV, Wei, Shanghai 200062 (CN); WANG, Lei, Shanghai 200062 (CN)
(74) Representative: Grünecker Patent- und Rechtsanwälte PartG mbB
(86) International application number: PCT/CN2020/094767
(87) International publication number: WO 2020/259258

(57) **Abstract**

Disclosed are an antibody-drug conjugate (ADC), an intermediate thereof, a preparation method therefor and an application thereof. The present invention provides an ADC. A structural general formula thereof is Ab-(L ₃-L ₂-L ₁-D)ₘ. The ADC has better biological activity, stability, and uniformity, has reduced toxic and side effects, and has a faster release rate of enzyme cutting in tumor cells. The use of the novel ADC can achieve a wide application of a cytotoxic drug particularly camptothecin in the field of ADCs in treating tumor patients resistant to microtubule ADC.

## Description

The present application claims the priority of the Chinese patent application CN2019105779096 filed on June 28, 2019. The entire disclosure of the above Chinese patent application is incorporated herein by reference in its entireties.

### Technical Field

The present disclosure relates to a field of biotechnology and medicine, especially relates to an antibody drug conjugate, an intermediate thereof, a preparation method therefor and an application thereof.

### Background

Antibody drug conjugate (ADC) has been one of the hot spots in the pharmaceutical industry in recent years. Due to the unsatisfactory clinical efficacy of many antibody drugs, many industry giants are increasingly turning their attention to ADC drugs. At present, seven ADC drugs have been approved for sale abroad. On May 17, 2000, the FDA approved the listing of Pfizer's Gemtuzumab Ozogamicin (trade name Mylotarg) for the treatment of acute myeloid leukemia (AML) patients who have relapsed for the first time, are over 60 years old, are CD33+, and are not suitable for cytotoxic chemotherapy. Gemtuzumab Ozogamicin was withdrawn from the market in 2010 but re-listed in 2017. In the same year, Pfizer's Inotuzumab ozogamicin (trade name Besponsa) was also approved by the FDA for the treatment of adult relapsed and refractory B-cell ALL. On August 19, 2011, the FDA approved the listing of Brentuximab Vedotin (trade name Adcetris) developed by Seattle Genetics for the treatment of CD30-positive Hodgkin's lymphoma (HL) and rare disease systemic anaplastic large cell lymphoma (SALCL). On February 22, 2013, the ado-trastuzumab emtansine (T-DM1, trade name Kadcyla) developed by Genentech was approved for sale by the FDA and is mainly used for the treatment of Her2-positive advanced (metastatic) breast cancer. Especially in 2019, polatuzumab vedotin (trade name Polivy), enfortumab vedotin (trade name Padcev) and fam-trastuzumabderuxtecan (trade name Enhertu) were approved for sale subsequently. In addition, there are more than 100 ADC drugs in the clinical and pre-clinical development stage internationally.

The basic modules of antibody drug conjugate include antibody, linker, and effector molecule. The antibody is used to transfer effector molecule to the tumor for enrichment, thereby killing tumor cells. Traditional effector molecules are mostly high-activity tubulin inhibitors, which usually have relatively large toxic and side effects, which limits the application of ADCs. Recently, Immunomedics company invented a new type of ADC drug IMMU-132 (ZL200980156218) with camptothecin compound as the effector molecule, which showed good anti-tumor effect. Daiichi Sankyo invented another ADC drug DS-8201a (ZL201380053256) with camptothecin compound as the effector molecule, which also showed good anti-tumor effects. In existing ADC technology, the linker used to connect the camptothecin compound and the antibody is seldom studied. Generally speaking, the ideal linker in ADC needs to meet the following requirements: first, ensure that the small molecule drug is not separated from the antibody in the plasma, after entering the cell, the linker will be broken under appropriate conditions to quickly release the active small molecule drug; secondly, the linker must have good physical and chemical properties so that it can be connected to the antibody to form a conjugate; and, the linker must be easy to prepare to lay the foundation for the large-scale production of ADC. IMMU-132 uses a pH-sensitive linker, which has poor stability. DS-8201a uses a tetrapeptide structure containing glycine-glycine-phenylalanine-glycine, compared with the general cathepsin B substrate sequence (such as valine-citrulline), the enzyme cleavage reaction is slow and there is poor physical and chemical properties and difficulty in synthesis.

### Content of the present invention

The technical problem to be solved in the present disclosure is for overcoming the defect of a single type of the existing antibody drug conjugate, and provide an antibody drug conjugate, an intermediate thereof, a preparation method therefor and an application thereof. The antibody drug conjugate can realize the wide application of cytotoxic drugs in the field of ADCs, and treat tumor patients who are resistant to microtubule ADCs.

The present disclosure provides antibody drug conjugates with a variety of specific structural linkers, the antibody drug conjugates inhibit the growth of mammalian tumors and can be used to treat a variety of cancers. The antibody drug conjugates have better biological activity, stability and uniformity, have reduced toxic and side effects, and faster release rate of enzyme cleavage in tumor cells.

The present disclosure solves the above technical problems through the following technical solutions:
The present disclosure provides an antibody drug conjugate, a general structural formula of the antibody drug conjugate is Ab-(L₃-L₂-L₁-D)ₘ;
wherein, Ab is an antibody;
D is a cytotoxic drug;
m is 2-8;
the structure of L₁ is as shown in formula I, II, III or IV, a-end of the L₁ is connected to the cytotoxic drug, and e-end of the L₁ is connected to c-end of the L₂; 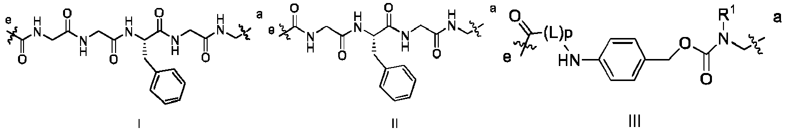 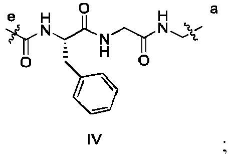
wherein L is independently phenylalanine residue, alanine residue, glycine residue, glutamic acid residue, aspartic acid residue, cysteine residue, histidine residue, isoleucine residue, leucine residue, lysine residue, methionine residue, proline residue, serine residue, threonine residue, tryptophan residue, tyrosine residue or valine residue; p is 2-4;
R¹ is C₁-C₆ alkyl substituted by -NR¹⁻¹R¹⁻², C₁-C₆ alkyl substituted by R¹⁻³ S(O)₂-, C₁-C₆ alkyl, C₃-C₁₀ cycloalkyl, C₆-C₁₄ aryl or 5 to 14-membered heteroaryl; the heteroatoms in the 5 to 14-membered heteroaryl are selected from one or more of N, O and S, and the number of heteroatoms is 1, 2, 3, or 4;
the R¹⁻¹, R¹⁻² and R¹⁻³ are independently C₁-C₆ alkyl; 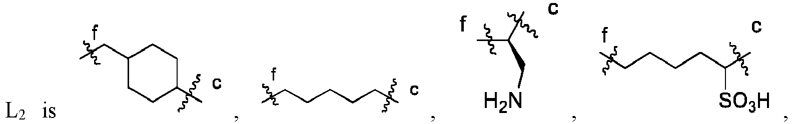 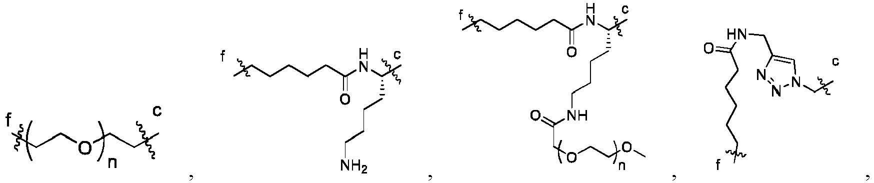 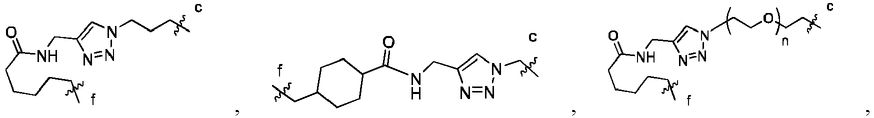 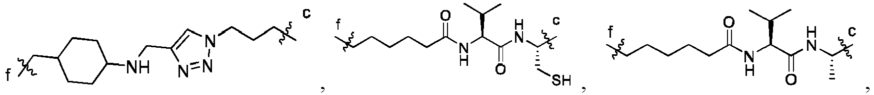 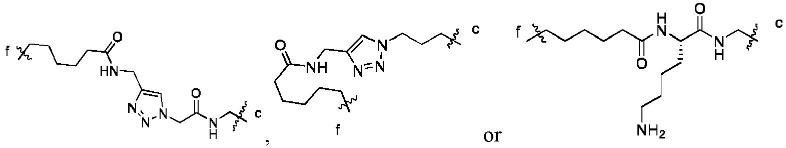 , wherein n is independently 1-12, c-end of the L₂ is connected to e-end of the L₁, f-end of the L₂ is connected to d-end of the L₃;
L₃ is 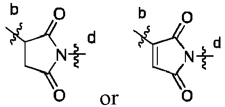 , wherein b-end of the L₃ is connected to the Ab, d-end of the L₃ is connected to f-end of the L₂;
when the structure of the L₁ is as shown in formula I, the L₃ is 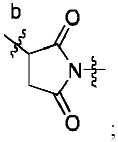 the L₂ is not

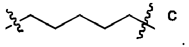

In a preferred embodiment of the present disclosure, in the antibody drug conjugates, some groups have the following definitions, and the definitions of unmentioned groups are as described in any of the above solutions (content of this paragraph is hereinafter referred to as "in a preferred embodiment of the present disclosure"):
the antibody can be a conventional antibody in the field of anti-tumor ADCs, preferably anti-HER2 antibody Trastuzumab or variant thereof, anti-B7-H3 antibody P2E5 or variant thereof, anti-Claudin18.2 antibody IMAB362 or variant thereof, or anti-Trop2 antibody RS7 or variant thereof, further preferably anti-HER2 antibody Trastuzumab or variant thereof, anti-B7-H3 antibody P2E5 or variant thereof, or anti-Claudin 18.2 antibody IMAB362 or variant thereof, further more preferably anti-HER2 antibody Trastuzumab or variant thereof, or anti-Claudin 18.2 antibody IMAB362 or variant thereof, and most preferably anti-HER2 antibody Trastuzumab or anti-Claudin 18.2 antibody IMAB362. The amino acid sequence of the light chain in the anti-HER2 antibody Trastuzumab is preferably shown in SEQ ID No. 5 in the sequence listing, and the amino acid sequence of the heavy chain in the anti-HER2 antibody Trastuzumab is preferably shown in SEQ ID No. 6 in the sequence listing. The amino acid sequence of the light chain in the anti-B7-H3 antibody P2E5 is preferably shown in SEQ ID No. 7 in the sequence listing, and the amino acid sequence of the heavy chain in the anti-B7-H3 antibody P2E5 is preferably shown in SEQ ID No. 8 in the sequence listing. The amino acid sequence of the light chain in the anti-Claudin 18.2 antibody IMAB362 is preferably shown in SEQ ID No. 1 in the sequence listing, and the amino acid sequence of the heavy chain in the anti-Claudin 18.2 antibody IMAB362 is preferably shown in SEQ ID No. 2 in the sequence listing. The amino acid sequence of the light chain in the anti-Trop2 antibody RS7 is preferably shown in SEQ ID No. 3 in the sequence listing, and the amino acid sequence of the heavy chain in the anti-Trop2 antibody RS7 is preferably shown in SEQ ID No. 4 in the sequence listing. The anti-HER2 antibody Trastuzumab variant has at least 70%, 75%, 80%, 85%, 90%, 95%, 98% or 99% homology compared with the anti-HER2 antibody Trastuzumab. The anti-B7-H3 antibody P2E5 variant has at least 70%, 75%, 80%, 85%, 90%, 95%, 98% or 99% homology compared with the anti-B7-H3 antibody P2E5. The anti-Trop2 antibody RS7 variant has at least 70%, 75%, 80%, 85%, 90%, 95%, 98% or99% homology compared with the anti-Trop2 antibody RS7. The anti-Claudin 18.2 antibody IMAB362 variant has at least 70%, 75%, 80%, 85%, 90%, 95%, 98% or 99% homology compared with the anti-Claudin 18.2 antibody IMAB362.

In a preferred embodiment of the present disclosure, b-end of the L₃ is preferably connected to the sulfhydryl in the antibody in the form of a thioether bond. Taking 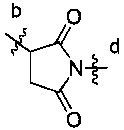 as an example, the connection form of 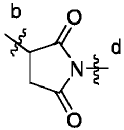 to the cysteine residue in the antibody is

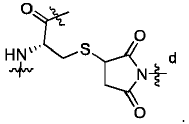

In a preferred embodiment of the present disclosure, the cytotoxic drug can be a conventional cytotoxic drug in the field of ADCs, particularly preferably a topoisomerase inhibitor containing a hydroxyl group, and more preferably a topoisomerase I inhibitor containing a hydroxyl group, further preferably camptothecin or derivatives thereof, and further more preferably

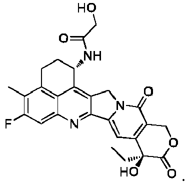

The L₁ is preferably connected to the hydroxyl group in the cytotoxic drug in the form of an ether bond. After the L₁ is connected to 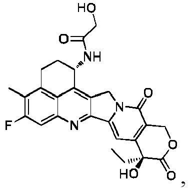 the fragment of the cytotoxic drug remaining in the antibody drug conjugate is preferably 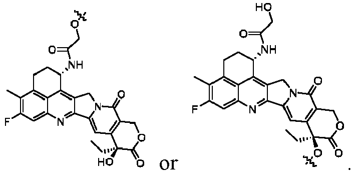 Taking 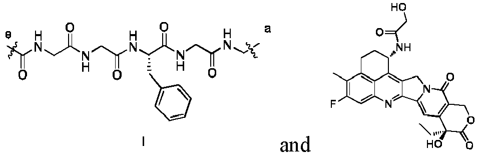 as examples, the -L₁-D can be

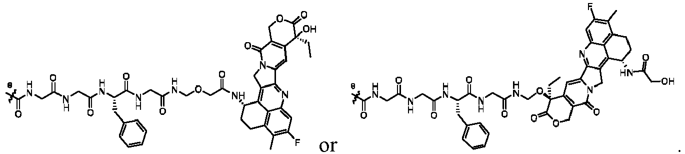

In a preferred embodiment of the present disclosure, when the R¹ is C₁-C₆ alkyl substituted by -NR¹⁻¹R¹⁻²; the C₁-C₆ alkyl is preferably C₁-C₄ alkyl, more preferably methyl, ethyl, *n*-propyl, isopropyl, *n*-butyl, isobutyl or *tert-butyl,* most preferably ethyl. The R¹⁻¹ and R¹⁻² are each independently preferably C₁-C₄ alkyl, more preferably methyl, ethyl, *n*-propyl, isopropyl, *n*-butyl, isobutyl or *tert-butyl,* most preferably methyl.

In a preferred embodiment of the present disclosure, when the R¹ is C₁-C₆ alkyl substituted by R¹⁻³S(O)₂-, the C₁-C₆ alkyl is preferably C₁-C₄ alkyl, more preferably methyl, ethyl, *n*-propyl, isopropyl, *n*-butyl, isobutyl or *tert-butyl,* most preferably ethyl. The R¹⁻³ is preferably C₁-C₄ alkyl, more preferably methyl, ethyl, *n*-propyl, isopropyl, *n*-butyl, isobutyl or *tert-butyl,* most preferably methyl.

In a preferred embodiment of the present disclosure, when the R¹ is C₁-C₆ alkyl; the C₁-C₆ alkyl is preferably C₁-C₄ alkyl, more preferably methyl, ethyl, *n*-propyl, isopropyl, *n*-butyl, isobutyl or *tert-butyl,* most preferably methyl or ethyl.

In a preferred embodiment of the present disclosure, the m is preferably 4-8, more preferably 7-8 (for example, 7.3, 7.4, 7.5, 7.6, 7.7, 7.8, 8.0).

In a preferred embodiment of the present disclosure, the L is preferably valine residue or alanine residue, and p is preferably 2. The (L)p is further preferably 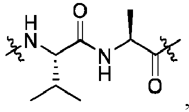 wherein the amino-end of the (L)p is connected to the carbonyl-end in the formula III.

In a preferred embodiment of the present disclosure, the n is preferably 8-12 (for example, 8 and 12).

In a preferred embodiment of the present disclosure, the R¹⁻¹, R¹⁻² and R¹⁻³ are independently preferably C₁-C₄ alkyl, more preferably methyl, ethyl, *n*-propyl, isopropyl, *n*-butyl, isobutyl or *tert-butyl,* most preferably methyl.

In a preferred embodiment of the present disclosure, the R¹ is preferably C₁-C₆ alkyl substituted by -NR¹⁻¹R¹⁻², C₁-C₆ alkyl substituted by R¹⁻³S(O)₂-, or C₁-C₆ alkyl, more preferably C₁-C₆ alkyl substituted by -NR¹⁻¹R¹⁻² or C₁-C₆ alkyl substituted by R¹⁻³S(O)₂-, most preferably C₁-C₆ alkyl substituted by R¹⁻³S(O)₂-. When R¹ is C₁-C₆ alkyl, the C₁-C₆ alkyl is preferably methyl or ethyl. The C₁-C₆ alkyl substituted by R¹⁻³S(O)₂- is preferably 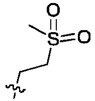 The C₁-C₆ alkyl substituted by -NR¹⁻¹R¹⁻² is preferably

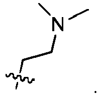

In a preferred embodiment of the present disclosure the 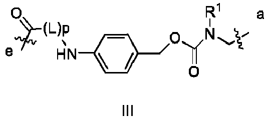 is preferably 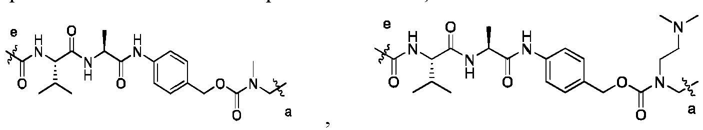 or

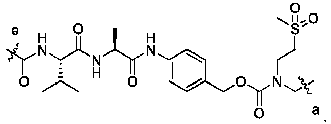

In a preferred embodiment of the present disclosure, the L₃ is preferably

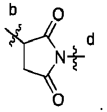

In a preferred embodiment of the present disclosure, when the structure of L₁ is as shown in formula I, the L₂ is preferably 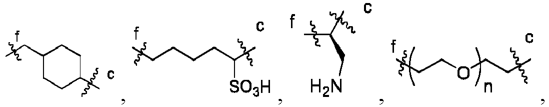 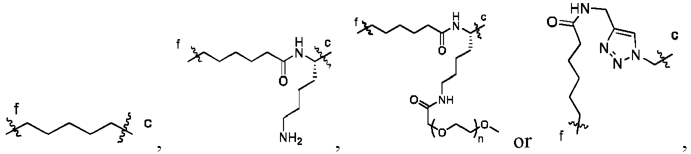 the L₃ is preferably

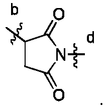

In a preferred embodiment of the present disclosure, when the structure of L₁ is as shown in formula II, the L₂ is preferably 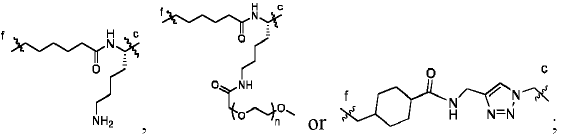 the L₃ is preferably

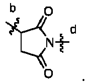

In a preferred embodiment of the present disclosure, when the structure of L₁ is as shown in formula III, the L₂ is preferably 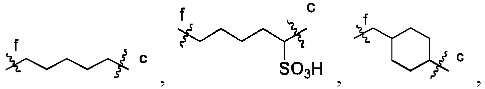 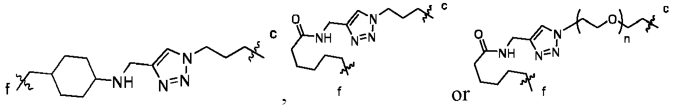 ; the L₃ is preferably

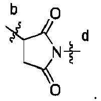

In a preferred embodiment of the present disclosure, when the structure of L₁ is as shown in formula IV, the L₂ is preferably 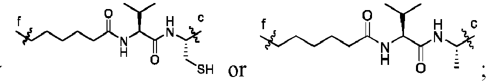 the L₃ is preferably

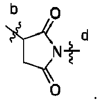

In a preferred embodiment of the present disclosure, the structure of L₁ is preferably as shown in formula I or III.

In a preferred embodiment of the present disclosure, when the structure of L₁ is as shown in formula I, the L₂ is preferably

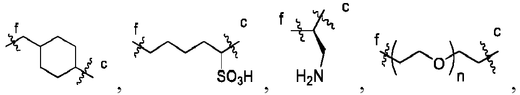

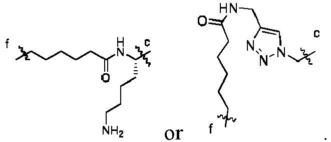

In a preferred embodiment of the present disclosure, when the structure of L₁ is as shown in formula I, the L₂ is preferably

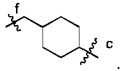

In a preferred embodiment of the present disclosure, when the structure of L₁ is as shown in formula III, L₂ is preferably

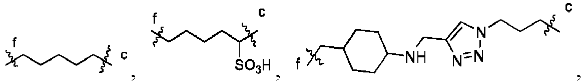

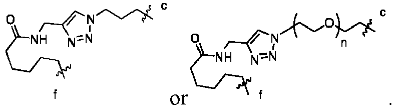

In a preferred embodiment of the present disclosure, when the structure of L₁ is as shown in formula III, L₂ is preferably

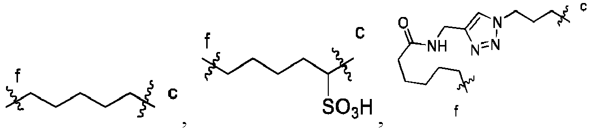

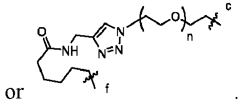

In a preferred embodiment of the present disclosure, when the structure of L₁ is as shown in formula III, L₂ is

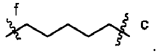

In a preferred embodiment of the present disclosure, when the structure of L₁ is as shown in formula III, L₁ is preferably

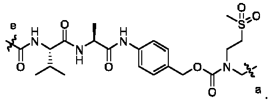

In a preferred embodiment of the present disclosure, in the antibody-drug conjugate, the Ab is anti-HER2 antibody Trastuzumab, anti-B7-H3 antibody P2E5 or variant thereof, or anti-Claudin 18.2 antibody IMAB362 or variant thereof; the D is a cytotoxic drug; the m is 2-8;
the structure of the L₁ is as shown in formula I, II, III or IV, 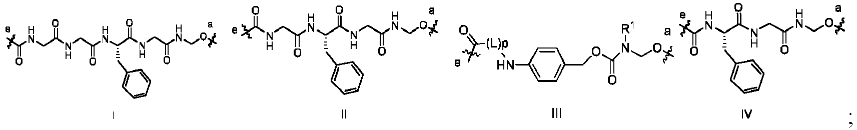
the L₂ is 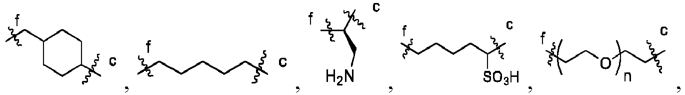 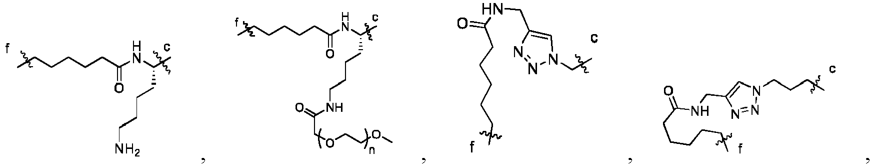 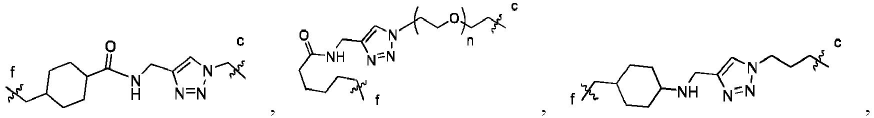 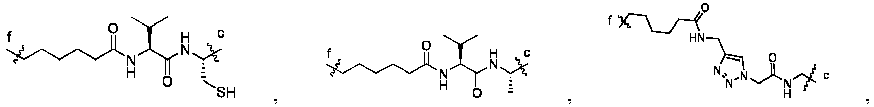 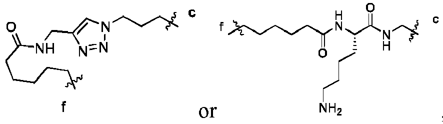 , the n is independently 8-12;
the L₃ is 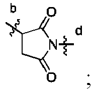
the L is independently valine residue or alanine residue; the p is 2 to 4;
the R¹ is C₁-C₆ alkyl substituted by -NR¹⁻¹R¹⁻², C₁-C₆ alkyl substituted by R¹⁻³S(O)₂-, or C₁-C₆ alkyl;
the R¹⁻¹, R¹⁻² and R¹⁻³ are each independently C₁-C₆ alkyl;
wherein, the amino acid sequence of the light chain in the anti-HER2 antibody Trastuzumab is preferably shown in SEQ ID No. 5 in the sequence listing, and the amino acid sequence of the heavy chain in the anti-HER2 antibody Trastuzumab is preferably shown in SEQ ID No. 6 in the sequence listing; the amino acid sequence of the light chain in the anti-B7-H3 antibody P2E5 is preferably shown in SEQ ID No. 7 in the sequence listing, and the amino acid sequence of the heavy chain in the anti-B7-H3 antibody P2E5 is preferably shown in SEQ ID No. 8 in the sequence listing; the amino acid sequence of the light chain in the anti-Claudin 18.2 antibody IMAB362 is preferably shown in SEQ ID No. 1 in the sequence listing, and the amino acid sequence of the heavy chain in the anti-Claudin 18.2 antibody IMAB362 is preferably shown in SEQ ID No. 2 in the sequence listing.

In a preferred embodiment of the present disclosure, in the antibody drug conjugate, the Ab is anti-HER2 antibody Trastuzumab, anti-B7-H3 antibody P2E5 or variant thereof, or anti-Claudin 18.2 antibody IMAB362 or variant thereof; the D is 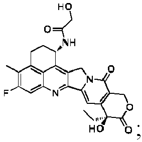 the m is 7-8;
the structure of the L₁ is as shown in formula I or III, 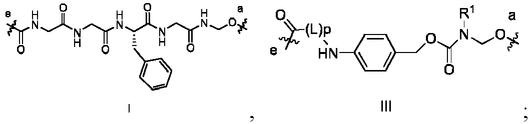
when the structure of the L₁ is as shown in formula I, the L₂ is 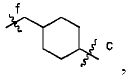 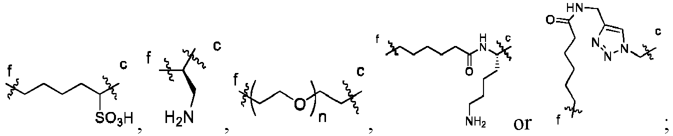 the n is independently 8-12;
when the structure of the L₁ is as shown in formula III, the L₂ is 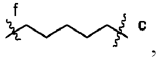 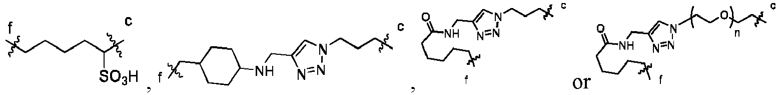 ; the n is independently 8-12;
the L₃ is 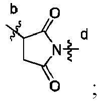
the L is independently valine residue or alanine residue; the p is 2 to 4;
the R¹ is C₁-C₄ alkyl substituted by -NR¹⁻¹R¹⁻², C₁-C₄ alkyl substituted by R¹⁻³S(O)₂-, or C₁-C₄ alkyl; the R¹⁻¹, R¹⁻² and R¹⁻³ are independently C₁-C₄ alkyl;
the amino acid sequence of the light chain in the anti-HER2 antibody Trastuzumab is preferably shown in SEQ ID No. 5 in the sequence listing, and the amino acid sequence of the heavy chain in the anti-HER2 antibody Trastuzumab is preferably shown in SEQ ID No. 6 in the sequence listing; the amino acid sequence of the light chain in the anti-B7-H3 antibody P2E5 is preferably shown in SEQ ID No. 7 in the sequence listing, and the amino acid sequence of the heavy chain in the anti-B7-H3 antibody P2E5 is preferably shown in SEQ ID No. 8 in the sequence listing; the amino acid sequence of the light chain in the anti-Claudin 18.2 antibody IMAB362 is preferably shown in SEQ ID No. 1 in the sequence listing, and the amino acid sequence of the heavy chain in the anti-Claudin 18.2 antibody IMAB362 is preferably shown in SEQ ID No. 2 in the sequence listing.

In a preferred embodiment of the present disclosure, wherein Ab is antibody; D is

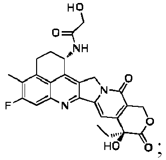

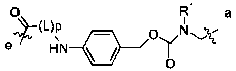

L₁ is III ; wherein, L is valine residue or alanine residue, p is 2, (L)p is preferably 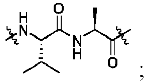 R¹ is C₁-C₆ alkyl substituted by -NR¹⁻¹R¹⁻², C₁-C₆ alkyl substituted by R¹⁻³S(O)₂-, or C₁-C₆ alkyl, preferably C₁-C₆ alkyl substituted by -NR¹⁻¹R¹⁻² or C₁-C₆ alkyl substituted by R¹⁻³ S(O)₂-, more preferably C₁-C₆ alkyl substituted by R¹⁻³S(O)₂-; the R¹⁻¹, R¹⁻² and R¹⁻³ are independently C₁-C₄ alkyl, preferably methyl; the C₁-C₆ alkyl substituted by -NR¹⁻¹R¹⁻² is preferably 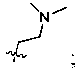 the C₁-C₆ alkyl substituted by R¹⁻³S(O)₂- is preferably

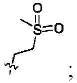

L₂ is 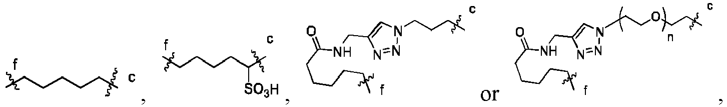 wherein, n is preferably 8; L₂ is preferably 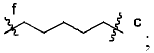 L₃ is

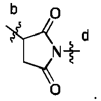

In a preferred embodiment of the present disclosure, the L₂ is

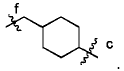

In a preferred embodiment of the present disclosure, the L₂ is

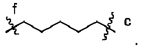

In a preferred embodiment of the present disclosure, the L₂ is

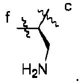

In a preferred embodiment of the present disclosure, the L₂ is

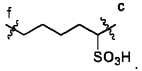

In a preferred embodiment of the present disclosure, the L₂ is

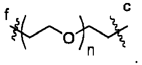

In a preferred embodiment of the present disclosure, the L₂ is

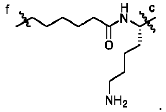

In a preferred embodiment of the present disclosure, the L₂ is

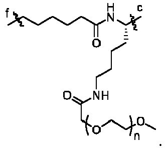

In a preferred embodiment of the present disclosure, the L₂ is

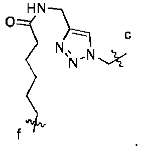

In a preferred embodiment of the present disclosure, the L₂ is

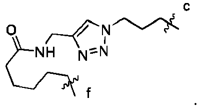

In a preferred embodiment of the present disclosure, the L₂ is

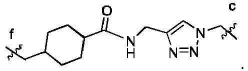

In a preferred embodiment of the present disclosure, the L₂ is

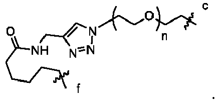

In a preferred embodiment of the present disclosure, the L₂ is

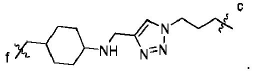

In a preferred embodiment of the present disclosure, the L₂ is

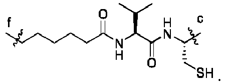

In a preferred embodiment of the present disclosure, the L₂ is

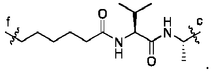

In a preferred embodiment of the present disclosure, the L₂ is

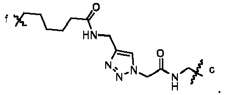

In a preferred embodiment of the present disclosure, the L₂ is

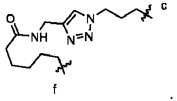

In a preferred embodiment of the present disclosure, the L₂ is

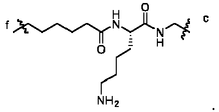

In a preferred embodiment of the present disclosure, the structure of the L₁ is as shown in formula III, the L₂ is

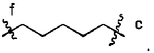

In a preferred embodiment of the present disclosure, the antibody drug conjugate is preferably any of the compounds shown below: 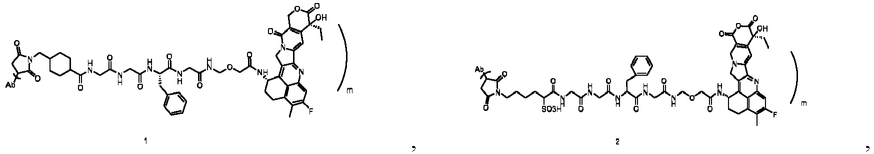 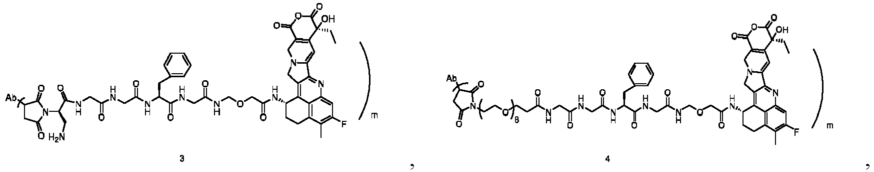 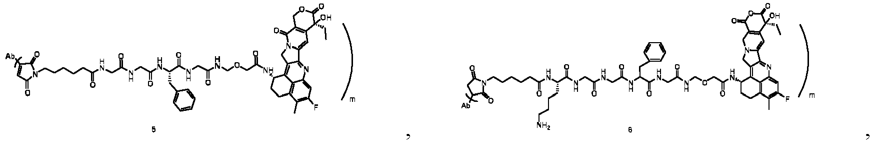 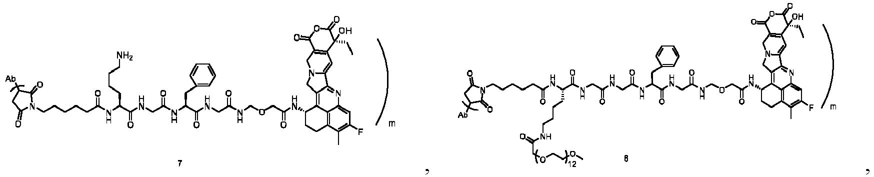 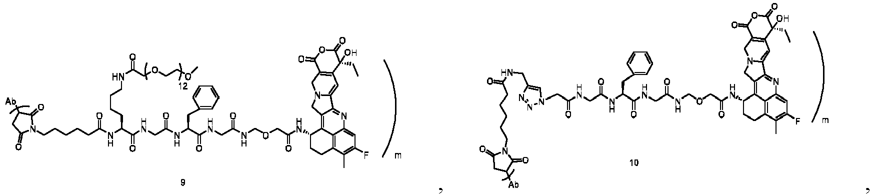 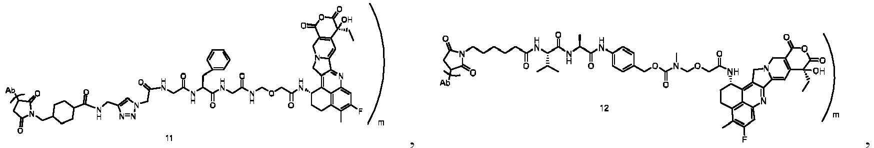 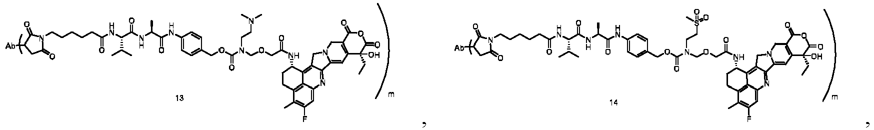 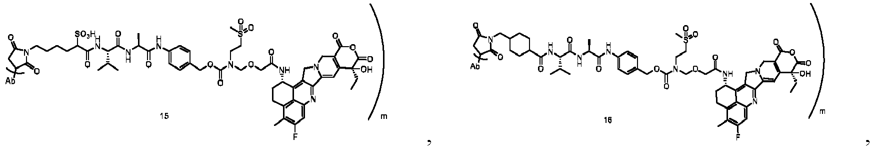 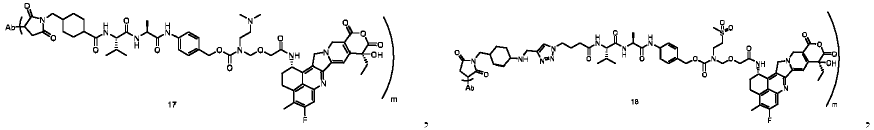 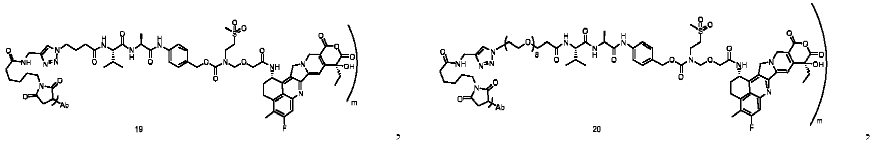 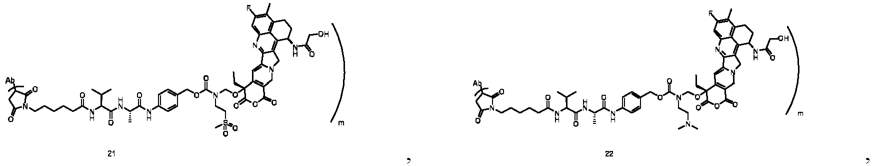 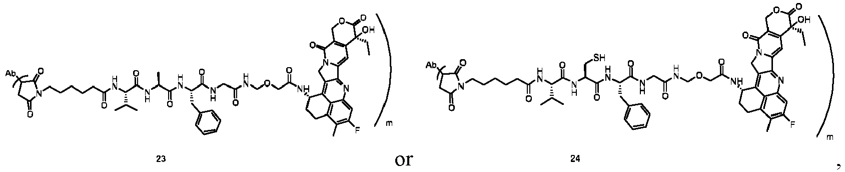
wherein, m is 2-8, preferably 7-8, for example 7.3, 7.4, 7.5, 7.6, 7.7, 7.8 or 8.0;
Ab is anti-HER2 antibody Trastuzumab, anti-B7-H3 antibody P2E5 or anti-Claudin 18.2 antibody IMAB362; the amino acid sequence of the light chain in the Ab is shown in SEQ ID No. 5 in the sequence listing, and the amino acid sequence of the heavy chain in the Ab is shown in SEQ ID No. 6 in the sequence listing; the amino acid sequence of the light chain in the anti-B7-H3 antibody P2E5 is shown in SEQ ID No. 7 in the sequence listing, and the amino acid sequence of the heavy chain in the anti-B7-H3 antibody P2E5 is shown in SEQ ID No. 8 in the sequence listing; the amino acid sequence of the light chain in the anti-Claudin 18.2 antibody IMAB362 is shown in SEQ ID No. 1 in the sequence listing, and the amino acid sequence of the heavy chain in the anti-Claudin 18.2 antibody IMAB362 is shown in SEQ ID No. 2 in the sequence listing.

In a preferred embodiment of the present disclosure, the antibody drug conjugate is preferably any of the compounds shown below: 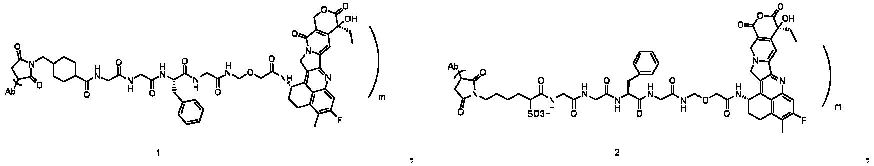 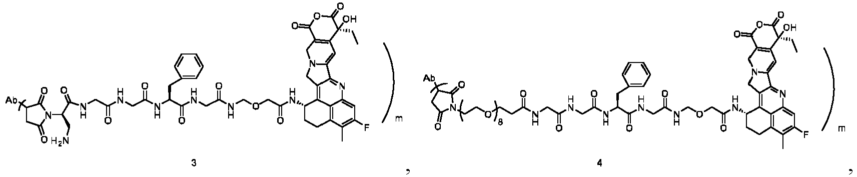 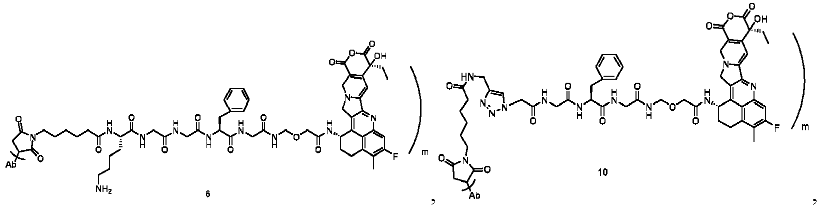 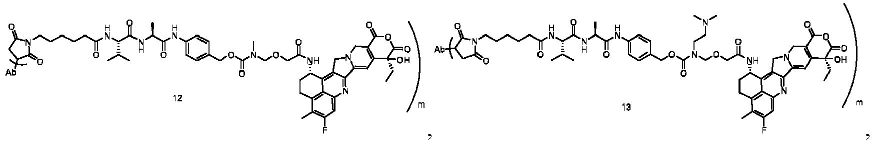 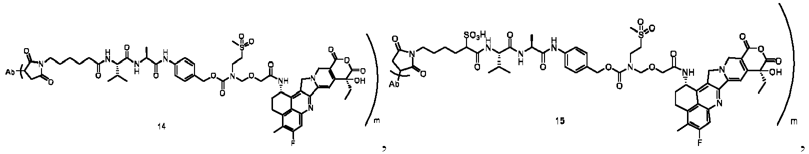 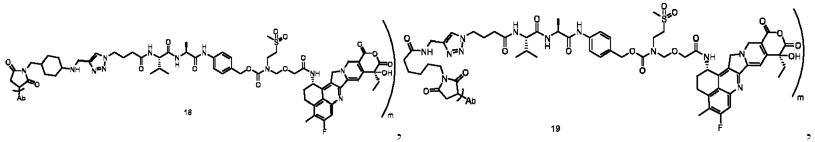 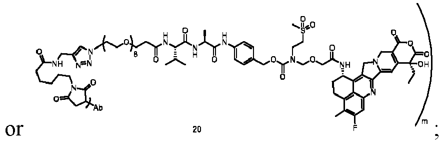 wherein, Ab is anti-HER2 antibody Trastuzumab; or, the amino acid sequence of the light chain in the Ab is shown in SEQ ID No. 5 in the sequence listing, and the amino acid sequence of the heavy chain in the Ab is shown in SEQ ID No. 6 in the sequence listing; wherein, m is 2-8, preferably 7-8, for example 7.3, 7.4, 7.5, 7.6, 7.7, 7.8 or 8.0.

In a preferred embodiment of the present disclosure, the antibody drug conjugate is preferably any of the compounds shown below: 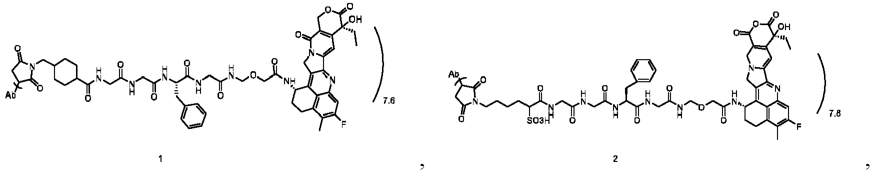 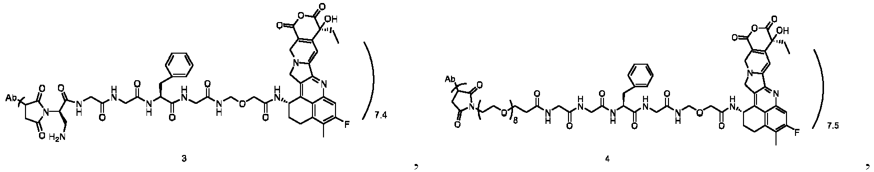 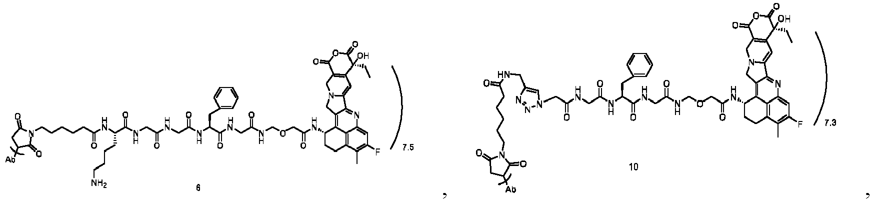 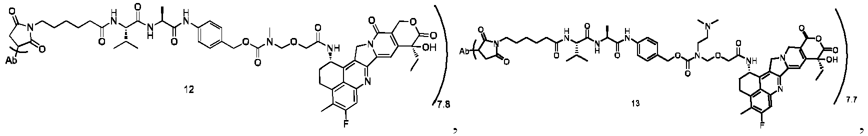 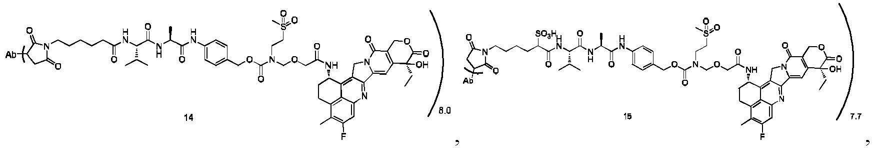 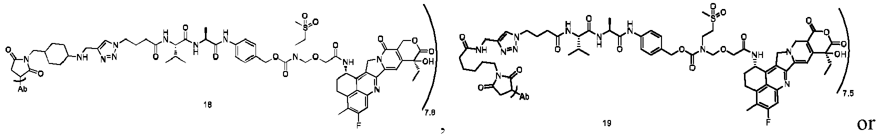 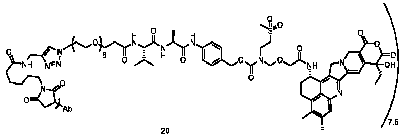 , preferably 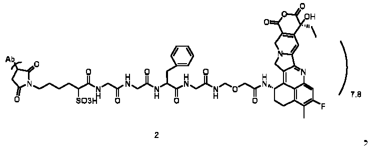 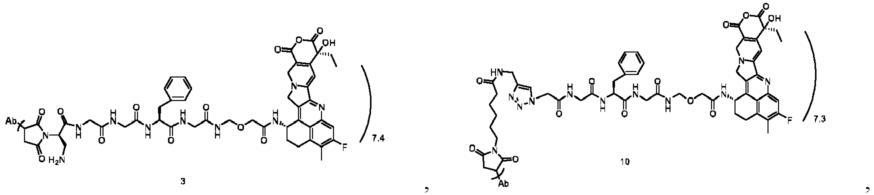 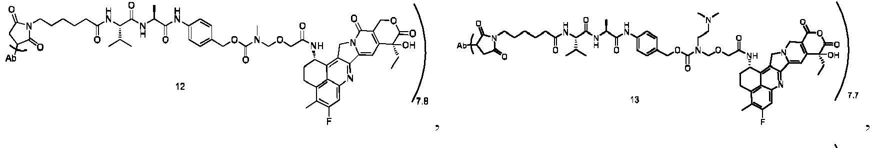 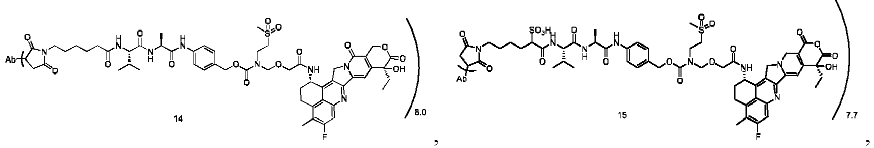 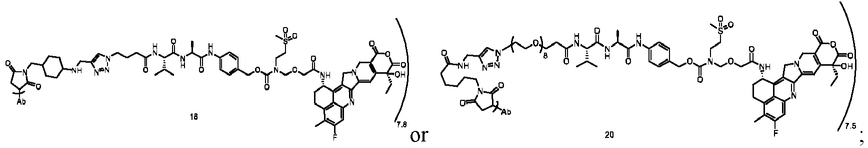 wherein, Ab is anti-HER2 antibody Trastuzumab; or, the amino acid sequence of the light chain in the Ab is shown in SEQ ID No. 5 in the sequence listing, and the amino acid sequence of the heavy chain in the Ab is shown in SEQ ID No. 6 in the sequence listing.

In a preferred embodiment of the present disclosure, the antibody drug conjugate is preferably any of the compounds shown below: 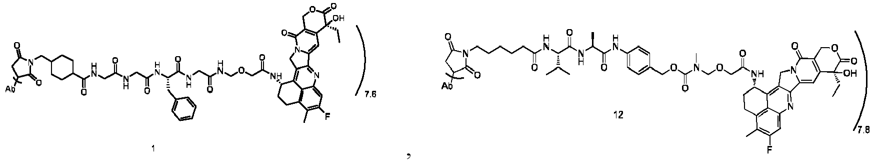 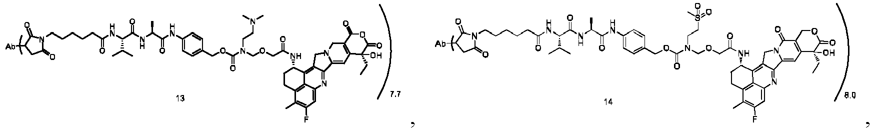 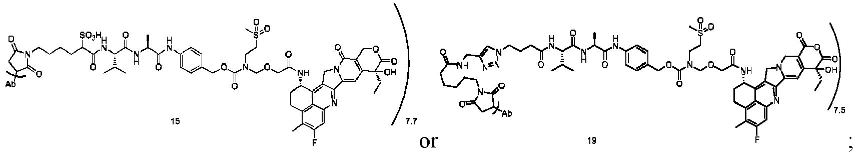 wherein, Ab is anti-HER2 antibody Trastuzumab; or, the amino acid sequence of the light chain in the Ab is shown in SEQ ID No. 5 in the sequence listing, and the amino acid sequence of the heavy chain in the Ab is shown in SEQ ID No. 6 in the sequence listing.

In a preferred embodiment of the present disclosure, the antibody drug conjugate is preferably any of the compounds shown below: 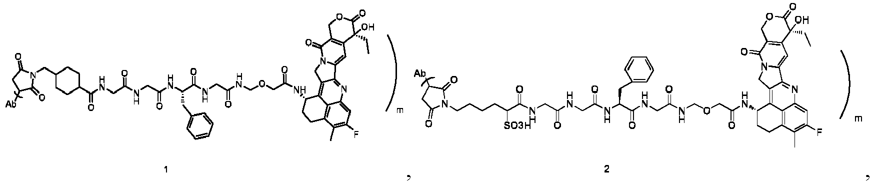 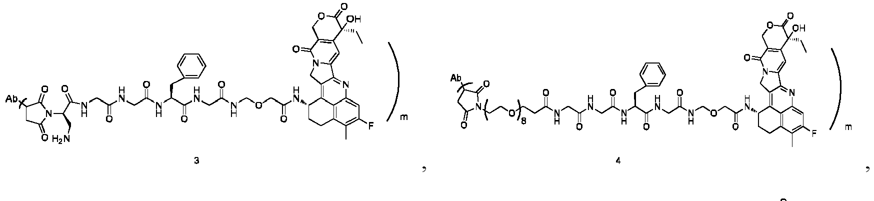 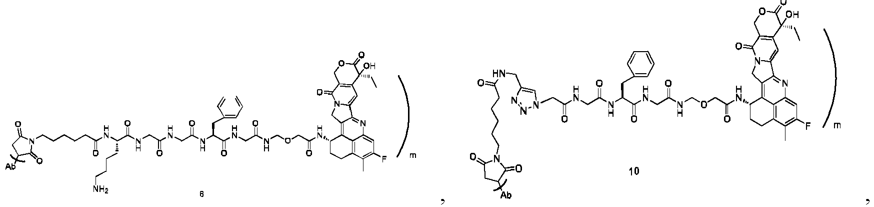 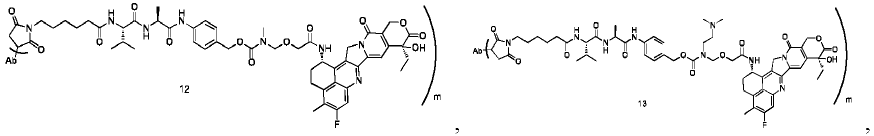 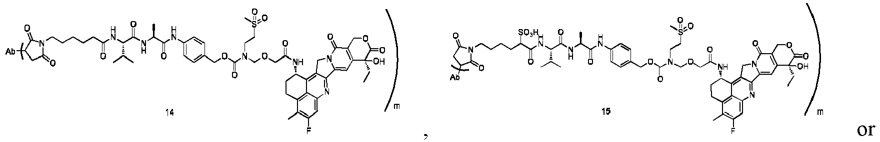 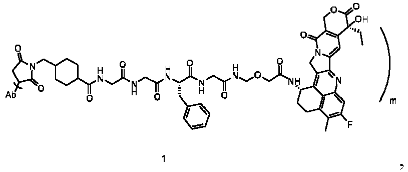 wherein, Ab is anti-B7-H3 antibody P2E5; or, the amino acid sequence of the light chain in the Ab is shown in SEQ ID No. 7 in the sequence listing, and the amino acid sequence of the heavy chain in the Ab is shown in SEQ ID No. 8 in the sequence listing, wherein, m is 2-8, preferably 7-8, for example 7.3, 7.4, 7.5, 7.6, 7.7, 7.8 or 8.0.

In a preferred embodiment of the present disclosure, the antibody drug conjugate is preferably any of the compounds shown below: 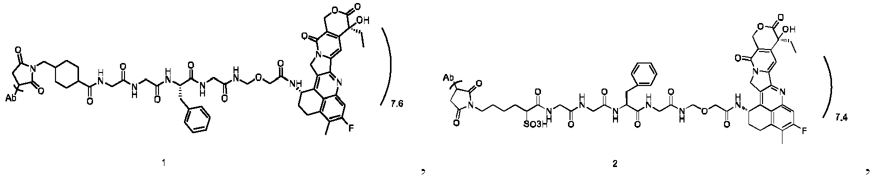 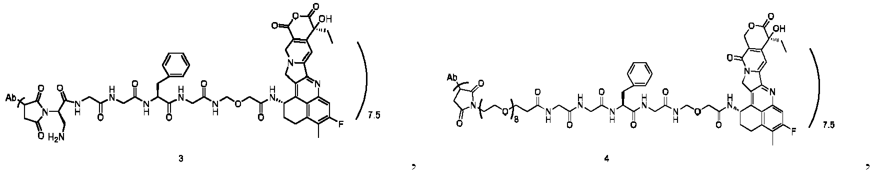 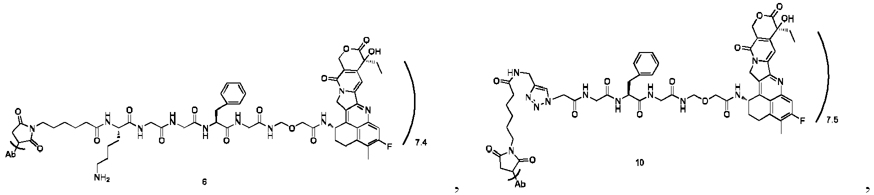 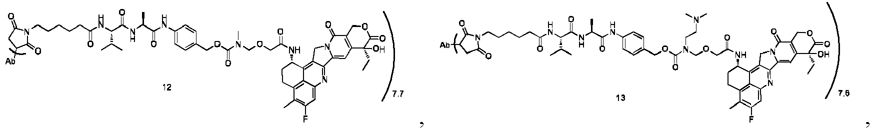 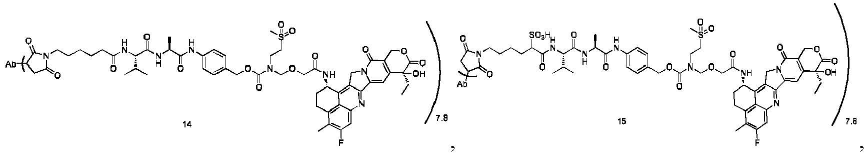 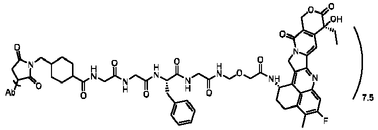 or ;
wherein, Ab is anti-B7-H3 antibody P2E5; or, the amino acid sequence of the light chain in the Ab is shown in SEQ ID No. 7 in the sequence listing, and the amino acid sequence of the heavy chain in the Ab is shown in SEQ ID No. 8 in the sequence listing.

In a preferred embodiment of the present disclosure, the antibody drug conjugate is preferably any of the compounds shown below: 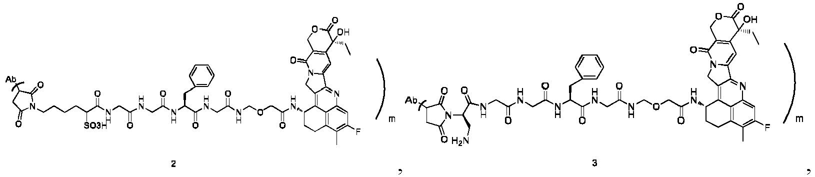 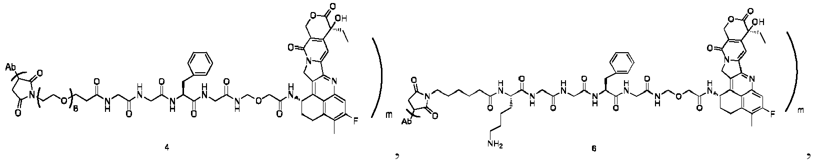 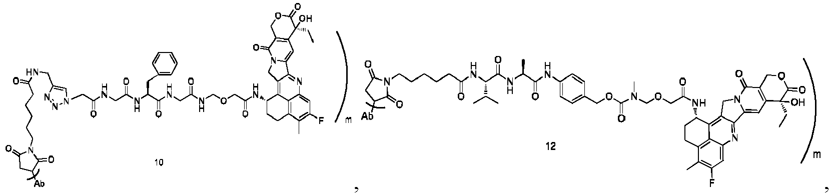 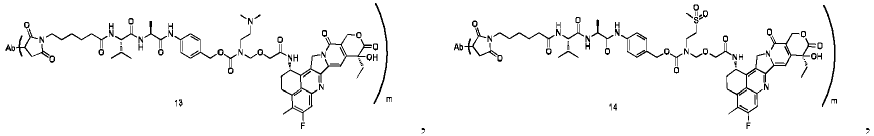 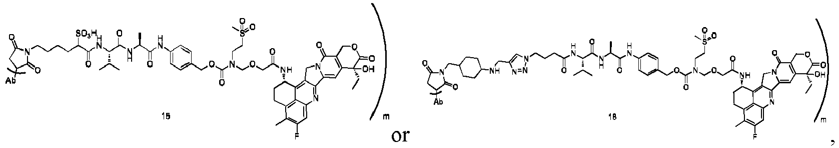 wherein, Ab is anti-Claudin18.2 antibody IMAB362; or, the amino acid sequence of the light chain in the Ab is shown in SEQ ID No. 1 in the sequence listing, and the amino acid sequence of the heavy chain in the Ab is shown in SEQ ID No. 2 in the sequence listing; wherein, m is 2-8, preferably 7-8, for example 7.3, 7.4, 7.5, 7.6, 7.7, 7.8 or 8.0.

In a preferred embodiment of the present disclosure, the antibody drug conjugate is preferably any of the compounds shown below: 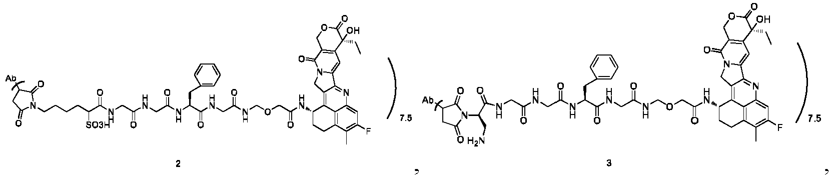 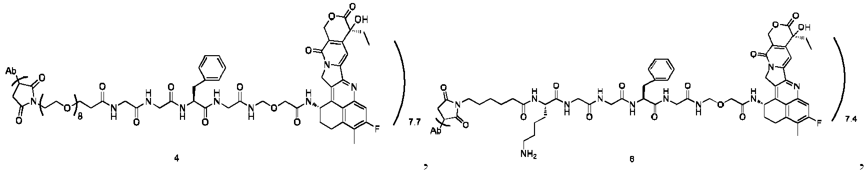 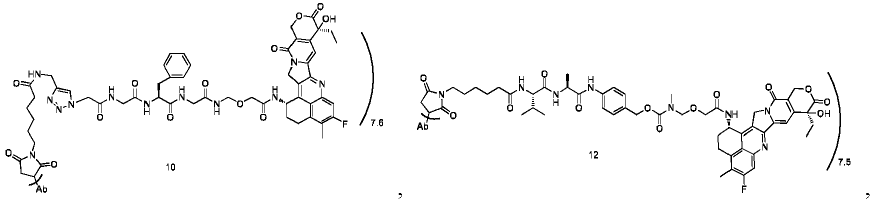 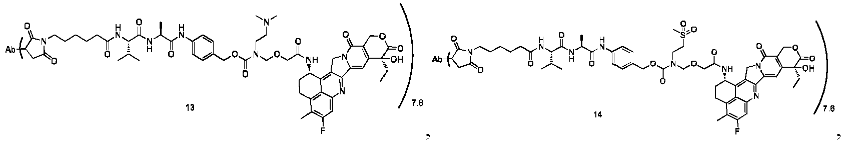 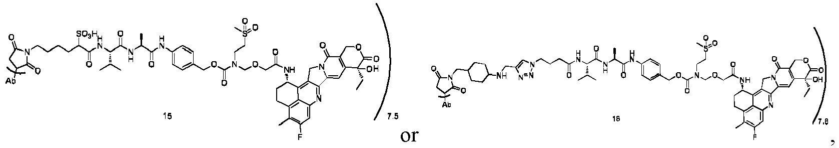 wherein, Ab is anti-Claudin18.2 antibody IMAB362; or, the amino acid sequence of the light chain in the Ab is shown in SEQ ID No. 1 in the sequence listing, and the amino acid sequence of the heavy chain in the Ab is shown in SEQ ID No. 2 in the sequence listing.

In a preferred embodiment of the present disclosure, the antibody drug conjugate is preferably any of the compounds shown below: 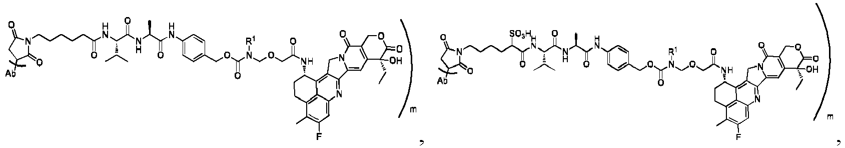 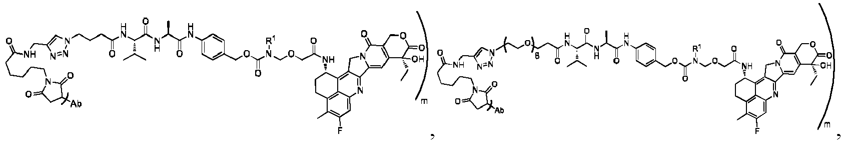 wherein, Ab, m and R¹ are as defined above.

The present disclosure also provides a linker-drug conjugate, a general structural formula of the linker-drug conjugate is L₄-L₂-L₁-D; wherein L₄ is 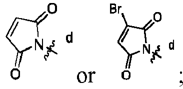 L₂, L₁, and D are as defined above, f-end of the L₂ is connected to d-end of the L₄; when the L₄ is 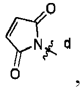 when the L₁ 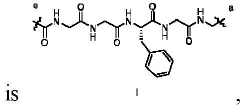 the L₂ is not

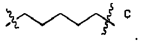

In a preferred embodiment of the present disclosure, the linker-drug conjugate is preferably any of the compounds shown below: 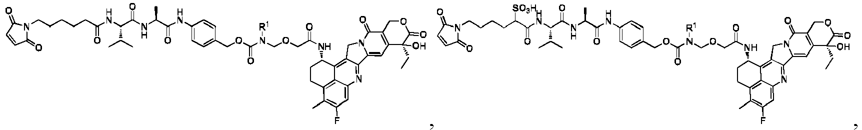 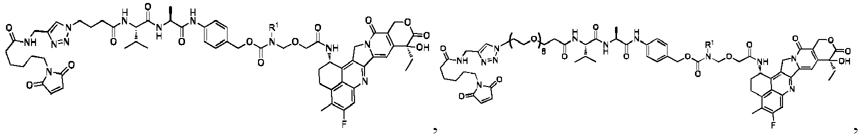 wherein, R¹ is as defined above.

In a preferred embodiment of the present disclosure, the linker-drug conjugate is preferably any of the compounds shown below:

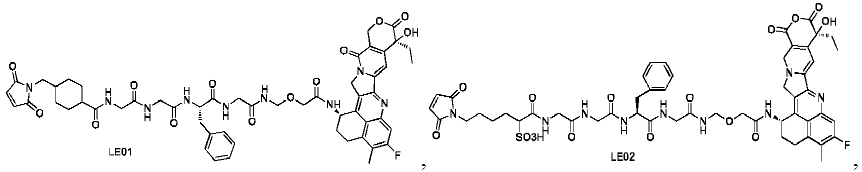

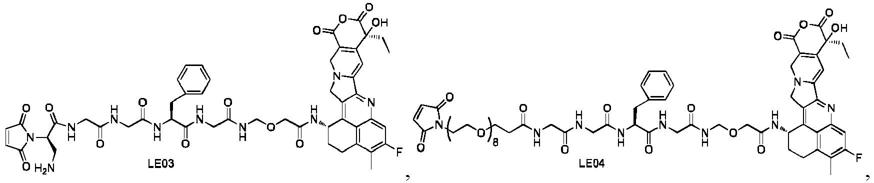

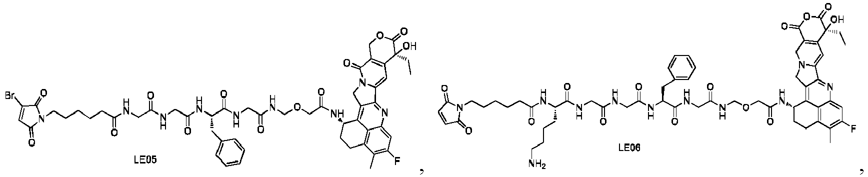

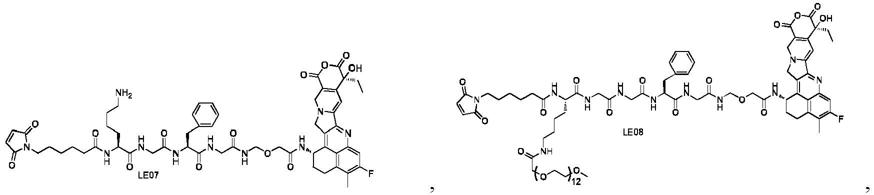

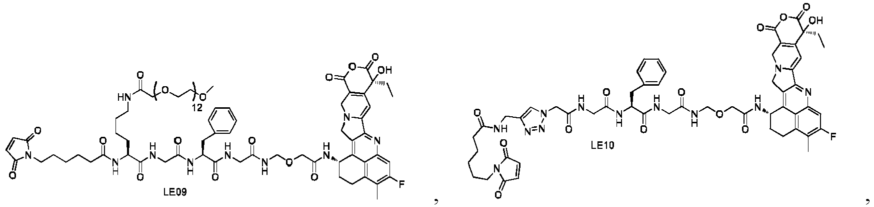

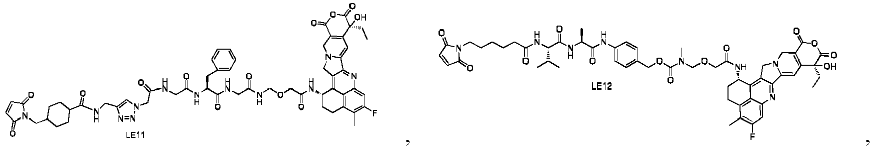

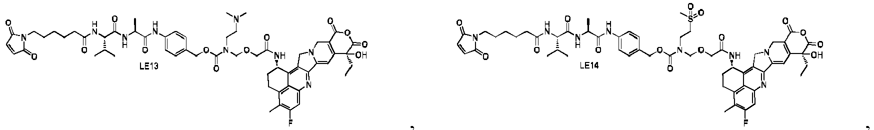

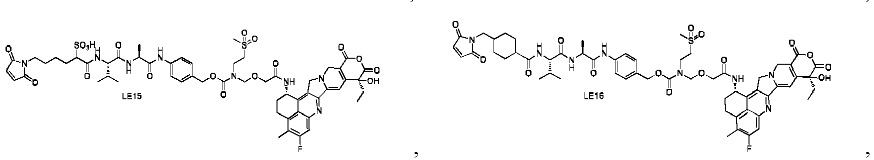

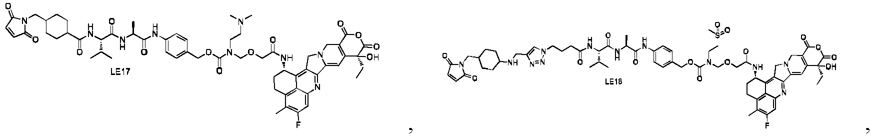

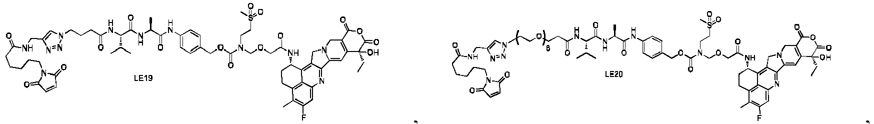

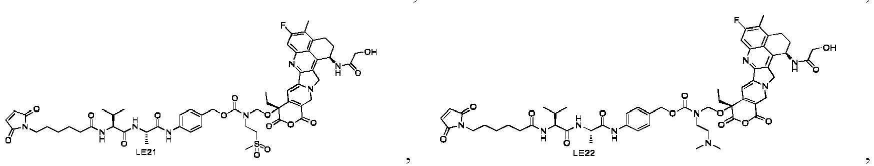

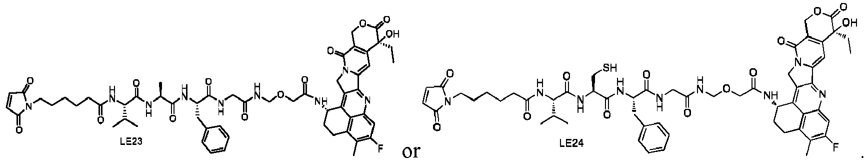

The present disclosure also provides a compound as follows, 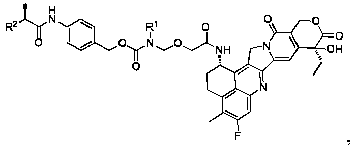
wherein, R¹ is as defined above;
R² is -N₃, -NH₂,

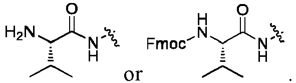

The present disclosure also provides the compounds as follows,

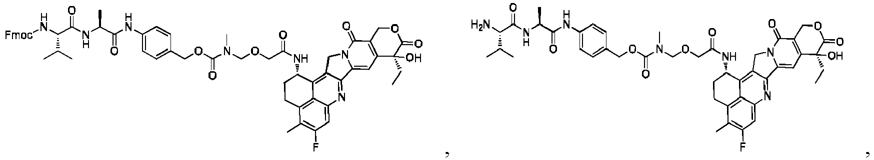

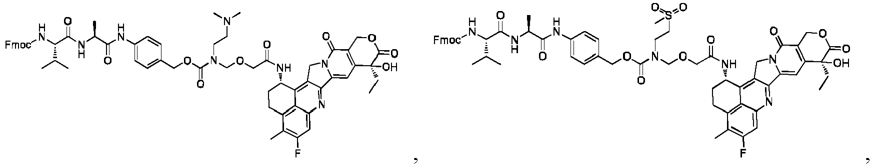

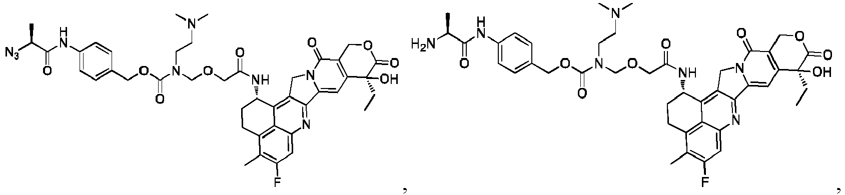

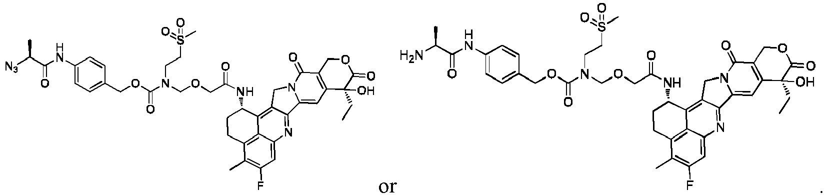

The present disclosure provides an antibody drug conjugate, a general structural formula of the antibody drug conjugate is Ab-(L₃-L₂-L₁-D)ₘ;
wherein, Ab is an antibody;
D is a cytotoxic drug;
m is 2-8;
the structure of L₁ is as shown in formula I, II, III or IV, a-end of the L₁ is connected to the cytotoxic drug, and e-end of the L₁ is connected to c-end of the L₂; 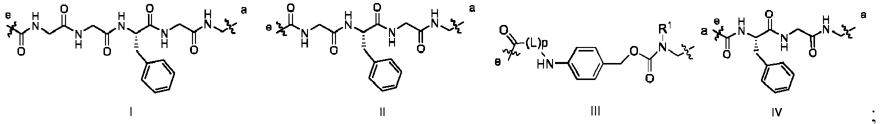
L₂ is 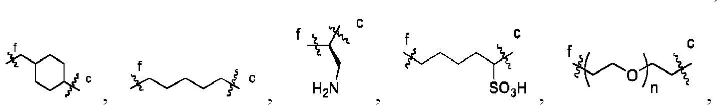 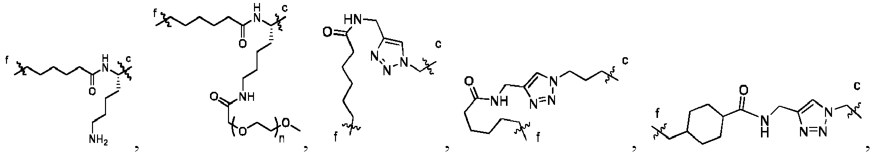 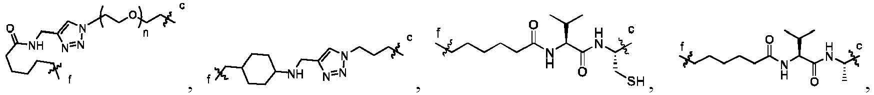 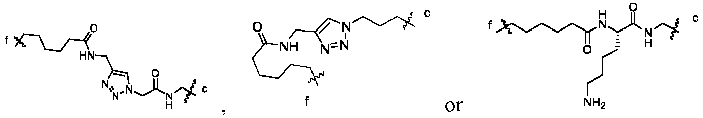 , wherein n is independently 1-12, c-end of the L₂ is connected to e-end of the L₁, f-end of the L₂ is connected to d-end of the L₃;
L₃ is 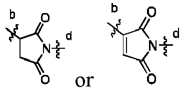 , wherein b-end of the L₃ is connected to the Ab, d-end of the L₃ is connected to f-end of the L₂;
wherein L is independently phenylalanine residue, glycine residue, glutamic acid residue, aspartic acid residue, cysteine residue, histidine residue, isoleucine residue, leucine residue, lysine residue, methionine residue, proline residue, serine residue, threonine residue, tryptophan residue, tyrosine residue or valine residue; p is 2-4;
R¹ is C₁-C₆ alkyl substituted by -NR¹⁻¹R¹⁻², C₁-C₆ alkyl substituted by R¹⁻³ S(O)₂-, C₁-C₆ alkyl, C₃-C₁₀ cycloalkyl, C₆-C₁₄ aryl or 5 to 14-membered heteroaryl; the heteroatoms in the 5 to 14-membered heteroaryl are selected from one or more of N, O and S, and the number of heteroatoms is 1, 2, 3, or 4;
the R¹⁻¹, R¹⁻² and R¹⁻³ are independently C₁-C₆ alkyl;
when the structure of the L₁ is as shown in formula I, the L₃ is 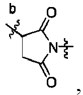 the L₂ is not

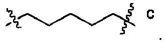

In a preferred embodiment of the present disclosure, in the antibody drug conjugates, some groups have the following definitions, and the definitions of unmentioned groups are as described in any of the above solutions (content of this paragraph is hereinafter referred to as "in a preferred embodiment of the present disclosure"):
the antibody can be a conventional antibody in the field of anti-tumor ADCs, preferably anti-HER2 antibody Trastuzumab or variant thereof, anti-B7-H3 antibody P2E5 or variant thereof, anti-Claudin18.2 antibody IMAB362 or variant thereof, or anti-Trop2 antibody RS7 or variant thereof, further preferably anti-HER2 antibody Trastuzumab or variant thereof, anti-B7-H3 antibody P2E5 or variant thereof, or anti-Claudin 18.2 antibody IMAB362 or variant thereof, further more preferably anti-HER2 antibody Trastuzumab or variant thereof, or anti-Claudin 18.2 antibody IMAB362 or variant thereof, and most preferably anti-HER2 antibody Trastuzumab or anti-Claudin 18.2 antibody IMAB362. The amino acid sequence of the light chain in the anti-HER2 antibody Trastuzumab is preferably shown in SEQ ID No. 5 in the sequence listing, and the amino acid sequence of the heavy chain in the anti-HER2 antibody Trastuzumab is preferably shown in SEQ ID No. 6 in the sequence listing. The amino acid sequence of the light chain in the anti-B7-H3 antibody P2E5 is preferably shown in SEQ ID No. 7 in the sequence listing, and the amino acid sequence of the heavy chain in the anti-B7-H3 antibody P2E5 is preferably shown in SEQ ID No. 8 in the sequence listing. The amino acid sequence of the light chain in the anti-Claudin 18.2 antibody IMAB362 is preferably shown in SEQ ID No. 1 in the sequence listing, and the amino acid sequence of the heavy chain in the anti-Claudin 18.2 antibody IMAB362 is preferably shown in SEQ ID No. 2 in the sequence listing. The amino acid sequence of the light chain in the anti-Trop2 antibody RS7 is preferably shown in SEQ ID No. 3 in the sequence listing, and the amino acid sequence of the heavy chain in the anti-Trop2 antibody RS7 is preferably shown in SEQ ID No. 4 in the sequence listing.

In a preferred embodiment of the present disclosure, b-end of the L₃ is preferably connected to the sulfhydryl in the antibody in the form of a thioether bond. Taking 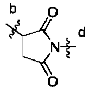 as an example, the connection form of 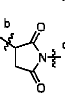 to the cysteine residue in the antibody is

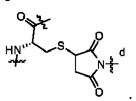

In a preferred embodiment of the present disclosure, the cytotoxic drug can be a conventional cytotoxic drug in the field of ADCs, particularly preferably a topoisomerase inhibitor containing a hydroxyl group, and more preferably a topoisomerase I inhibitor containing a hydroxyl group, further preferably camptothecin or derivatives thereof, and further more preferably

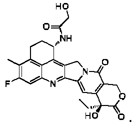

The L₁ is preferably connected to the hydroxyl group in the cytotoxic drug in the form of an ether bond. After the L₁ is connected to 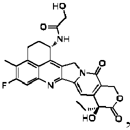 the fragment of the cytotoxic drug remaining in the antibody drug conjugate is preferably 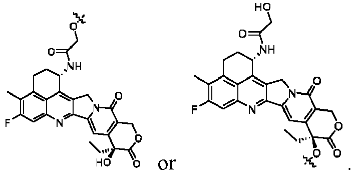 Taking 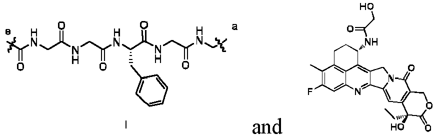 as examples, the -L₁-D can be

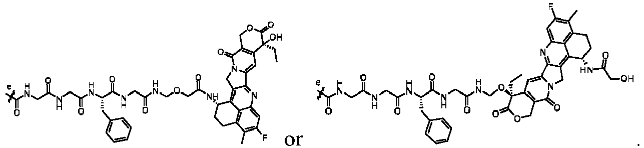

In a preferred embodiment of the present disclosure, when the R¹ is C₁-C₆ alkyl substituted by -NR¹⁻¹R¹⁻², the C₁-C₆ alkyl is preferably C₁-C₄ alkyl, more preferably methyl, ethyl, *n*-propyl, isopropyl, *n*-butyl, isobutyl or *tert-butyl,* most preferably ethyl. The R¹⁻¹ and R¹⁻² are each independently preferably C₁-C₄ alkyl, more preferably methyl, ethyl, *n*-propyl, isopropyl, *n*-butyl, isobutyl or *tert-butyl,* most preferably methyl.

In a preferred embodiment of the present disclosure, when the R¹ is C₁-C₆ alkyl substituted by R¹⁻³S(O)₂-, the C₁-C₆ alkyl is preferably C₁-C₄ alkyl, more preferably methyl, ethyl, *n*-propyl, isopropyl, *n*-butyl, isobutyl or *tert-butyl,* most preferably ethyl. The R¹⁻³ is preferably C₁-C₄ alkyl, more preferably methyl, ethyl, *n*-propyl, isopropyl, *n*-butyl, isobutyl or *tert-butyl,* most preferably methyl.

In a preferred embodiment of the present disclosure, when the R¹ is C₁-C₆ alkyl, the C₁-C₆ alkyl is preferably C₁-C₄ alkyl, more preferably methyl, ethyl, *n*-propyl, isopropyl, *n*-butyl, isobutyl or *tert-butyl,* most preferably methyl.

In a preferred embodiment of the present disclosure, the m is preferably 4-8, more preferably 7-8 (for example, 7.3, 7.4, 7.5, 7.6, 7.7, 7.8).

In a preferred embodiment of the present disclosure, the L is preferably valine residue or alanine residue, and p is preferably 2. The (L)p is further preferably 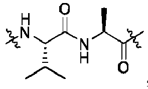 , wherein the amino-end of the (L)ₚ is connected to the carbonyl-end in the formula III.

In a preferred embodiment of the present disclosure, the n is preferably 8-12 (for example, 8 and 12).

In a preferred embodiment of the present disclosure, the R¹⁻¹, R¹⁻² and R¹⁻³ are independently preferably C₁-C₄ alkyl, more preferably methyl, ethyl, *n*-propyl, isopropyl, *n*-butyl, isobutyl or *tert-butyl,* most preferably methyl.

In a preferred embodiment of the present disclosure, the 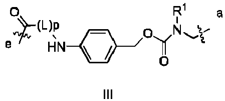 is preferably

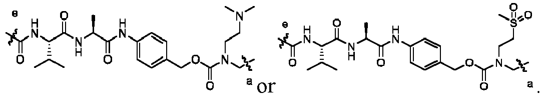

In a preferred embodiment of the present disclosure, the L₃ is preferably

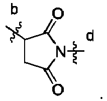

In a preferred embodiment of the present disclosure, when the structure of L₁ is as shown in formula I, the L₂ is preferably

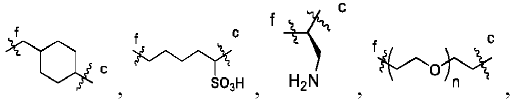

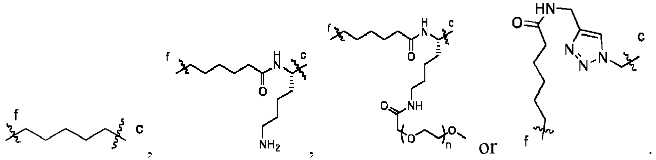

In a preferred embodiment of the present disclosure, when the structure of L₁ is as shown in formula II, the L₂ is preferably 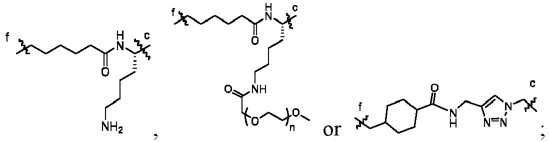 the L₃ is preferably

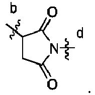

In a preferred embodiment of the present disclosure, when the structure of L₁ is as shown in formula III, the L₂ is preferably 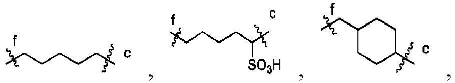 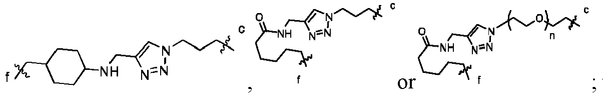 the L₃ is preferably

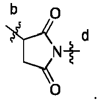

In a preferred embodiment of the present disclosure, when the structure of L₁ is as shown in formula IV, the L₂ is preferably 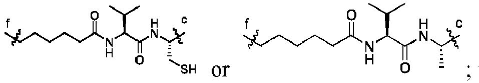 the L₃ is preferably

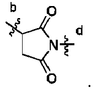

In a preferred embodiment of the present disclosure, the R¹ is preferably C₁-C₆ alkyl substituted by -NR¹⁻¹R¹⁻², C₁-C₆ alkyl substituted by R¹⁻³S(O)₂-, or C₁-C₆ alkyl.

In a preferred embodiment of the present disclosure, in the antibody drug conjugate, the Ab is anti-HER2 antibody Trastuzumab, anti-B7-H3 antibody P2E5 or variant thereof, anti-Claudin18.2 antibody IMAB362 or variant thereof; the D is a cytotoxic drug; the m is 2-8;
the structure of the L₁ is as shown in formula I, II, III or IV, 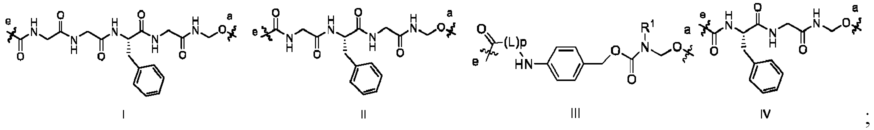
the L₂ is 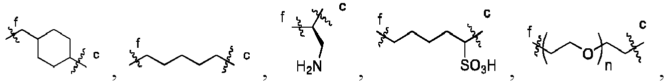 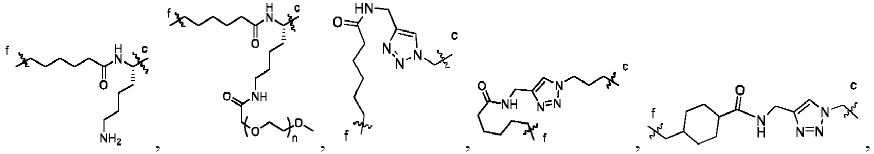 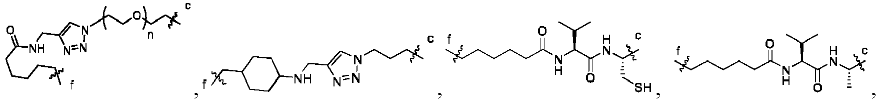 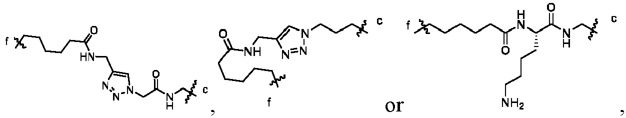 the n is independently 8-12;
the L₃ is 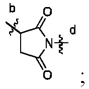
the L is independently valine residue or alanine residue; the p is 2-4;
the R¹ is C₁-C₆ alkyl substituted by -NR¹⁻¹R¹⁻², C₁-C₆ alkyl substituted by R¹⁻³ S(O)₂-, or C₁-C₆ alkyl;
the R¹⁻¹, R¹⁻² and R¹⁻³ are independently C₁-C₆ alkyl;
wherein, the amino acid sequence of the light chain in the anti-HER2 antibody Trastuzumab is preferably shown in SEQ ID No. 5 in the sequence listing, and the amino acid sequence of the heavy chain in the anti-HER2 antibody Trastuzumab is preferably shown in SEQ ID No. 6 in the sequence listing; the amino acid sequence of the light chain in the anti-B7-H3 antibody P2E5 is preferably shown in SEQ ID No. 7 in the sequence listing, and the amino acid sequence of the heavy chain in the anti-B7-H3 antibody P2E5 is preferably shown in SEQ ID No. 8 in the sequence listing; the amino acid sequence of the light chain in the anti-Claudin 18.2 antibody IMAB362 is preferably shown in SEQ ID No. 1 in the sequence listing, and the amino acid sequence of the heavy chain in the anti-Claudin 18.2 antibody IMAB362 is preferably shown in SEQ ID No. 2 in the sequence listing.

In a preferred embodiment of the present disclosure, in the antibody drug conjugate, the Ab is anti-HER2 antibody Trastuzumab, anti-B7-H3 antibody P2E5 or variant thereof, anti-Claudin18.2 antibody IMAB362 or variant thereof; the D is 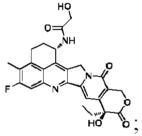 the m is 7-8;
when the structure of L₁ is as shown in formula I, the L₂ is 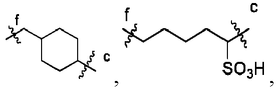 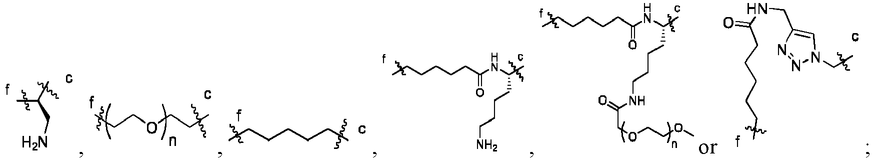 the n is independently 8-12;
when the structure of L₁ is as shown in formula II, the L₂ is 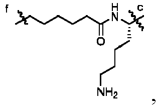 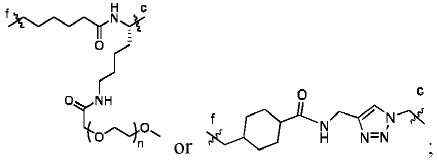 the n is 8-12;
when the structure of L₁ is as shown in formula III, the L₂ is 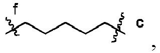 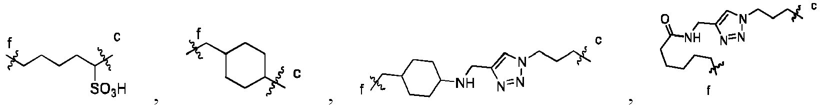 or 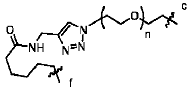 ; the n is 8-12;
when the structure of L₁ is as shown in formula IV, the L₂ is 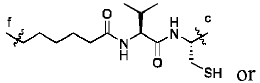 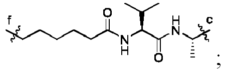
the L₃ is 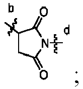
the L is independently valine residue or alanine residue; the p is 2 to 4;
the R¹ is C₁-C₄ alkyl substituted by -NR¹⁻¹R¹⁻², C₁-C₄ alkyl substituted by R¹⁻³S(O)₂-, or C₁-C₄ alkyl; the R¹⁻¹, R¹⁻² and R¹⁻³ are independently C₁-C₄ alkyl;
the amino acid sequence of the light chain in the anti-HER2 antibody Trastuzumab is preferably shown in SEQ ID No. 5 in the sequence listing, and the amino acid sequence of the heavy chain in the anti-HER2 antibody Trastuzumab is preferably shown in SEQ ID No. 6 in the sequence listing; the amino acid sequence of the light chain in the anti-B7-H3 antibody P2E5 is preferably shown in SEQ ID No. 7 in the sequence listing, and the amino acid sequence of the heavy chain in the anti-B7-H3 antibody P2E5 is preferably shown in SEQ ID No. 8 in the sequence listing; the amino acid sequence of the light chain in the anti-Claudin 18.2 antibody IMAB362 is preferably shown in SEQ ID No. 1 in the sequence listing, and the amino acid sequence of the heavy chain in the anti-Claudin 18.2 antibody IMAB362 is preferably shown in SEQ ID No. 2 in the sequence listing.

In a preferred embodiment of the present disclosure, the antibody drug conjugate is preferably any of the compounds shown below: 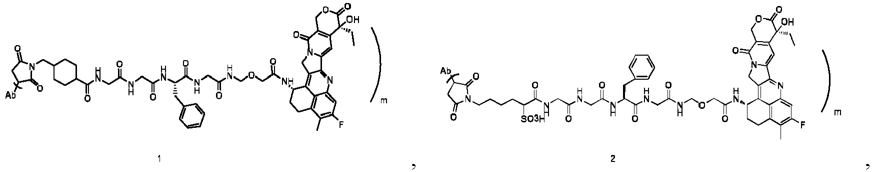 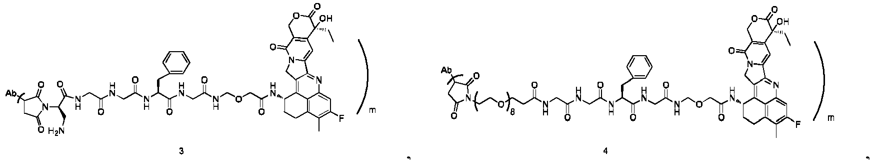 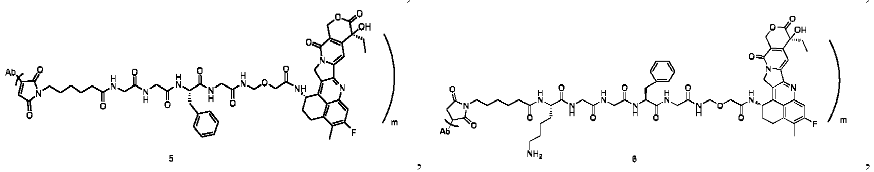 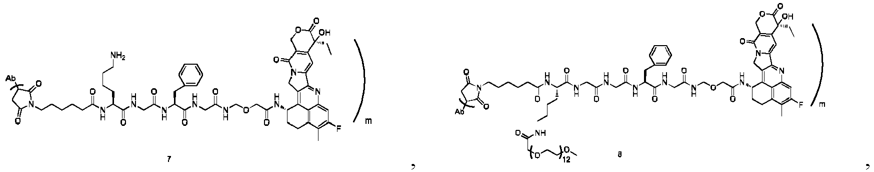 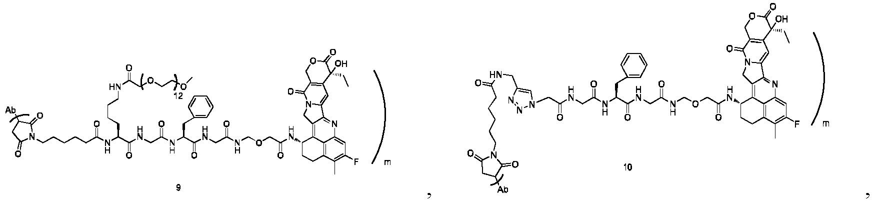 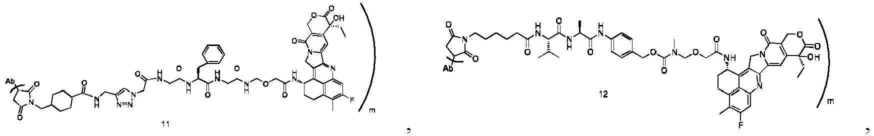 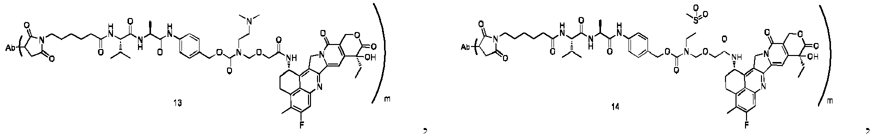 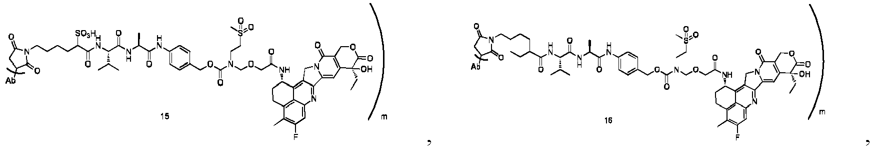 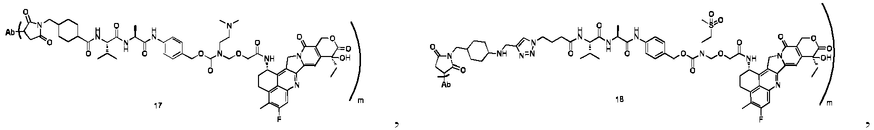 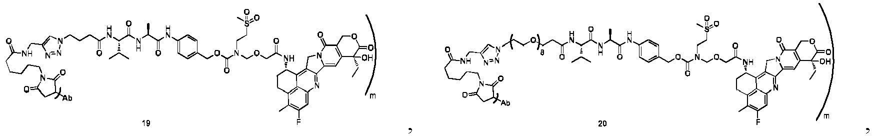 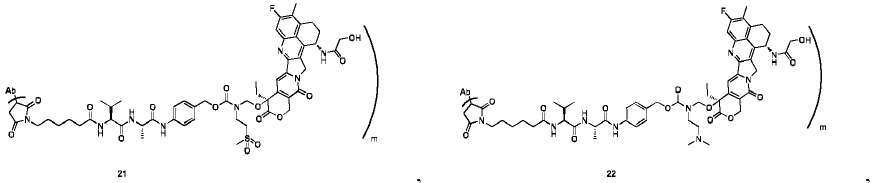 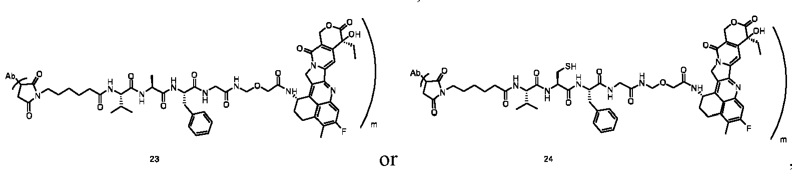 , wherein, Ab is anti-HER2 antibody Trastuzumab, anti-B7-H3 antibody P2E5 or anti-Claudin 18.2 antibody IMAB362, m is 7.3, 7.4, 7.5, 7.6, 7.7 or 7.8; the amino acid sequence of the light chain in the anti-HER2 antibody Trastuzumab is preferably shown in SEQ ID No. 5 in the sequence listing, and the amino acid sequence of the heavy chain in the anti-HER2 antibody Trastuzumab is preferably shown in SEQ ID No. 6 in the sequence listing; the amino acid sequence of the light chain in the anti-B7-H3 antibody P2E5 is preferably shown in SEQ ID No. 7 in the sequence listing, and the amino acid sequence of the heavy chain in the anti-B7-H3 antibody P2E5 is preferably shown in SEQ ID No. 8 in the sequence listing; the amino acid sequence of the light chain in the anti-Claudin 18.2 antibody IMAB362 is preferably shown in SEQ ID No. 1 in the sequence listing, and the amino acid sequence of the heavy chain in the anti-Claudin 18.2 antibody IMAB362 is preferably shown in SEQ ID No. 2 in the sequence listing.

In a preferred embodiment of the present disclosure, the antibody drug conjugate is preferably any of the compounds shown below: 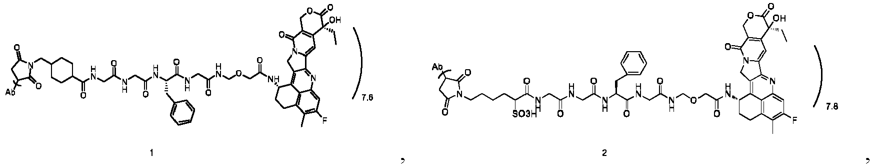 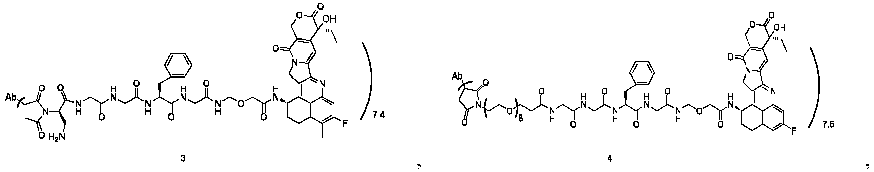 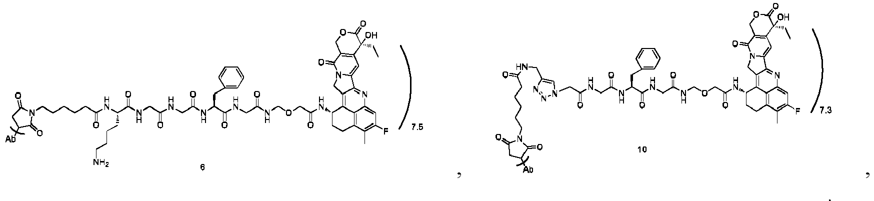 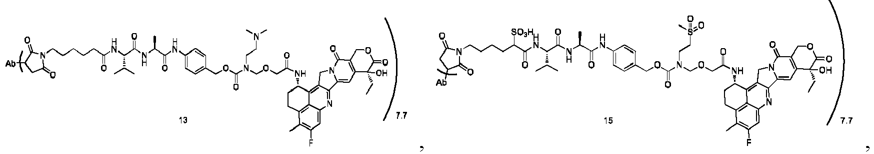 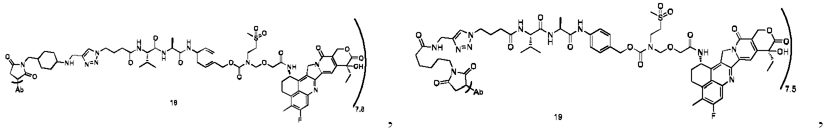 or 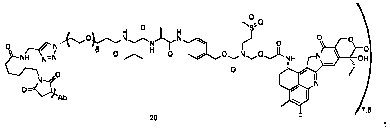 preferably 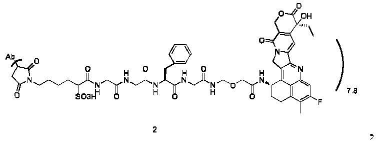 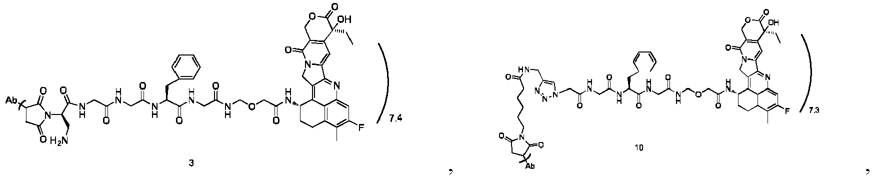 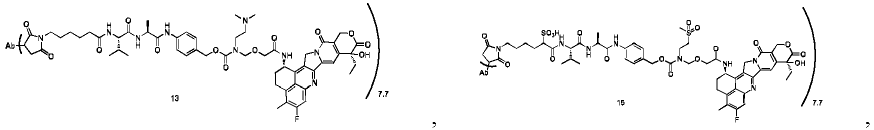 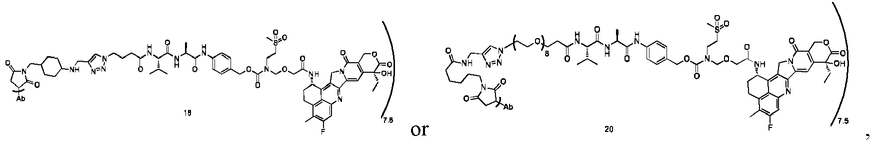 wherein, Ab is anti-HER2 antibody Trastuzumab; the amino acid sequence of the light chain in the anti-HER2 antibody Trastuzumab is preferably shown in SEQ ID No. 5 in the sequence listing, and the amino acid sequence of the heavy chain in the anti-HER2 antibody Trastuzumab is preferably shown in SEQ ID No. 6 in the sequence listing.

In a preferred embodiment of the present disclosure, the antibody drug conjugate is preferably any of the compounds shown below: 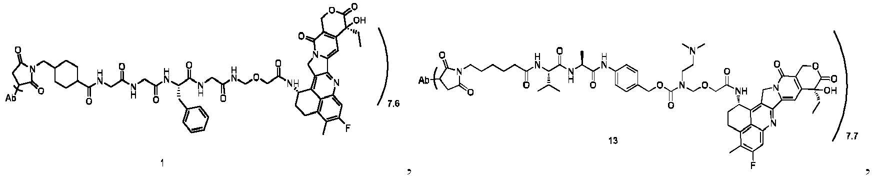 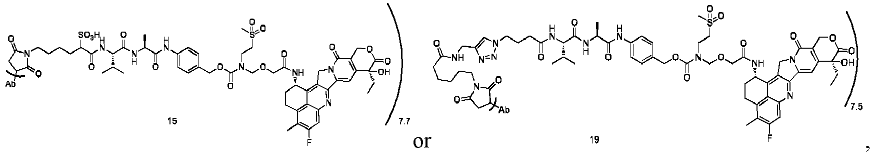 wherein, Ab is anti-HER2 antibody Trastuzumab; the amino acid sequence of the light chain in the anti-HER2 antibody Trastuzumab is preferably shown in SEQ ID No. 5 in the sequence listing, and the amino acid sequence of the heavy chain in the anti-HER2 antibody Trastuzumab is preferably shown in SEQ ID No. 6 in the sequence listing.

In a preferred embodiment of the present disclosure, the antibody drug conjugate is preferably any of the compounds shown below: 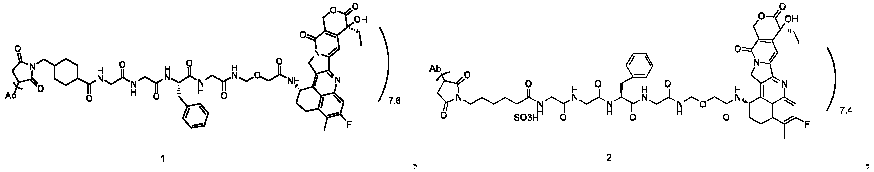 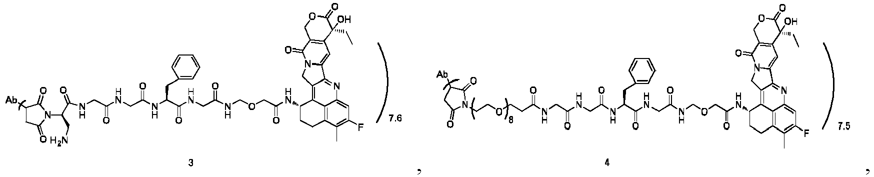 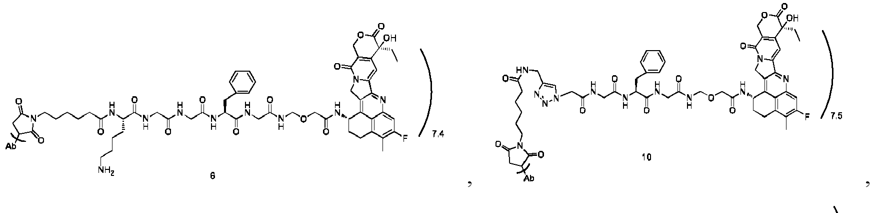 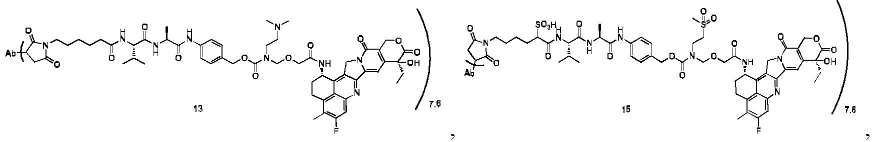 or 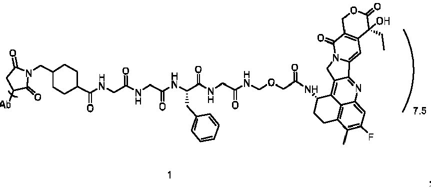 , wherein, Ab is anti-B7-H3 antibody P2E5; the amino acid sequence of the light chain in the anti-B7-H3 antibody P2E5 is preferably shown in SEQ ID No. 7 in the sequence listing, and the amino acid sequence of the heavy chain in the anti-B7-H3 antibody P2E5 is preferably shown in SEQ ID No. 8 in the sequence listing.

In a preferred embodiment of the present disclosure, the antibody drug conjugate is preferably any of the compounds shown below: 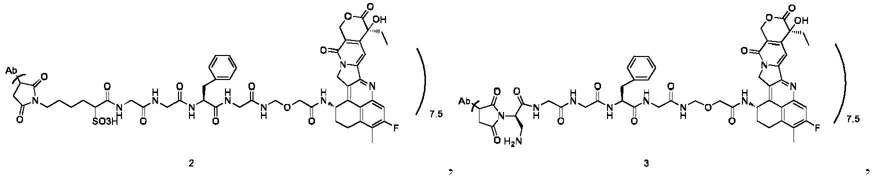 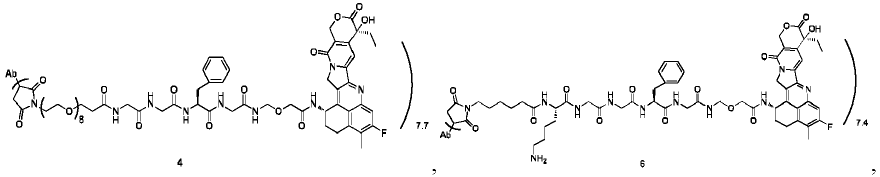 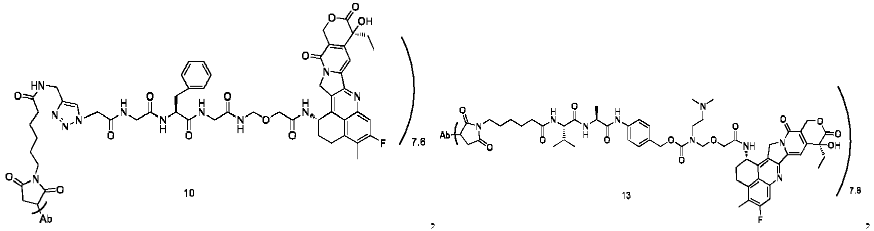 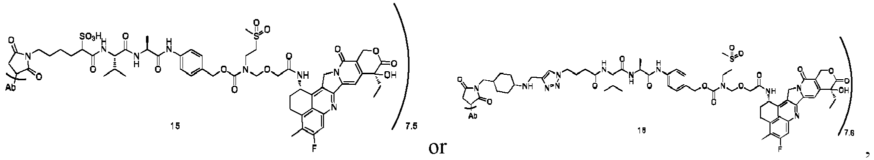 wherein, Ab is anti-Claudin 18.2 antibody IMAB362; the amino acid sequence of the light chain in the anti-Claudin 18.2 antibody IMAB362 is shown in SEQ ID No. 1 in the sequence listing, and the amino acid sequence of the heavy chain in the anti-Claudin 18.2 antibody IMAB362 is shown in SEQ ID No. 2 in the sequence listing.

The present disclosure also provides a linker-drug conjugate, a general structural formula of the linker-drug conjugate is L₄-L₂-L₁-D; wherein L₄ is 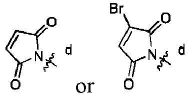 ; L₂, L₁, and D are as defined above, f-end of the L₂ is connected to d-end of the L₄; when the L₄ is 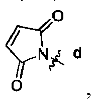 the L₁ is 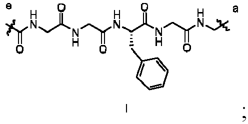 the L₂ is not

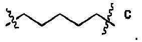

In a preferred embodiment of the present disclosure, the linker-drug conjugate is preferably any of the compounds shown below:

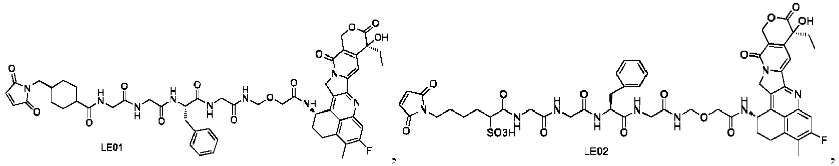

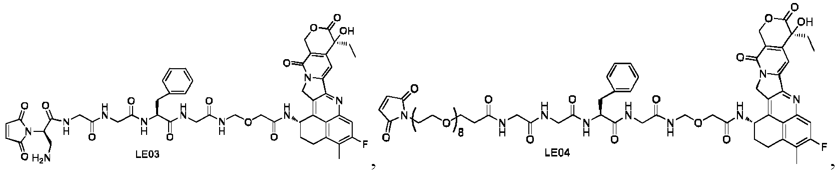

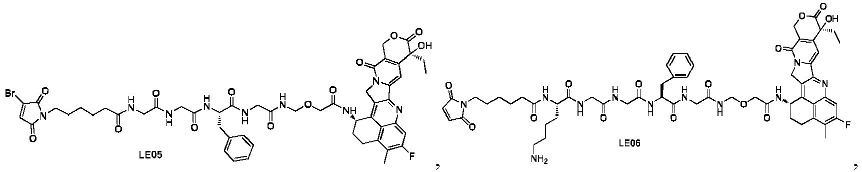

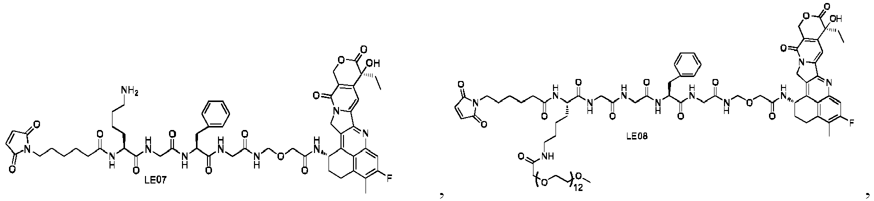

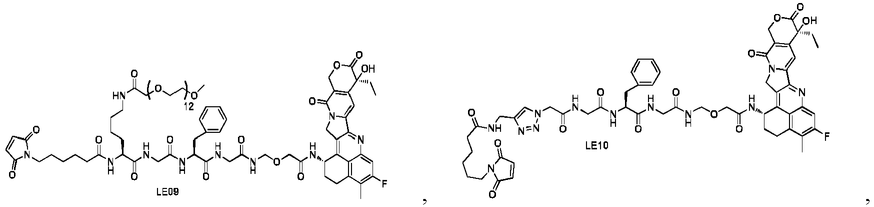

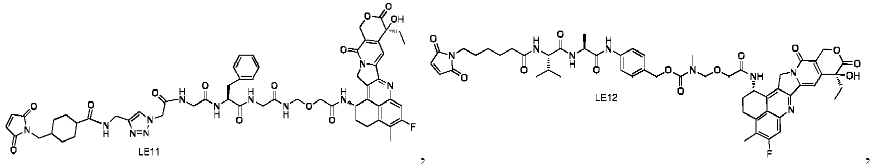

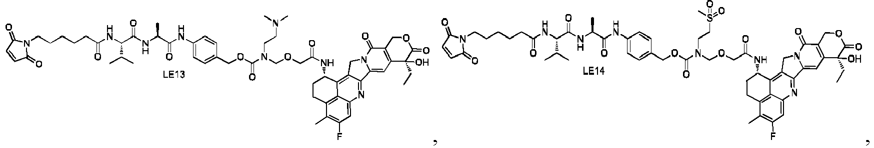

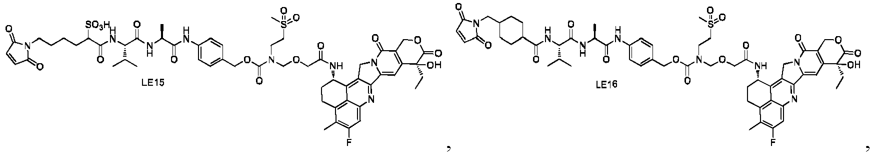

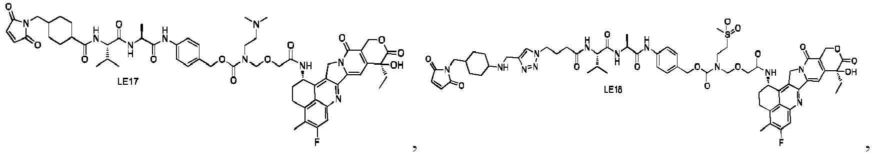

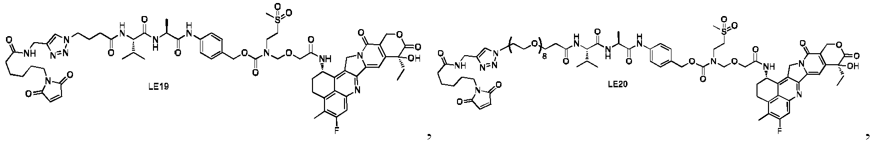

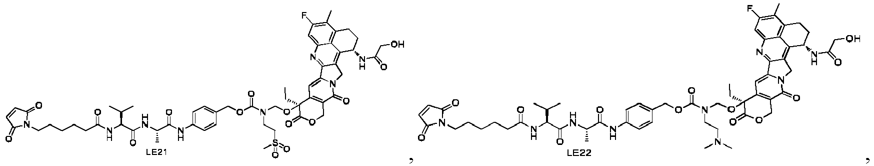

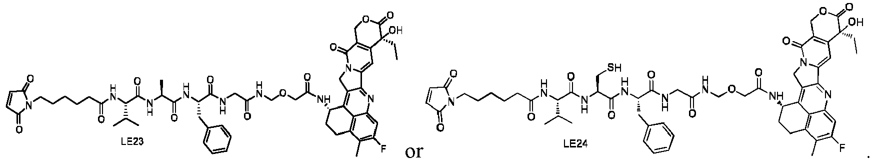

The present disclosure also provides compounds as follows,

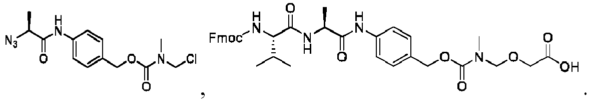

The present disclosure provides a method for preparing the antibody drug conjugate, comprising the following steps, coupling the linker-drug conjugate with the antibody.

In the present disclosure, the coupling conditions and operations can be conventional conditions and operations for coupling in the art.

The present disclosure also provides a pharmaceutical composition, comprising the antibody drug conjugate and a pharmaceutically acceptable carrier.

The present disclosure also provides a use of the antibody drug conjugate or the pharmaceutical composition in the preparation of a medicament for the prevention or treatment of a cancer. The cancer is preferably gastric cancer, breast cancer, non-small cell lung cancer, urothelial cancer or pancreatic cancer.

The present disclosure also provides a method for the prevention and/or treatment of a cancer, comprising administrating a therapeutically effective amount of the antibody drug conjugate or the pharmaceutical composition to a subject. The cancer is preferably gastric cancer, breast cancer, non-small cell lung cancer, urothelial cancer or pancreatic cancer.

In the present disclosure, m represents the molar ratio of cytotoxic drug molecule to Ab (also known as DAR, that is, drug antibody coupling ratio), m can be an integer or a decimal, and is preferably understood as: the average value of the molar ratio of the drug molecule to the monoclonal antibody molecule in the antibody drug conjugate obtained by coupling a single monoclonal antibody molecule with cytotoxic drug, generally can be measured by Hydrophobic-Interaction Chromatography (HIC), polyacrylamide-SDS gel electrophoresis (SDS-PAGE, electrophoresis), liquid chromatograph-mass spectrometer (LC-MS) and other methods.

In the present disclosure, the term "C₁-C₆ alkyl" alone or in combination represents a saturated linear or branched alkyl group containing 1 to 6, especially 1 to 4 carbon atoms, such as methyl and ethyl, *n*-propyl, isopropyl, *n*-butyl, isobutyl or *tert-butyl,* preferably "C₁-C₆ alkyl" represents methyl or ethyl.

The antibody of the present disclosure can be prepared by well-known techniques in the art, such as hybridoma methods, recombinant DNA techniques, phage display techniques, synthesis techniques, or a combination of these techniques, or other techniques known in the art.

Variants refer to mutants of the amino acid sequence of antibody and covalent derivatives of natural polypeptides, provided that the biological activity equivalent to that of natural polypeptides is retained. The difference between amino acid sequence mutants and natural amino acid sequences is generally that one or more amino acids in the natural amino acid sequence are replaced or one or more amino acids are deleted and/or inserted in the polypeptide sequence. Deletion mutants include fragments of natural polypeptides and N-terminal and/or C-terminal truncation mutants. Generally, amino acid sequence mutants have at least 70%, 75%, 80%, 85%, 90%, 95%, 98% or 99% homology compared with natural sequence.

The term "treatment" or its equivalent expression when applied to, for example, cancer, refers to a procedure or process used to reduce or eliminate the number of cancer cells in a patient or alleviate the symptoms of cancer. "Treatment" of cancer or other proliferative disorders does not necessarily mean that cancer cells or other disorders will actually be eliminated, the number of cells or disorders will actually be reduced or the symptoms of cancer or other disorders will actually be alleviated. Normally, even if there is only a low probability of success, the method of treating cancer will be performed, but the patient's medical history and estimated survival expectations are taken into account, it is still considered to induce an overall beneficial course of action.

The term "pharmaceutically acceptable carrier" refers to any formulation or carrier medium that can deliver an effective amount of the active substance of the present disclosure, does not interfere with the biological activity of the active substance, and has no toxic side effects on the host or patient. Representative carriers include water, oil, vegetables and minerals, cream base, lotion base, ointment base, etc. These bases include suspending agents, tackifiers, penetration enhancers and the like. Their formulations are well known to those skilled in the art of cosmetics or topical medicine.

On the basis of not violating common knowledge in the art, the preferred conditions can be combined arbitrarily to obtain preferred embodiments of the present disclosure.

The reagents and raw materials used in the present disclosure are all commercially available.

The positive and progressive effect of the present disclosure is that: the antibody drug conjugate of the present disclosure has better biological activity, stability and uniformity, has reduced toxic and side effects, and has a faster release rate of enzyme cleavage in tumor cells. The use of this new type of antibody drug conjugate can achieve the widely use of cytotoxic drugs, especially camptothecin compounds in the field of ADCs, and treat tumor patients who are resistant to microtubule ADCs.

### Detailed description of the preferred embodiment

**Table 1. Description of abbreviations**

| | |
|---|---|
| SMCC | 4-(N-malcimidomethyl)cyclohexane-1-carboxylic acid succinimidyl ester |
| DMF | N,N-Dimethylformamide |
| ESI-MS | Electrospray mass spectrometry |
| HATU | 2-(7-Azabenzotriazol-1-yl)-N,N,N',N'- tetramethyluronium hexafluorophosphate |
| TCEP | Tris(2-carboxyethyl)phosphine |
| DMSO | Dimethyl sulfoxide |
| UV | Ultraviolet visible light |
| V/V | Volume ratio |
| mmol | Millimole |
| h | Hour |
| g | Gram |
| IC₅₀ | Half inhibitory concentration |
| PB | Phosphate buffer |
| EDTA | Ethylenediaminetetraacetic acid |
| MMT | 4- Methoxytrityl |

The following embodiments further illustrate the present disclosure, but the present disclosure is not limited thereto. In the following embodiments, the experimental methods without specific conditions are selected according to conventional methods and conditions, or according to the product specification.

### Embodiment 1: Synthesis of LE 01

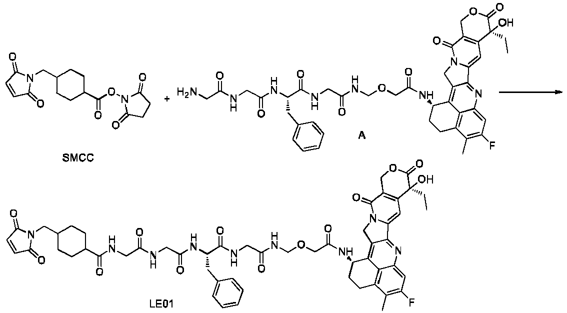

Commercially available SMCC (1 mmol, 0.32 g) and compound A (0.5 mmol, 0.42g) were dissolved in 10 mL of DMF, the mixture was stirred at room temperature for 3 h, and the solvent was removed by distillation under reduced pressure, the crude product was purified by silica gel column chromatography [chloroform-chloroform: methanol=9:1 (V/V)] to obtain the title compound (0.5 g, 0.47 mmol) as a pale yellow solid, yield: 94%, ESI-MS m/z: 1060.3 (M+H); wherein the compound A can be synthesized according to the known method reported in WO2015146132A1.

The compound GGFG-Dxd (the structure is as follows) was also synthesized according to the known method reported in WO2015146132A1, ESI-MS m/z: 1034.5 (M+H), ¹H-NMR (400 MHz, DMSO-*d₆*) δ 8.61 (t, *J* = 6.4 Hz, 1H), 8.50 (d, *J* = 8.5 Hz, 1H), 8.28 (t, *J* = 5.1 Hz, 1H), 8.11 (d, *J=* 7.5 Hz, 1H), 8.05 (t, *J* = 5.7 Hz, 1H), 7.99 (t, *J* = 5.9 Hz, 1H), 7.77 (d, *J=* 11.0 Hz, 1H), 7.31 (s, 1H), 7.25 - 7.16 (m, 5H), 6.98 (s, 2H), 6.51 (s, 1H), 5.59 (dt*, J* = 7.4, 4.1 Hz, 1H), 5.41 (s, 2H), 5.20 (s, 2H), 4.64 (d, *J* = 6.1 Hz, 2H), 4.53 - 4.40 (m, 1H), 4.02 (s, 2H), 3.74 - 3.37 (m, 8H), 3.18 - 3.00 (m, 2H), 3.04 - 2.97 (m, 1H), 2.77 (dd, *J=* 13.5 , 9.4 Hz, 1H), 2.38 (s, 3H), 2.19 (dd, *J=* 14.9, 8.5 Hz, 2H), 2.11 - 2.05 (m, 2H), 1.86 (dd, *J* = 14.0, 6.7 Hz, 2H) , 1.45 (s, 4H), 1.20 - 1.14 (m, 2H), 0.87 (t, *J* = 7.1 Hz, 3H).

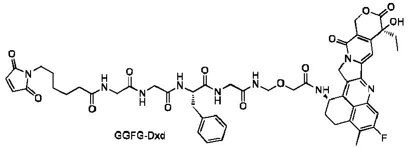

### Embodiment 2: Synthesis of LE02-LE06 and LE08

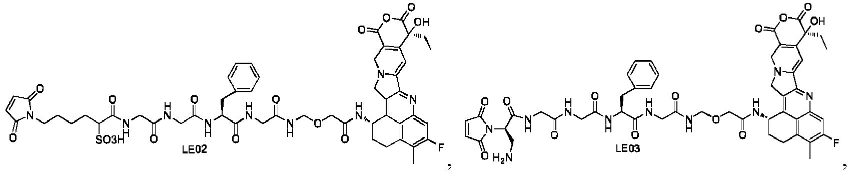

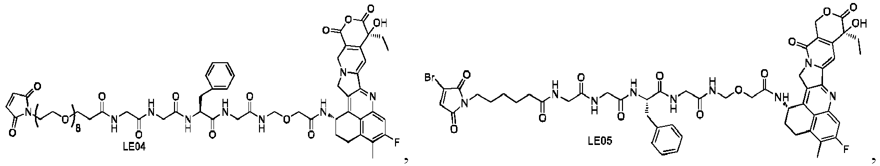

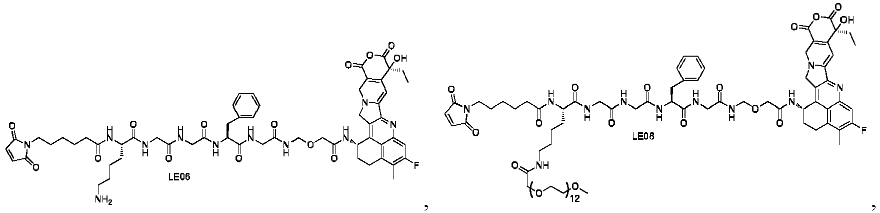

Referring to Embodiment 1, compounds were obtained by condensation reaction (LE02, LE03 and LE06 need to be acidified after the condensation reaction) between appropriate maleamide fragments with compound A. The structures of the specific maleamide fragments used are shown in Table 2. Compound LE02: pale yellow solid, ESI-MS m/z: 1114.2 (M+H); compound LE03: pale yellow solid, ESI-MS m/z: 1007.2 (M+H); compound LE04: slightly yellow solid, ESI-MS m/z: 1344.5 (M+H); compound LE05: yellow solid, ESI-MS m/z: 1112.3 (M+H); compound LE06: yellow solid, ESI-MS m/z: 1162.5 (M+H); compound LE08: pale yellow oil, ESI-MS m/z: 1719.1 (M+H).

**Table 2. The structures of the maleamide fragment used in the synthesis of LE02-LE06 and LE08**

| Product | Structure of maleamide fragment | Product | Structure of maleamide fragment |
|---|---|---|---|
| LE02 | 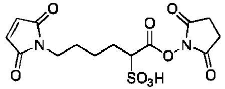 | LE05 | 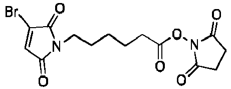 |
| LE03 | 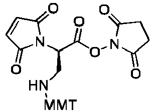 | LE06 | 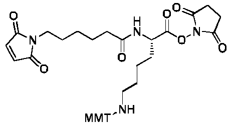 |
| LE04 | 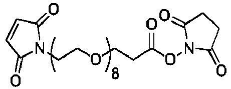 | LE08 | 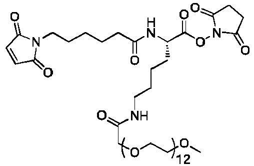 |

### Embodiment 3: Synthesis of LE07

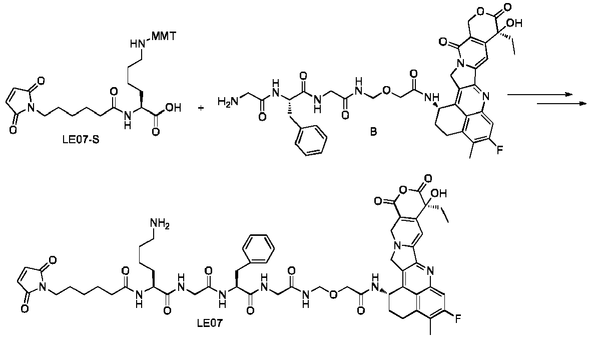

Commercially available LE07-S (1 mmol, 0.34 g) and compound B (0.5 mmol, 0.38 g) were dissolved in 10 mL of DMF; HATU (0.5 mmol, 0.19 g), 0.5 mL of triethylamine were added, and the mixture was stirred at room temperature for 3 h, 0.5 mL of trifluoroacetic acid was added, then the mixture was stirred at room temperature for 10 min, the solvent was removed by distillation under reduced pressure, and the obtained crude product was purified by silica gel column chromatography [chloroform - chloroform: methanol = 9:1 (V/V)] to obtain trifluoroacetic acid salt of the title compound (0.33 g, 0.3 mmol) as pale yellow solid , yield: 60%, ESI-MS m/z: 1105.3 (M+H). Compound B can be synthesized according to the known method reported in WO2015146132A1.

### Embodiment 4: Synthesis of LE09-LE11

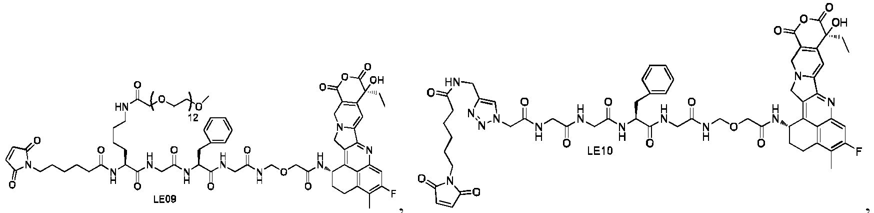

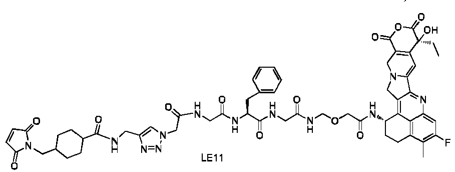

Referring to the method of Embodiment 3, compounds were obtained by condensation reaction between compound B and appropriate carboxylic acid fragments (commercially available) with a condensing agent. The structures of the specific carboxylic acid fragments used are shown in Table 3. Compound LE09: pale light yellow oil, ESI-MS m/z: 1705.9 (M+H). Compound LE10: ESI-MS: m/z: 1115.9 (M+H), ¹H-NMR (400 MHz, DMSO-*d₆*) δ 8.66 (t, *J* = 6.7 Hz, 1H), 8.53 (d, *J* = 9.0 Hz , 1H), 8.47 (t, *J* = 5.2 Hz, 1H), 8.37 (t, *J* = 5.8 Hz, 1H), 8.30 (d, *J* = 8.4 Hz, 2H), 7.81 (s, 1H), 7.78 (d *, J* = 11.1 Hz, 1H), 7.31 (s, 1H), 7.20 (dd, *J* = 21.9, 7.3 Hz, 5H), 7.00 (d, *J* = 5.3 Hz, 2H), 6.54 (s, 1H), 5.60 (dd, *J* = 13.7, 6.8 Hz, 1H), 5.42 (s, 2H), 5.20 (s, 2H), 5.10 (s, 2H), 4.64 (d, *J* = 6.3 Hz, 2H), 4.55 - 4.45 (m, 1H), 4.26 (d, *J* = 5.3 Hz, 2H), 4.02 (s, 2H), 3.74 (ddd, *J* = 31.4, 16.7, 5.6 Hz, 6H), 3.17 (dd, *J* = 14.3, 8.5 Hz , 2H), 3.02 (dd, *J* = 14.1, 4.3 Hz, 1H), 2.74 (dd, *J* = 13.4, 10.0 Hz, 1H), 2.38 (s, 3H), 2.23 - 2.15 (m, 2H), 2.05 (t, *J* = 7.4 Hz, 2H), 1.90 - 1.80 (m, 2H), 1.46 (dd, *J* = 14.7, 7.3 Hz, 4H), 1.19 - 1.14 (m, 2H), 0.87 (t, *J* = 7.2 Hz , 3H). Compound LE11: pale yellow solid, ESI-MS m/z: 1141.5 (M+H).

**Table 3. Structures of the carboxylic acid fragments used in the synthesis of LE09-LE11**

| Product | Structure of carboxylic acid fragment |
|---|---|
| LE09 | 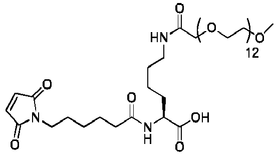 |
| LE10 | 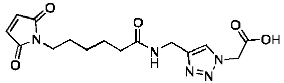 |
| LE11 | 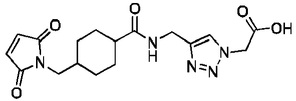 |

### Embodiment 5: Synthesis of LE23-LE24

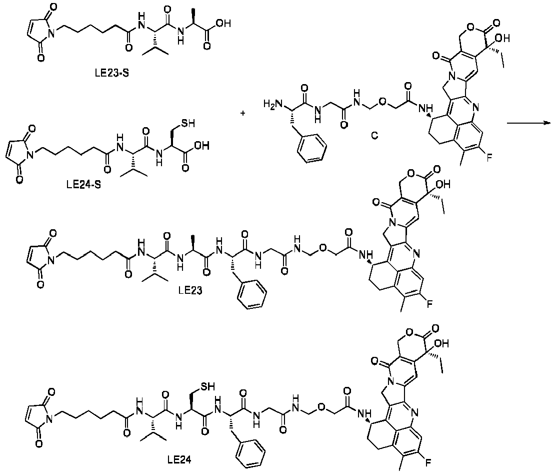

Commercially available compound LE23-S or LE24-S (2 equivalents) and compound C (1 equivalent) were dissolved in an appropriate amount of DMF, the mixture was stirred at room temperature for 3 h, and the solvent was removed by distillation under reduced pressure, and the obtained crude product was purified by silica gel column chromatography [chloroform-chloroform: methanol = 10:1 (V/V)] to obtain the title compound as a pale yellow solid. Wherein, compound C can be synthesized according to the known method reported in WO2015146132A1. Compound LE23: yellow solid, ESI-MS m/z: 1090.2 (M+H); compound LE24: yellow solid, ESI-MS m/z: 1122.1 (M+H).

### Embodiment 6 Synthesis of LE12

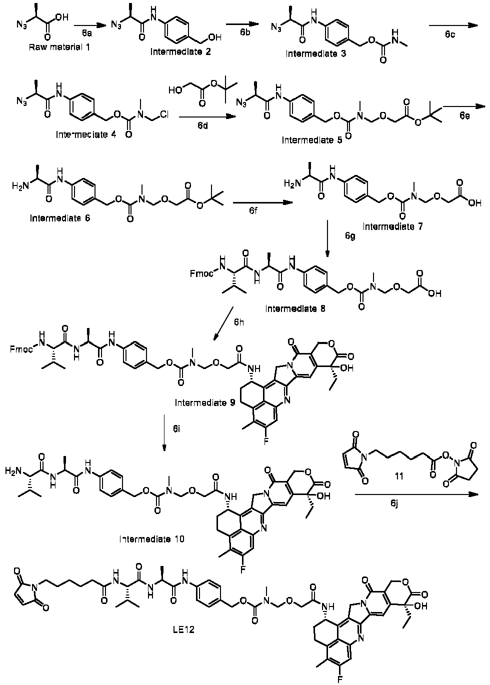

### Synthesis of Intermediate 2:

(S)-2-azidopropanoic acid (10 g, 86.9 mmol) and 4-aminobenzyl alcohol (21.40 g, 173.8 mmol) were dissolved in 300 mL of a mixed solvent of dichloromethane and methanol (volume ratio: 2:1), 2-ethoxy-1-ethoxycarbonyl-1,2-dihydroquinoline (21.49 g, 86.9 mmol) was added, the reaction was reacted at room temperature for 5 hours, the solvent was evaporated under reduced pressure, and then the obtained residue was purified by silica gel column chromatography [dichloromethane: ethyl acetate=1:1 (v/v)] to obtain Intermediate 2 (16.3 g, yield: 85%), ESI-MS m/z: 221 (M+H).

### Synthesis of Intermediate 3:

Intermediate 2 (15 g, 68.2 mmol) and bis(p-nitrophenyl) carbonate (22.82 g, 75.02 mmol) were mixed and dissolved in 200 mL of anhydrous N,N-dimethylformamide, and 25 mL of triethylamine was added, the mixture was reacted at room temperature for 2 hours. After the completion of the reaction of the raw materials was monitored by liquid chromatography-mass spectroscopy, methylamine hydrochloride (6.91 g, 102.3 mmol) was added, and the reaction was continued for 1 hour. After the reaction was completed, most of the solvent was removed by distillation under reduced pressure, and then 200 mL of water and 200 mL of ethyl acetate were added. After the phases were separated, the organic phase was collected, dried and concentrated. The obtained crude product was purified by silica gel column chromatography [dichloromethane: ethyl acetate=10 :1 (v/v)] to obtain Intermediate 3 (18.9 g, yield: 100%), ESI-MS m/z: 278 (M+H).

### Synthesis of Intermediate 5:

Intermediate 3 (10 g, 36.1 mmol) and paraformaldehyde (1.63 g, 54.2 mmol) were dissolved in 150 mL of anhydrous dichloromethane, trimethylchlorosilane (6.28 g, 57.76 mmol) was slowly added, and the mixture was reacted at room temperature for 2 hours to obtain the solution of crude product of Intermediate 4. After the reaction mixture was sampled, quenched by adding methanol, and the reaction was monitored by LC/MS. After the reaction was completed, the reaction solution was filtered and then *tert-butyl* glycolate (9.54 g, 72.2 mmol) and triethylamine (10 mL, 72.2 mmol) were added to the filtrate, and the mixture was reacted at room temperature for 2 hours. After the reaction was completed, most of the solvent was removed by distillation under reduced pressure, and the obtained crude product was purified by silica gel column chromatography [petroleum ether: ethyl acetate=3:1 (v/v)] to obtain Intermediate 5 (11.2 g, yield: 74%), ESI- MS m/z: 422 (M+H).

### Synthesis of Intermediate 6:

Intermediate 5 (10 g, 23.8 mmol) was dissolved in 80 mL of anhydrous tetrahydrofuran, 80 mL of water was added, then tris(2-carboxyethyl)phosphine hydrochloride (13.6 g, 47.6 mmol) was added, and the mixture was reacted at room temperature for 4 hours. After the reaction was completed, the tetrahydrofuran was removed by distillation under reduced pressure, and then the residue was extracted with ethyl acetate. After the obtained organic phase was dried, the solvent was evaporated under reduced pressure, and the obtained crude product was purified by silica gel column chromatography [dichloromethane: methanol = 10:1 (v/v)] to obtain Intermediate 6 (8.1 g, yield: 86%), ESI-MS m/z: 396 (M+H).

### Synthesis of Intermediate 8:

Intermediate 6 (5 g, 12.7 mmol) was dissolved in 60 mL of a mixed solvent of dichloromethane and methanol (v/v=2:1), 3 mL of trifluoroacetic acid was slowly added, and the mixture was reacted at room temperature for 30 minutes. After the reaction was completed, equal volumes of water and ethyl acetate were added, the organic phase was dried and then concentrated, and the obtained crude product was directly used in the next step.

The crude product obtained in the above step was dissolved in 50 mL of anhydrous N,N-dimethylformamide, Fmoc-valine hydroxysuccinimide ester (8.3 g, 19.1 mmol), triethylamine (5 mL) were added, the mixture was reacted at room temperature for 2 hours. After the reaction was completed, most of the solvent was removed by distillation under reduced pressure. The obtained crude product was purified by silica gel column chromatography [dichloromethane: methanol=10:1 (v/v)] to obtain Intermediate 8 (5.4 g, yield: 64%), ESI-MS m/z: 661 (M+H).

### Synthesis of Intermediate 9:

Intermediate 8 (1 g, 1.5 mmol) and Exatecan methanesulfonate (0.568 g, 1 mmol) were mixed in 30 mL of anhydrous N,N-dimethylformamide, and 2-(7-azabenzotriazol-1-yl)-N,N,N',N'-tetramethyluronium hexafluorophosphate (1.14 g, 3.0 mmol), 2 mL of triethylamine were added, the mixture was reacted at room temperature for 2 h. After the reaction was completed, the solvent was removed by distillation under reduced pressure, and the obtained crude product was purified by silica gel column chromatography [chloroform: methanol=10:1 (v/v)] to obtain Intermediate 9 (0.94 g, yield 87%), ESI-MS m/z: 1078 (M+H).

### Synthesis of compound LE12:

Intermediate 9 (1 g, 0.929 mmol) was dissolved in 20 mL of anhydrous DMF, 0.5 mL of 1,8-diazabicycloundec-7-ene was added, and the mixture was reacted at room temperature for 1 hour. After the reaction of the raw materials was completed, 6-(maleimido)hexanoic acid succinimidyl ester (428.5 mg, 1.39 mmol) was directly added, and the mixture was stirred at room temperature for 1 hour. The solvent was removed by distillation under reduced pressure, and the obtained crude product was purified by silica gel column chromatography [chloroform: methanol=8:1 (v/v)] to obtain the title compound (0.7 g, yield: 73%), ESI-MS m/z: 1035 (M +H).

### Embodiment 7 Synthesis of LE13-LE20

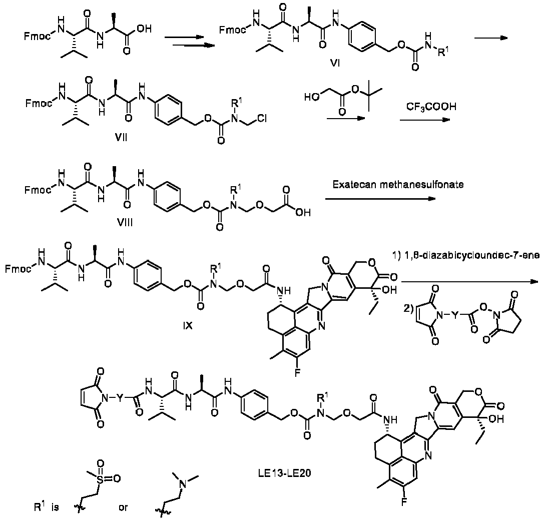

Intermediate VI could be prepared by using Fmoc-L-valine-L-alanine as the starting raw material, referring to steps 6a and 6b in the synthesis method of Intermediate 3 in Embodiment 6, wherein the methylamine hydrochloride in step 6b was replaced by corresponding commercially available amino compound. The subsequent steps were started from Intermediate VI, according to the same method as steps 6c, 6d, 6f and 6h in Embodiment 6 to obtain Intermediate IX similar to Intermediate 9, and then according to the same steps as steps 6i and 6j in Embodiment 6 to treat, remove the amino protective group, and then the residue was condensed with commercially available different maleimides to obtain the final product. The amino compounds and maleimide structures used are shown in Table 4. Compound LE13: pale yellow solid, ESI-MS m/z: 1106.5 (M+H); compound LE14: pale yellow solid, ESI-MS m/z: 1141.4 (M+H); compound LE15: off-white solid, ESI- MS m/z: 1121.2 (M+H); compound LE16: pale yellow solid, ESI-MS m/z: 1167.1 (M+H); compound LE17: yellow solid, ESI-MS m/z: 1132.3 (M+ H); compound LE18: pale yellow solid, ESI-MS m/z: 1305.4 (M+H); compound LE19: pale yellow solid, ESI-MS m/z: 1307.4 (M+H); compound LE20: pale yellow solid, ESI-MS m/z: 1337.6 (M+H).

**Table 4. Intermediates used in the synthesis of LE13-LE20**

| Product | R¹ | Amino compound | Maleimide structure | 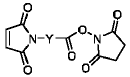 |
|---|---|---|---|---|
| LE13 | 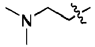 | Dimethyl ethylamine hydrochloride | 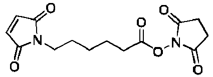 | |
| LE14 | 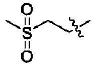 | Methylsulfone ethylamine hydrochloride | 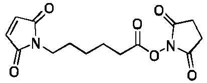 | |
| LE15 | 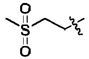 | Methylsulfone ethylamine hydrochloride | 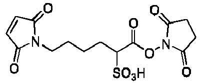 | |
| LE16 | 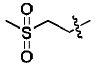 | Methylsulfone ethylamine hydrochloride | 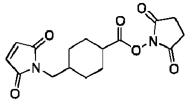 | |
| LE17 | 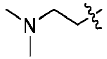 | Dimethyl ethylamine hydrochloride | 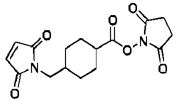 | |
| LE18 | 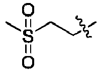 | Methylsulfone ethylamine hydrochloride | 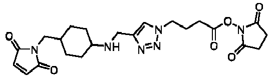 | |
| LE19 | 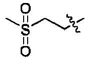 | Methylsulfone ethylamine hydrochloride | 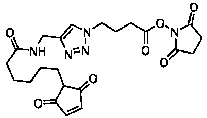 | |
| LE20 | 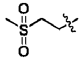 | Methylsulfone ethylamine hydrochloride | 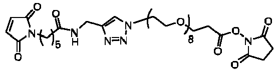 | |

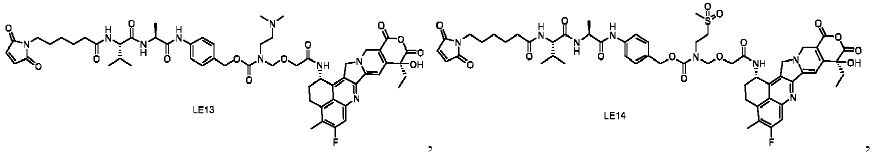

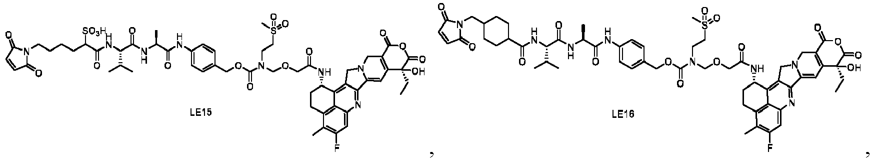

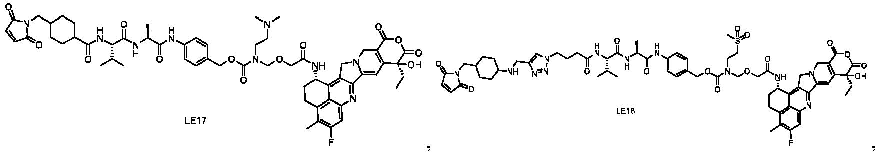

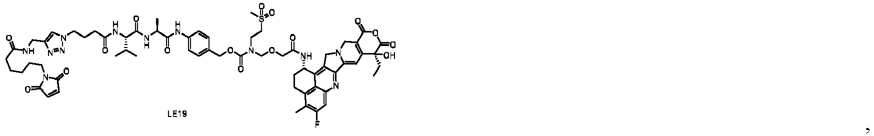

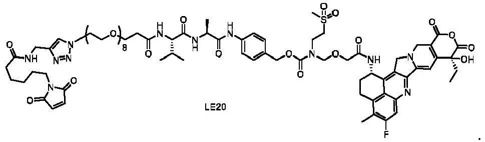

### Embodiment 8 Synthesis of LE21-LE22

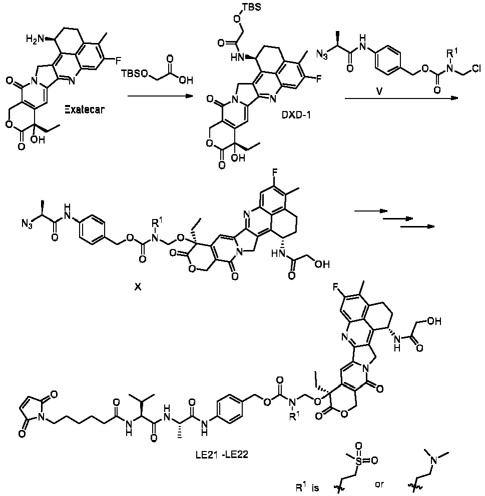

### Synthesis of compound DXD-1

Commercially available Exatecan methanesulfonate (0.568 g, 1 mmol) and 2-(*tert-*butyldimethylsiloxy)acetic acid (CAS: 105459-05-0, 0.38 g, 2 mmol) were dissolved in 20 mL of anhydrous dichloromethane, condensing agent HATU (0.76 g, 2 mmol) and 1 mL of pyridine were added, and the mixture was stirred at room temperature for 2 hours. After the reaction was completed, the solvent was evaporated to dryness under reduced pressure, and the obtained crude product was purified by column chromatography [dichloromethane: methanol=50:1 (v/v)] to obtain the title compound DXD-1 (0.55 g, yield: 90%), ESI-MS m/z: 608.1 (M+H). ¹H NMR (400 MHz, CDCl₃) δ 7.73 (d, *J* = 10.5 Hz, 1H), 7.64 (s, 1H), 7.05 (d, *J* = 9.2 Hz, 1H), 5.80 - 5.62 (m, 2H), 5.41 - 5.14 (m, 4H), 4.29 - 4.15 (m, 2H), 4.08-4.03 (m, 1H), 3.27 - 3.07 (m, 2H), 2.45 (s, 3H), 2.38 - 2.28 (m, 2H), 1.96 - 1.81 (m, 2H), 1.04 (t, *J=* 7.4 Hz, 3H), 0.80 (s, 9H), 0.11 (s, 3H), 0.03 (s, 3H).

### Preparation of Intermediate V

Intermediate V could be prepared by referring to the preparation method of compound 4 in Embodiment 6, wherein the methylamine hydrochloride in step 6b was replaced with the corresponding commercially available amino compound.

### Synthesis of LE21-LE22

Intermediate V was reacted with DXD-1, and then the residue was treated with 10% trifluoroacetic acid/dichloromethane solution to obtain Intermediate X, and then Intermediate X was reacted with reference to the subsequent steps 6e, 6g, 6i and 6j of compound 5 in Embodiment 6: Intermediate X was reduced to obtain an amino compound, the obtained amino compound was condensed with Fmoc-valine hydroxysuccinimide ester, and then the Fmoc protecting group of the amino group in the obtained product was removed, and the obtained amino product was reacted with 6-(maleimido)hexanoic acid succinimidyl ester to obtain the final product. Compound LE21: yellow solid, ESI-MS m/z: 1141.2 (M+H); compound LE22: yellow solid, ESI-MS m/z: 1106.6 (M+H).

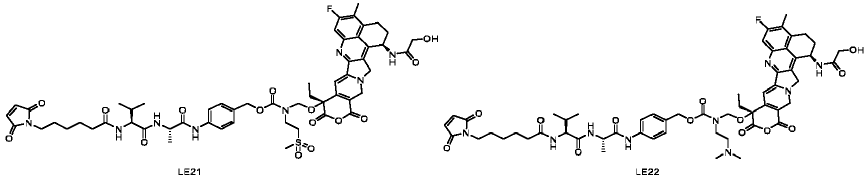

### Embodiment 9: synthesis of compound LE13

Compound LE13 could be prepared according to the following synthetic route:

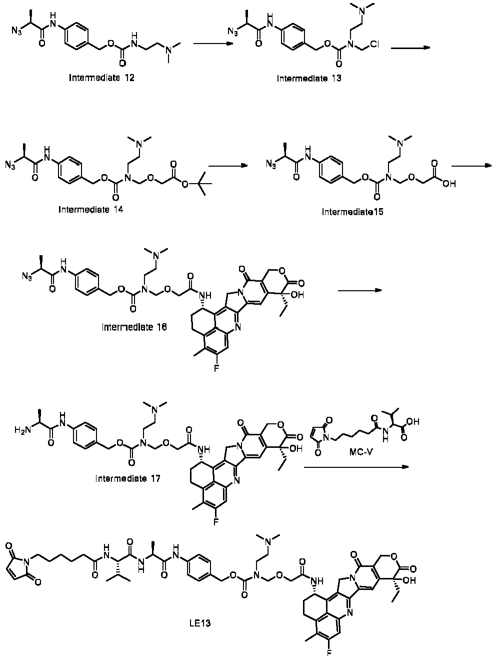

The specific preparation steps were as follows:

### Synthesis of Intermediate 14

Commercially available Intermediate 12 (267 mg, 0.8 mmol) and paraformaldehyde (50 mg, 1.6 mmol) were dissolved in 20 mL of anhydrous dichloromethane, and trimethylchlorosilane (0.3 mL, 3.4 mmol) was slowly added, the mixture was reacted at room temperature for 2 hours after the addition was completed. Then the reaction was sampled and quenched by adding methanol to monitor the reaction by liquid chromatography mass spectrometry. After the reaction was completed, the reaction solution was filtered, and then *tert-butyl* glycolate (211 mg, 1.6 mmol) and 0.5 mL of pempidine were added to the filtrate, and the mixture was reacted at room temperature for about 2 hours. After the reaction was completed, most of the solvent was removed by distillation under reduced pressure. The crude product was purified by silica gel column chromatography [dichloromethane: methanol=20:1 (v/v)] to obtain Intermediate 14 (260 mg, yield: 68%), ESI-MS m/z: 479 (M+H).

### Synthesis of Intermediate 15

Intermediate 14 (238 mg, 0.50 mmol) was dissolved in 6 mL of a mixed solvent of dichloromethane and methanol (v/v=2:1), 0.3 mL of trifluoroacetic acid was slowly added, and the mixture was reacted at room temperature for 30 minutes. After the reaction was completed, equal volumes of water and ethyl acetate were added, the organic phase was dried and concentrated, and the obtained crude product was directly used in the next step.

### Synthesis of Intermediate 16

The crude product obtained in the above step and Exatecan methanesulfonate (170mg, 0.30 mmol) were mixed in 5 mL of anhydrous N,N-dimethylformamide, and 2-(7-azabenzotriazol-1-yl)-N,N,N',N'-tetramethyluronium hexafluorophosphate (341 mg, 0.90 mmol), 0.60 mL of triethylamine were added, the mixture was reacted at room temperature for 2 h. After the reaction was completed, the solvent was removed by distillation under reduced pressure, and the obtained crude product was purified by silica gel column chromatography [chloroform: methanol=10:1 (v/v)] to obtain Intermediate 16 (210 mg, 83%), ESI-MS m/z: 840 (M+H).

### Synthesis of Intermediate 17

Intermediate 16 (100 mg, 0.12 mmol) was dissolved in 15 mL of anhydrous tetrahydrofuran, 3 mL of water was added, and then 0.3 mL of 1 mol/L triethylphosphine aqueous solution was added, and the mixture was reacted at room temperature for 4 hours. The reaction was monitored until the reaction was completed, the reaction solution was distilled under reduced pressure to remove tetrahydrofuran, sodium bicarbonate was added to the remaining aqueous solution to adjust the pH to neutral, and then dichloromethane was added for extraction. The obtained organic phase was dried and the solvent was evaporated under reduced pressure, the crude product was purified by silica gel column chromatography [dichloromethane: methanol = 10:1 (v/v)] to obtain Intermediate 17 (69 mg, yield: 71%), ESI-MS m/z: 814 (M+H).

### Synthesis of LE13

Intermediate 17 (120 mg, 0.15 mmol) obtained in the previous step and the commercially available raw material MC-V (102 mg, 0.33 mmol) were mixed in 40 mL of dichloromethane, and the condensing agent 2-ethoxy-1-ethoxycarbonyl-1,2-dihydroquinoline (82 mg, 0.33 mmol) was added and the mixture was reacted at room temperature overnight. After the reaction was completed, the solvent was evaporated to dryness under reduced pressure. The obtained crude productwas purified by silica gel column chromatography [dichloromethane: methanol=10 :1 (v/v)] to obtain compound LE13 (116 mg, yield: 70%), ESI-MS m/z: 1106.5 (M+H).

### Embodiment 10: Synthesis of compound LE14

Compound LE14 could be prepared according to the following synthetic route:

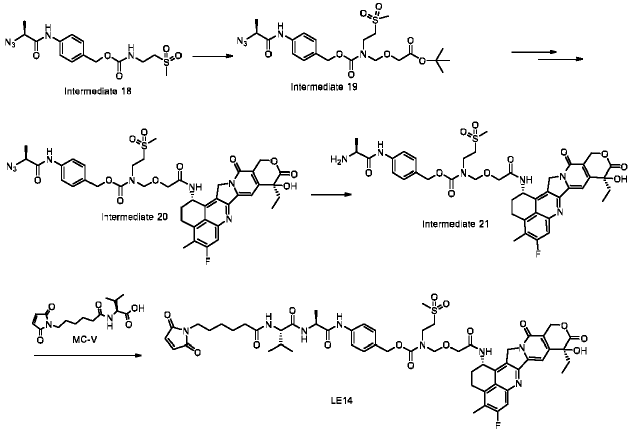

The specific preparation steps were as follows:

### Synthesis of Intermediate 19

Commercially available Intermediate 18 (300mg, 0.8mmol) and paraformaldehyde (50 mg, 1.6 mmol) were dissolved in 20 mL of anhydrous dichloromethane, and trimethylchlorosilane (0.3 mL, 3.4 mmol) was slowly added, the mixture was reacted at room temperature for 2 hours. Then the reaction was sampled and quenched by adding methanol to monitor the reaction by liquid chromatography mass spectrometry. After the reaction was completed, the reaction solution was filtered, and then *tert-butyl* glycolate (211 mg, 1.6 mmol) and triethylamine (0.22 m, 1.6 mmol) were added to the filtrate, and the mixture was reacted at room temperature for about 2 hours. After the reaction was completed, most of the solvent was removed by distillation under reduced pressure. The obtained crude product was purified by silica gel column chromatography [dichloromethane: methanol=20:1 (v/v)] to obtain Intermediate 19 (349 mg, yield 85%), ESI-MS m/z: 514 (M+H), ¹H NMR (400 MHz, CDCl₃) δ 8.13 (s, 1H), 7.56 (d, *J* = 7.5 Hz, 2H), 7.35 (s, 2H), 5.14 (s, 2H), 4.91 (s, 2H), 4.25 (q, *J* = 7.1 Hz, 1H), 3.99 (d, *J* = 42.5 Hz, 2H), 3.85 (t, *J* = 6.2 Hz, 2H), 3.40 (dd, *J* = 18.5, 7.6 Hz, 2H), 2.89 (d, *J* = 48.6 Hz, 3H), 1.65 (d, *J* = 6.8 Hz, 3H), 1.46 (s, 9H).

### Synthesis of Intermediate 20

Intermediate 19 (257 mg, 0.50 mmol) was dissolved in 6 mL of a mixed solvent of dichloromethane and methanol (v/v=2:1), 0.3 mL of trifluoroacetic acid was slowly added, and the mixture was reacted at room temperature for 30 minutes. After the reaction was completed, equal volumes of water and ethyl acetate were added, the organic phase was dried and concentrated, and the obtained crude product was directly used in the next step.

The obtained crude product and Exatecan methanesulfonate (170mg, 0.30 mmol) were mixed in 5 mL of anhydrous N,N-dimethylformamide, and 2-(7-azabenzotriazol-1-yl)-N,N,N',N'-tetramethyluronium hexafluorophosphate (341 mg, 0.90 mmol), 0.60 mL of triethylamine were added, the mixture was reacted at room temperature for 2 h. After the reaction was completed, the solvent was removed by distillation under reduced pressure, and the obtained crude product was purified by silica gel column chromatography [chloroform: methanol=20:1 (v/v)] to obtain Intermediate 20 (212 mg, yield: 81%), ESI-MS m/z: 875 (M+H). ¹H NMR (400 MHz, CDCl₃) δ 8.27 (d, J= 34.7 Hz, 1H), 7.63 - 7.35 (m, 5H), 7.21 - 7.10 (m, 1H), 5.71 - 5.48 (m, 2H), 5.24 - 4.95 (m, 3H), 4.95 - 4.72 (m, 4H), 4.45 (s, 1H), 4.33 - 3.97 (m, 3H), 3.75 (s, 2H), 3.39 - 2.99 (m, 4H), 2.76 (d, *J* = 15.3 Hz, 3H), 2.43 - 2.15 (m, 5H), 2.04 (s, 1H), 1.94 - 1.75 (m, 2H), 1.62 (d, *J* = 6.6 Hz, 3H), 1.11 - 0.89 (m, 3H).

### Synthesis of Intermediate 21

Intermediate 20 (77 mg, 0.09 mmol) was dissolved in 12 mL of anhydrous tetrahydrofuran, 3 mL of water was added, and then 0.3 mL of 1 mol/L triethylphosphine aqueous solution was added, and the mixture was reacted at room temperature for 4 hours. After the reaction was completed, tetrahydrofuran was removed by distillation under reduced pressure, sodium bicarbonate was added to the remaining aqueous solution to adjust the pH to neutral, and then dichloromethane was added for extraction. The obtained organic phase was dried and the solvent was evaporated under reduced pressure, the obtained crude product was purified by silica gel column chromatography [dichloromethane: methanol = 10:1 (v/v)] to obtain Intermediate 21 (53 mg, yield: 69%), ESI-MS m/z: 849 (M+H). ¹H NMR (400 MHz, DMSO) δ 8.52 (s, 1H), 7.79 (d, *J* = 10.8 Hz, 1H), 7.67 - 7.55 (m, 2H), 7.47 - 7.21 (m, 3H), 6.51 (s, 1H), 5.60 (s, 1H), 5.52 - 5.32 (m, 2H), 5.30 - 5.11 (m, 2H), 5.11 - 4.94 (m, 2H), 4.94 - 4.74 (m, 2H), 4.02 (s, 2H), 3.81 - 3.66 (m, 2H), 3.60 - 3.35 (m, 4H), 3.24 - 3.08 (m, 2H), 2.94 (d, J= 30.8 Hz, 3H), 2.39 (s, 3H), 2.28 - 2.04 (m,2H), 2.00 - 1.73 (m, 2H), 1.22 (d, *J* = 6.6 Hz, 3H), 0.96 - 0.70 (m, 3H).

### Synthesis of compound LE14

Intermediate 21 (134 mg, 0.16 mmol) and commercially available raw material MC-V (102 mg, 0.33 mmol) were mixed in 40 mL of dichloromethane, and the condensing agent 2-ethoxy-1-ethoxycarbonyl-1,2-dihydroquinoline (82 mg, 0.33 mmol) was added and the mixture was reacted at room temperature overnight. After the reaction was completed, the solvent was evaporated to dryness under reduced pressure. The crude product was purified by silica gel column chromatography [dichloromethane: methanol=10 :1 (v/v)] to obtain compound LE14 (137 mg, yield: 75%), ESI-MS m/z: 1141.4 (M+H). ¹H NMR (400 MHz, DMSO) δ 9.97 (s, 1H), 8.52 (s, 1H), 8.27 - 8.09 (m, 1H), 7.88 - 7.70 (m, 2H), 7.63 - 7.51 (m, 2H), 7.28 (s, 3H), 6.99 (s, 2H), 6.51 (s, 1H), 5.59 (s, 1H), 5.50 - 5.32 (m, 2H), 5.17 (s, 2H), 4.98 (s, 2H), 4.85 (d, *J* = 17.3 Hz, 2H), 4.43 - 4.33 (m, 1H), 4.21 - 4.12 (m, 1H), 4.03 (s, 2H), 3.74 - 3.64 (m, 2H), 3.20 - 3.03 (m, 3H), 3.02 - 2.84 (m, 4H), 2.36 (s, 3H), 2.23 - 2.09 (m, 4H), 2.01 - 1.90 (m, 1H), 1.90 - 1.78 (m, 2H), 1.55 - 1.39 (m, 4H), 1.30 (d, *J* = 6.7 Hz, 3H), 1.23 - 1.11 (m, 2H), 0.93 - 0.77 (m, 9H).

### Embodiment 11: synthesis of compound LS13

Referring to the synthesis method of LE14 in Embodiment 7, after SN-38 (7-ethyl-10-hydroxycamptothecin) was reacted with Intermediate VII (R¹ is methylsulfone ethyl), the compound LS13 was obtained by deprotection, condensation and other steps: ¹H NMR (400 MHz, DMSO) δ 9.92 (d, *J* = 22.4 Hz, 1H), 8.14 (s, 1H), 8.08 (d, *J* = 9.1 Hz, 1H), 7.81 (d, *J* = 8.0 Hz, 1H), 7.70 - 7.50 (m, 3H), 7.47 (d, *J* = 7.2 Hz, 1H), 7.34 (d, *J* = 7.2 Hz, 1H), 7.27 (s, 1H), 7.20 (s, 1H), 6.98 (s, 2H), 6.51 (s, 1H), 5.61 (s, 2H), 5.48 - 5.35 (m, 2H), 5.27 (s, 2H), 5.10 (d, *J* = 20.6 Hz, 2H), 4.36 (s, 1H), 4.21 - 4.07 (m, 1H), 3.84 (s, 2H), 3.48 (s, 2H), 3.21 - 2.92 (m, 6H), 2.25 - 2.04 (m, 2H), 2.04 - 1.78 (m, 3H), 1.55 - 1.36 (m, 4H), 1.36 - 1.10 (m, 9H), 0.95 - 0.71 (m, 10H).

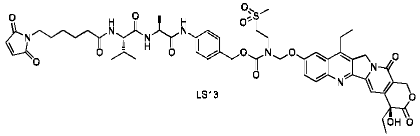

### Embodiment 12: general method for connecting linker-drug conjugates to antibodies

The anti-B7-H3 antibody P2E5, anti-Claudin18.2 antibody IMAB362, and anti-HER2 antibody Trastuzumab (concentration was 15 mg/mL) were replaced into 50 mM PB/1.0 mM EDTA buffer (pH 7.0) by a G25 desalting column, respectively. 12 equivalents of TECP was added and the mixture was stirred at 37°C for 2 hours to fully open the disulfide bonds between the antibody chains. Then phosphoric acid was used to adjust the pH of the reduced antibody solution to 6.0, and the temperature of the water bath was lowered to 25°C for coupling reaction. The linker-drug conjugate prepared in Embodiments 1-11 and GGFG-Dxd were respectively dissolved in DMSO, and 12 equivalents of the linker-drug conjugate was drawn and added dropwise into the reduced antibody solution, and DMSO was added until final concentration of the solution was 10% (v/v), the mixture was stirred and reacted at 25°C for 0.5 hours. After the reaction was completed, the sample was filtered with a 0.22 µm membrane. Uncoupled small molecules were purified and removed by a tangential flow ultrafiltration system. The buffer was 50 mM PB/1.0 mM EDTA solution (pH 6.0). After purification, sucrose (final concentration was 6%) was added, and the mixture was stored in a refrigerator at -20°C. The UV method was used to measure the absorbance values at 280 nm and 370 nm respectively, and the DAR value was calculated. The results are shown in Table 5 below. The amino acid sequence of the light chain in the anti-HER2 antibody Trastuzumab is shown in SEQ ID No. 5 in the sequence listing, and the amino acid sequence of the heavy chain in the anti-HER2 antibody Trastuzumab is shown in SEQ ID No. 6 in the sequence listing. The amino acid sequence of the light chain in the anti-B7-H3 antibody P2E5 is shown in SEQ ID No. 7 in the sequence listing, and the amino acid sequence of the heavy chain in the anti-B7-H3 antibody P2E5 is shown in SEQ ID No. 8 in the sequence listing. The amino acid sequence of the light chain in the anti-Claudin 18.2 antibody IMAB362 is shown in SEQ ID No. 1 in the sequence table, and the amino acid sequence of the heavy chain in the anti-Claudin 18.2 antibody IMAB362 is shown in SEQ ID No. 2 in the sequence listing.

**Table 5 DAR values of different antibody drug conjugates (ADC) measured under UV method**

| No. ADC | Antibody | Linker-drug conjugate | DAR value |
|---|---|---|---|
| ADC-8201 | Trastuzumab | GGFG-Dxd | 7.6 |
| ADC001 | Trastuzumab | LE01 | 7.6 |
| ADC002 | Trastuzumab | LE02 | 7.8 |
| ADC003 | Trastuzumab | LE03 | 7.4 |
| ADC004 | Trastuzumab | LE04 | 7.5 |
| ADC005 | Trastuzumab | LE06 | 7.5 |
| ADC006 | Trastuzumab | LE10 | 7.3 |
| ADC007 | Trastuzumab | LE13 | 7.7 |
| ADC008 | Trastuzumab | LE15 | 7.7 |
| ADC009 | Trastuzumab | LE18 | 7.8 |
| ADC010 | Trastuzumab | LE19 | 7.5 |
| ADC011 | Trastuzumab | LE20 | 7.5 |
| ADC029 | Trastuzumab | LE14 | 8.0 |
| ADC030 | Trastuzumab | LS13 | 8.0 |
| ADC033 | Trastuzumab | LE12 | 7.8 |
| ADC012 | P2E5 | LE01 | 7.6 |
| ADC013 | P2E5 | LE02 | 7.4 |
| ADC014 | P2E5 | LE03 | 7.6 |
| ADC015 | P2E5 | LE04 | 7.5 |
| ADC016 | P2E5 | LE06 | 7.4 |
| ADC017 | P2E5 | LE10 | 7.5 |
| ADC018 | P2E5 | LE13 | 7.6 |
| ADC019 | P2E5 | LE15 | 7.6 |
| ADC020 | P2E5 | LE01 | 7.5 |
| ADC031 | P2E5 | LE14 | 7.8 |
| ADC034 | P2E5 | LE12 | 7.7 |
| ADC021 | IMAB362 | LE02 | 7.5 |
| ADC022 | IMAB362 | LE03 | 7.5 |
| ADC023 | IMAB362 | LE04 | 7.7 |
| ADC024 | IMAB362 | LE06 | 7.4 |
| ADC025 | IMAB362 | LE10 | 7.6 |
| ADC026 | IMAB362 | LE13 | 7.8 |
| ADC027 | IMAB362 | LE15 | 7.5 |
| ADC028 | IMAB362 | LE18 | 7.6 |
| ADC032 | IMAB362 | LE14 | 7.6 |
| ADC035 | IMAB362 | LE12 | 7.5 |

### Effect Embodiment 1: In vitro cell activity test

The HEK293 cells stably transfected with high expression of Claudin 18.2, SK-BR-3 and NCI-N87 cells with high expression of HER2 were selected as the cell lines for *in vitro* activity detection in this experiment. NCI-N87 cells also highly expressed B7-H3. The dose effect of different antibody drug conjugates on cell killing were observed. The seed plate density of each cell was preliminarily selected: 2×10³ cells/well, and the cytotoxic activity was tested after 16 to 24 hours; secondly, the final concentration of antibody drug conjugate prepared in Embodiment 12 after loading was tested, and the initial concentration was set at 5000 nM. Series of 10 concentrations was designed in 5000-0.006 nM (4-10 times diluted), the killing (or inhibition) changes in 96 hours was observed, chemiluminescence staining was performed by CellTiter-Glo^{®} Luminescent Cell Viability Assay, IC₅₀ was calculated after reading the fluorescence data. From the activity test results (see Table 6), all ADCs show certain anti-tumor activity, and the activity of some ADCs are better than ADC-8201.

**Table 6 In vitro cytotoxic activity of different ADCs**

| No. ADC | IC 50 (nM) | | |
|---|---|---|---|
| | SK-BR-3 cell | NCI-N87 cell | HEK293 cell |
| ADC-8201 | 0.729 | 0.586 | greater than 5 µM |
| ADC001 | 0.535 | 0.651 | greater than5 µM |
| ADC002 | 0.683 | 0.468 | greater than5 µM |
| ADC003 | 0.411 | 0.510 | greater than 5 µM |
| ADC004 | 0.951 | 1.256 | greater than 5 µM |
| ADC005 | 5.609 | 3.595 | greater than 5 µM |
| ADC006 | 0.362 | 0.419 | greater than 5 µM |
| ADC007 | 0.185 | 0.278 | greater than 5 µM |
| ADC008 | 0.103 | 0.169 | greater than 5 µM |
| ADC009 | 0.297 | 0.190 | greater than 5 µM |
| ADC010 | 0.334 | 0.624 | greater than 5 µM |
| ADC011 | 0.621 | 0.323 | greater than 5 µM |
| ADC029 | 0.480 | 0.641 | Not tested |
| ADC030 | 15 µM ^{∗} | 11 µM ^{∗} | Not tested |
| ADC033 | 0.615 | 0.701 | Not tested |
| ADC012 | Not tested | 1.690 | greater than 5 µM |
| ADC013 | Not tested | 3.158 | greater than 5 µM |
| ADC014 | Not tested | 2.160 | greater than 5 µM |
| ADC015 | Not tested | 1.578 | greater than 5 µM |
| ADC016 | Not tested | 1.268 | greater than 5 µM |
| ADC017 | Not tested | 1.463 | greater than 5 µM |
| ADC018 | Not tested | 10.361 nM | greater than 5 µM |
| ADC019 | Not tested | 2.891 | greater than 5 µM |
| ADC020 | Not tested | 0.863 | greater than 5 µM |
| ADC031 | Not tested | 0.732 | Not tested |
| ADC034 | Not tested | 0.624 | Not tested |
| ADC021 | Not tested | greater than 5 µM | 0.278 |
| ADC022 | Not tested | greater than 5 µM | 0.676 |
| ADC023 | Not tested | greater than 5 µM | 0.335 |
| ADC024 | Not tested | greater than 5 µM | 0.125 |
| ADC025 | Not tested | greater than 5 µM | 0.924 |
| ADC026 | Not tested | greater than 5 µM | 0.115 |
| ADC027 | Not tested | greater than 5 µM | 0.364 |
| ADC028 | Not tested | greater than 5 µM | 0.824 |
| ADC032 | Not tested | greater than 5 µM | 0.391 |
| ADC035 | Not tested | Not tested | 0.352 |

| | | | |
|---|---|---|---|
| ^{∗}: This data was obtained according to the same experimental operation as that of this effect embodiment based on the initial concentration of 30 µM. | | | |

### Effect Embodiment 2: In vitro plasma stability test

This embodiment evaluates the stability of the antibody drug conjugate of Embodiment 12 in human plasma. Specifically, in this embodiment, part of the antibody drug conjugates of Embodiment 12 were added to human plasma and placed in a 37°C water bath for 1, 3, 7, 14, 21, 28 days, internal standard (Exatecan was used as an internal standard substance) was added, and the mixture was extracted and then the release amount of free drug was detected by high performance liquid chromatography. The results are shown in Table 7.

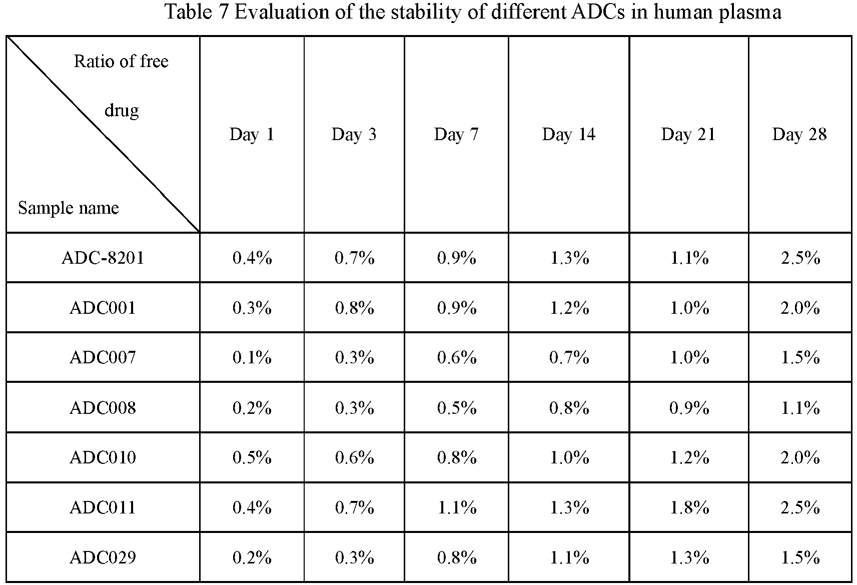

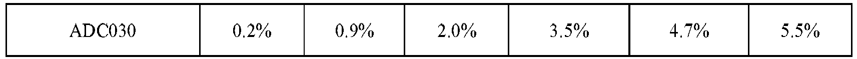

Plasma stability results show that the ADC stability obtained by the new technical solution is not inferior to ADC-8201, and some of them are more stable. At the same time, the above activity test results also prove that the activities of some of the newly obtained ADC are better than ADC-8201.

### Effect Embodiment 3: Evaluation of different linker-drug conjugates

When the linker-drug conjugate was coupled with different antibodies, the universality of the linker-drug conjugate was reflected in the aggregates, recovery rate and whether the precipitation was occurred, etc. Although Trastuzumab did not produce precipitation during the coupling process with the linker-drug conjugate of ADC-8201 (GGFG-Dxd), among the antibodies listed in the present disclosure, the P2E5 and IMAB362 antibodies produced precipitation during the coupling process with GGFG-Dxd. Therefore, P2E5, IMAB362 and Trastuzumab were selected to evaluate universality of the linker-drug conjugate of the present disclosure in this experiment, the coupling reactions were performed according to the method in Embodiment 12, the samples were prepared according to the highest DAR (i.e., excessive coupling) and the results are shown in Table 8.

**Table 8 The situations of different linker-drug conjugates when coupled with different antibodies**

| Linker-drug conjugate | Coupling situation of P2E5 | | | Coupling situation of IMAB362 | | | Coupling situation of Trastuzumab | | |
|---|---|---|---|---|---|---|---|---|---|
| | Whether precipitat ion | Ratio of aggrega tes | Recover y rate | Whethe r precipit ation | Ratio of aggrega tes | Recover y rate | Wheth er precipi tation | Ratio of aggreg ates | Recov ery rate |
| GGFG-Dxd | precipitat ion | 20% | 30% | precipit ation | 35% | 32% | No | 0.1% | 90% |
| LE01 | No | 2.1% | / | No | 2.0% | / | No | 0.2% | 80% |
| LE02 | No | 1.3% | / | No | 1.8% | / | No | 0.2% | 80% |
| LE03 | No | 1.1% | 80% | No | 1.5% | 86% | No | 0.2% | 87% |
| LE04 | No | 2.5% | 76% | No | 2.1% | 85% | No | 0.1% | 88% |
| LE05 | No | 1.6% | 80% | No | 2.3% | 82% | No | 0.1% | 86% |
| LE06 | No | 1.2% | 72% | No | 1.7% | / | No | 0.5% | 85% |
| LE07 | No | 1.3% | / | No | 1.6% | / | No | 0.5% | / |
| LE08 | No | 2.2% | / | No | 2.3% | / | No | 0.2% | / |
| LE09 | No | 2.6% | 90% | No | 1.5% | 90% | No | 0.1% | / |
| LE10 | No | 2.5% | 85% | No | 1.2% | 88% | No | 0.1% | 90% |
| LE11 | No | 3.1% | 79% | No | 1.4% | 82% | No | 0.2% | 90% |
| LE12 | No | 1.0% | 83% | No | 2.9% | / | No | 0.3% | 86% |
| LE13 | No | 1.5% | / | No | 2.3% | / | No | 0.1% | 85% |
| LE14 | No | 1.3% | / | No | 2.5% | / | No | 0.3% | 91% |
| LE15 | No | 2.1% | / | No | 1.6% | / | No | 0.2% | 90% |
| LE16 | No | 2.0% | / | No | 1.5% | / | No | 0.5% | 80% |
| LE17 | No | 1.5% | / | No | 1.6% | / | No | 0.1% | 82% |
| LE18 | No | 1.6% | / | No | 2.1% | / | No | 0.1% | 95% |
| LE19 | No | 1.2% | / | No | 1.4% | / | No | 0.2% | 90% |
| LE20 | No | 3.0% | / | No | 1.2% | / | No | 0.2% | 80% |
| LE21 | No | 2.2% | / | No | 2.3% | / | No | 0.2% | 90% |
| LE22 | No | 2.1% | / | No | 1.5% | / | No | 0.5% | 90% |
| LE23 | No | 1.9% | / | No | 1.6% | / | No | 0.1% | 88% |
| LE24 | No | 1.2% | / | No | 2.0% | / | No | 0.1% | 92% |

| | | | | | | | | | |
|---|---|---|---|---|---|---|---|---|---|
| "/" means the recovery rate is not calculated. | | | | | | | | | |

In actual research, it was also found that precipitation would be produced when the linker-drug conjugate of ADC-8201 (GGFG-Dxd) was coupled with other antibodies, and the ratio of aggregates was high, which was not universal. However, most of the linker-drug conjugates in this technical solution were coupled with different antibodies, and no precipitation was produced, and the ratio of aggregates was in the normal range, indicating that the linker-drug conjugates in the present disclosure have better physical and chemical properties.

### Effect Embodiment 4: In vitro enzyme cleavage experiment of linker-drug conjugates

Linker-drug conjugates (LE14 and GGFG-Dxd) and cathepsin B were incubated in three different pH (5.0, 6.0, 7.0) buffers, and samples were taken at different time points into the high performance liquid chromatography-mass spectrometer. The release percentage of the drug was determined by external standard method (with Dxd as the external standard). The experimental results (shown in Table 9) show that GGFG-Dxd has a slower enzyme cleavage speed in the pH range used, while the LE14 of the present disclosure could quickly cleave in the range of pH 5.0 to pH 7.0.

**Table 9. Enzyme cleavage of LE14 and GGFG-Dxd at different pH in vitro**

| Time (h) | Release percentage of drug in samples % | | | | | |
|---|---|---|---|---|---|---|
| | GGFG-Dxd | | | LE14 | | |
| | pH 5.0 | pH 6.0 | pH 7.0 | pH 5.0 | pH 6.0 | pH 7.0 |
| 0 | 21.62 | 23.58 | 22.98 | 15 | 14.28 | 17.59 |
| 1 | 25 | 24.8 | 26.53 | 96.93 | 95.98 | 98.05 |
| 2 | 25.85 | 27.02 | 29.52 | 98.35 | 96.8 | 99.08 |
| 3 | 27.76 | 29.29 | 31.95 | 99.01 | 98.45 | 99.33 |
| 4 | 29.72 | 31.37 | 34.78 | 99.21 | 98.81 | 99.2 |
| 5 | 31.69 | 33.05 | 36.17 | 99.32 | 98.9 | 100 |
| 6 | 34.17 | 35.95 | 38.25 | 97.39 | 99 | 99.39 |

### Effect Embodiment 5: In vitro enzyme cleavage experiment of ADC030

The NCI-N87 cell line was selected as the experimental cell line. After the sample was incubated in the cathepsin B system (100 mM sodium acetate-acetic acid buffer, 4 mM dithiothreitol, pH 5.0) at 37 °C for 4 hours, the obtained sample was diluted to different concentrations by culture medium, 8 concentrations (1.5-10 times diluted) were set in 70 nM-0.003 nM according to SN-38 concentration, the changes in the killing (inhibition) ability for the cell line for 144 hours were observed, and chemiluminescence staining was performed by CellTiter-Glo^{®} Luminescent Cell Viability Assay, IC₅₀ value was calculated after reading the fluorescence data.

The sample of enzyme cleavage obtained by incubating in the cathepsin B system at 37 °C for 4 hours was subjected to an appropriate amount of ethanol to precipitate and remove the protein, and the released small molecule compounds were detected by high performance liquid chromatography, and the equal amount of SN-38 was used as a reference. The release rate at 4 hours was detected, the results showed that the release rate reached 99%.

The experimental results (shown in Table 10) show that the cytotoxic activity of ADC030 after enzyme cleavage was almost the same as that of equivalent amount of SN-38, also show that ADC030 almost completely releases SN-38 and functions with the action of cathepsin B. However, the endocytosis of ADC030 into the lysosome might cause unpredictable changes to cause SN-38 to not function effectively.

**Table 10. Changes in the killing activity of ADC030 on NCI-N87 cell line before and after enzyme cleavage by cathepsin B system**

| | IC₅₀ (Based on SN-38 equivalent, nM) | |
|---|---|---|
| Sample | Before enzyme cleavage | After enzyme cleavage |
| ADC030 | greater than70 nM | 7.011 nM |
| SN38 | 6.471 nM | 6.853 nM |

### Effect Embodiment 6: In vivo evaluation 1

Female Balb/c nude mice aged 6-8 weeks were injected subcutaneously on the back of the neck with 5×10⁶ human pancreatic cancer cells (Capan-1) dissolved in 100 µL of PBS solution. When the average tumor volume was about 160 mm³, the nude mice were randomly grouped according to the tumor size. The 36 nude mice were randomly divided into 6 groups with 6 animals in each group, and the group was administered by tail vein injection: 01 was the blank control group, and 02 was the ADC- 8201 (5 mg/kg), 03 was ADC-8201 (2 mg/kg), 04 was ADC-029 (5 mg/kg), 05 was ADC-029 (2 mg/kg), 06 was ADC-030 (5mg/kg), administered once. The body weight and tumor volume of the experimental animals were measured twice a week, and the survival status of the animals was observed during the experiment. The experimental results (shown in Table 11) show that ADC029 has good anti-tumor activity *in vivo.* At the same time, all experimental mice have no death or weight loss, indicating that ADC029 has good safety.

**Table 11. In vivo efficacy evaluation experiment results of ADCs**

| Gro up | Observation days | | | | | | | | | |
|---|---|---|---|---|---|---|---|---|---|---|
| | 13 | 15 | 19 | 22 | 26 | 29 | 33 | 36 | 40 | 43 |
| Average tumor volume/ mm³ | | | | | | | | | | |
| 01 | 165.9 7 | 188.0 5 | 220.11 | 288.34 | 375.37 | 487.37 | 652.21 | 731.11 | 886.69 | 1013.90 |
| 02 | 166.2 4 | 191.6 8 | 120.85 | 97.49 | 84.33 | 78.49 | 84.20 | 84.11 | 87.65 | 91.82 |
| 03 | 165.8 6 | 179.7 7 | 117.95 | 112.81 | 110.70 | 96.03 | 95.66 | 105.63 | 142.51 | 189.84 |
| 04 | 166.1 1 | 174.0 6 | 103.92 | 80.47 | 57.99 | 46.15 | 36.92 | 35.36 | 49.96 | 47.78 |
| 05 | 166.4 7 | 193.0 1 | 130.30 | 120.07 | 118.62 | 123.08 | 154.60 | 178.78 | 212.77 | 236.80 |
| 06 | 165.9 7 | 189.2 5 | 206.11 | 268.34 | 335.26 | 427.46 | 552.21 | 611.66 | 726.69 | 832.58 |

| Standard deviation | | | | | | | | | | |
|---|---|---|---|---|---|---|---|---|---|---|
| 01 | 8.80 | 11.86 | 10.55 | 25.68 | 42.69 | 60.51 | 86.63 | 100.19 | 118.87 | 143.97 |
| 02 | 10.16 | 10.13 | 10.78 | 11.27 | 14.13 | 15.29 | 19.38 | 22.54 | 29.99 | 37.98 |
| 03 | 8.46 | 5.52 | 9.79 | 11.62 | 12.07 | 14.78 | 19.00 | 16.67 | 30.07 | 42.31 |
| 04 | 10.26 | 10.76 | 10.85 | 8.58 | 11.41 | 12.49 | 9.57 | 9.89 | 16.65 | 15.96 |
| 05 | 9.67 | 17.59 | 12.76 | 13.26 | 20.41 | 32.95 | 39.89 | 53.93 | 70.96 | 80.51 |
| 06 | 9.10 | 10.86 | 11.27 | 20.54 | 33.63 | 40.89 | 66.52 | 87.45 | 90.87 | 124.27 |

### Effect 7: In vivo evaluation 2

Female Balb/c nude mice aged 6-8 weeks were injected subcutaneously on the right of back of the neck with 1×10⁷ human gastric cancer cells (NCI-N87) dissolved in 100 µL of PBS solution. When the average tumor volume was about 200 mm³, the nude mice were randomly grouped according to the tumor size. The 42 nude mice were randomly divided into 7 groups with 6 animals in each group, and the group was administered by tail vein injection: 01 was the blank control group, and 02 was the ADC- 8201 (2 mg/kg), 03 was ADC-8201 (1 mg/kg), 04 was ADC-029 (4 mg/kg), 05 was ADC-029 (2 mg/kg), 06 was ADC-029 (1mg/kg), 07 was ADC-030 (4 mg/kg), administered once. The body weight and tumor volume of the experimental animals were measured twice a week, and the survival status of the animals was observed during the experiment. The experimental results (shown in Table 12) show that ADC029 has good anti-tumor activity *in vivo.* At the same time, all experimental mice have no death or weight loss, indicating that ADC029 has good safety.

**Table 12. In vivo efficacy evaluation experiment results of ADCs**

| Grou p | Observation days | | | | | | | | | | |
|---|---|---|---|---|---|---|---|---|---|---|---|
| | 5 | 7 | 11 | 14 | 18 | 21 | 25 | 28 | 32 | 35 | 39 |
| | Average tumor volume/ mm³ | | | | | | | | | | |
| 01 | 205.3 | 283.81 | 395.5 | 489.3 | 621. | 721. | 783.8 | 890.7 | 994.8 | 1176. | 1348. |
| | 1 | | 0 | 6 | 74 | 41 | 5 | 9 | 0 | 92 | 83 |
| 02 | 205.3 | 260.96 | 235.6 | 202.2 | 250. | 341. | 363.7 | 412.4 | 479.7 | 527.3 | 655.9 |
| | 5 | | 5 | 7 | 53 | 51 | 6 | 4 | 4 | 0 | 6 |
| 03 | 205.5 | 315.13 | 332.4 | 284.4 | 419. | 489. | 529.2 | 628.7 | 725.0 | 892.2 | 1065. |
| | 4 | | 6 | 1 | 84 | 93 | 4 | 5 | 2 | 9 | 85 |
| 04 | 206.1 | 272.70 | 183.8 | 92.97 | 86.3 | 103. | 120.8 | 134.4 | 190.9 | 205.0 | 260.9 |
| | 2 | | 1 | | 0 | 46 | 5 | 4 | 5 | 7 | 4 |
| 05 | 206.9 | 339.51 | 268.5 | 192.5 | 191. | 217. | 250.7 | 273.1 | 324.7 | 368.2 | 426.1 |
| | 4 | | 8 | 5 | 16 | 61 | 8 | 3 | 7 | 0 | 2 |
| 06 | 205.3 | 296.64 | 320.0 | 307.4 | 386. | 510. | 595.4 | 699.0 | 809.6 | 922.7 | 1192. |
| | 2 | | 3 | 5 | 87 | 03 | 5 | 4 | 3 | 2 | 74 |
| 07 | 205.4 | 292.72 | 355.8 | 449.4 | 521. | 701. | 752.5 | 830.2 | 924.2 | 989.5 | 1248. |
| | 1 | | 9 | 0 | 77 | 56 | 6 | 9 | 9 | 0 | 83 |

| Standard deviation | | | | | | | | | | | |
|---|---|---|---|---|---|---|---|---|---|---|---|
| 01 | 11.93 | 26.98 | 38.06 | 34.44 | 32.8 | 19.2 | 21.21 | 35.51 | 47.44 | 75.40 | 72.37 |
| | | | | | 4 | 4 | | | | | |
| 02 | 11.17 | 23.58 | 35.14 | 36.43 | 47.4 | 59.9 | 56.95 | 73.14 | 88.12 | 90.84 | 135.7 |
| | | | | | 3 | 2 | | | | | 4 |
| 03 | 11.39 | 29.11 | 42.69 | 50.34 | 85.7 | 88.2 | 100.8 | 111.5 | 131.4 | 180.3 | 196.7 |
| | | | | | 8 | 4 | 4 | 8 | 3 | 6 | 9 |
| 04 | 12.53 | 33.17 | 38.18 | 15.98 | 19.1 | 28.8 | 40.77 | 46.78 | 59.68 | 59.36 | 65.89 |
| | | | | | 1 | 3 | | | | | |
| 05 | 12.72 | 23.80 | 30.81 | 36.69 | 47.2 | 72.8 | 83.71 | 87.46 | 101.8 | 113.6 | 142.4 |
| | | | | | 0 | 4 | | | 4 | 8 | 1 |
| 06 | 10.90 | 32.35 | 33.83 | 34.59 | 44.2 | 43.5 | 68.98 | 57.29 | 87.10 | 89.34 | 136.0 |
| | | | | | 7 | 5 | | | | | 2 |
| 07 | 12.69 | 30.12 | 39.85 | 39.29 | 54.1 | 62.5 | 78.29 | 94.24 | 100.2 | 111.4 | 137.9 |
| | | | | | 2 | 3 | | | 2 | 7 | 8 |

### Effect Embodiment 8: Safety evaluation

The male and female ICR mice were divided into two groups, respectively. ADC-8201 and ADC029 were given respectively at the dose of 300 mg/kg, and the body weight was 18.6-21.8 g at the time of administration. The mice were administered by tail vein injection. The body weight of the mice was measured at different time points within 14 days after administration. The results are summarized in the table below. The groups 01 and 02 were given ADC-8201, the groups 03 and 04 were given ADC029, the groups 01 and 03 were male mice, and the groups 02 and 04 were female mice. The test results (shown in Table 13) show that the weight of the mice does not decrease significantly when the dose of ADC029 to mice reached 300 mg/kg, indicating that the ADC has good safety.

**Table 13. In vivo safety evaluation of ADCs in mice**

| Group | Observation days | | | | | | |
|---|---|---|---|---|---|---|---|
| | 0 | 1 | 2 | 3 | 7 | 10 | 14 |

| Weight/g | | | | | | | |
|---|---|---|---|---|---|---|---|
| 01 | 20.9 | 19.8 | 19.8 | 20.5 | 25.2 | 28.2 | 32.4 |
| 02 | 19.8 | 19.0 | 18.3 | 18.7 | 22.5 | 23.1 | 25.3 |
| 03 | 21.0 | 20.0 | 19.7 | 20.0 | 25.9 | 28.8 | 33.0 |
| 04 | 19.8 | 18.5 | 18.0 | 18.5 | 22.0 | 22.5 | 25.5 |

| Standard deviation | | | | | | | |
|---|---|---|---|---|---|---|---|
| 01 | 0.4 | 0.4 | 0.6 | 3.1 | 4.4 | 4.7 | 4.0 |
| 02 | 0.4 | 1.2 | 1.1 | 0.7 | 1.7 | 1.4 | 3.8 |
| 03 | 0.4 | 0.8 | 0.4 | 1.2 | 5.0 | 3.1 | 2.4 |
| 04 | 0.5 | 2.1 | 1.7 | 4.2 | 1.7 | 4.1 | 6.3 |

Although the specific embodiments of the present disclosure are described above, those skilled in the art should understand that these are only embodiments, and various changes or modifications can be made to these embodiments without departing from the principle and essence of the present disclosure. Therefore, the protection scope of the present invention is defined by the appended claims.

## Claims

1. An antibody drug conjugate, a general structural formula of the antibody drug conjugate is Ab-(L₃-L₂-L₁-D)ₘ;
wherein, Ab is an antibody;
D is a cytotoxic drug;
m is 2-8;
the structure of L₁ is as shown in formula I, II, III or IV, a-end of the L₁ is connected to the cytotoxic drug, and e-end of the L₁ is connected to c-end of the L₂; 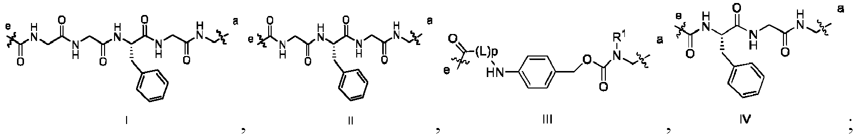
wherein L is independently phenylalanine residue, alanine residue, glycine residue, glutamic acid residue, aspartic acid residue, cysteine residue, histidine residue, isoleucine residue, leucine residue, lysine residue, methionine residue, proline residue, serine residue, threonine residue, tryptophan residue, tyrosine residue or valine residue; p is 2-4;
R¹ is C₁-C₆ alkyl substituted by -NR¹⁻¹R¹⁻², C₁-C₆ alkyl substituted by R¹⁻³ S(O)₂-, C₁-C₆ alkyl, C₃-C₁₀ cycloalkyl, C₆-C₁₄ aryl or 5 to 14-membered heteroaryl; the heteroatoms in the 5 to 14-membered heteroaryl are selected from one or more of N, O and S, and the number of heteroatoms is 1, 2, 3, or 4;
the R¹⁻¹, R¹⁻² and R¹⁻³ are independently C₁-C₆ alkyl;
L₂ is 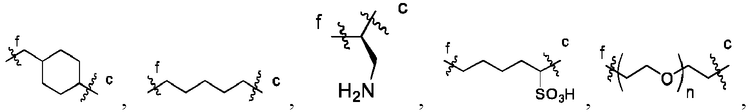 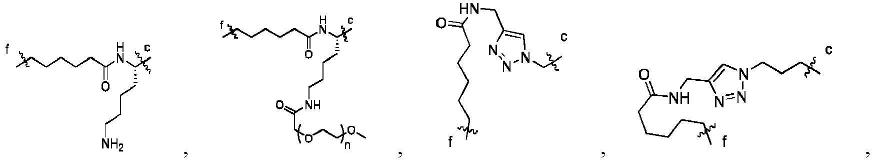 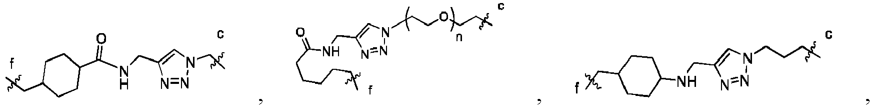 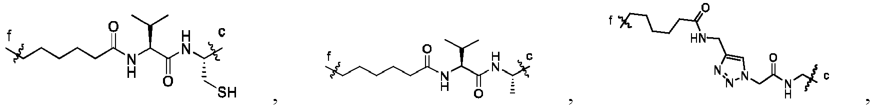 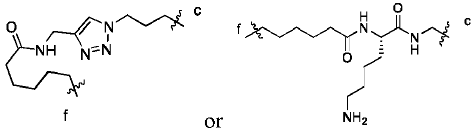 , wherein n is independently 1-12, c-end of the L₂ is connected to e-end of the L₁, f-end of the L₂ is connected to d-end of the L₃;
L₃ is 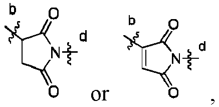 wherein b-end of the L₃ is connected to the Ab, d-end of the L₃ is connected to f-end of the L₂;
when the structure of the L₁ is as shown in formula I, the L₃ is 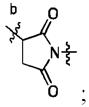 the L₂ is not

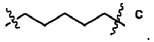

2. The antibody drug conjugate as defined in claim 1, wherein,
the antibody is anti-HER2 antibody Trastuzumab or variant thereof, anti-B7-H3 antibody P2E5 or variant thereof, anti-Claudin18.2 antibody IMAB362 or variant thereof, or anti-Trop2 antibody RS7 or variant thereof, preferably anti-HER2 antibody Trastuzumab or variant thereof, anti-B7-H3 antibody P2E5 or variant thereof, or anti-Claudin 18.2 antibody IMAB362 or variant thereof; the amino acid sequence of the light chain in the anti-HER2 antibody Trastuzumab is preferably shown in SEQ ID No. 5 in the sequence listing, and the amino acid sequence of the heavy chain in the anti-HER2 antibody Trastuzumab is preferably shown in SEQ ID No. 6 in the sequence listing; the amino acid sequence of the light chain in the anti-B7-H3 antibody P2E5 is preferably shown in SEQ ID No. 7 in the sequence listing, and the amino acid sequence of the heavy chain in the anti-B7-H3 antibody P2E5 is preferably shown in SEQ ID No. 8 in the sequence listing; the amino acid sequence of the light chain in the anti-Claudin 18.2 antibody IMAB362 is preferably shown in SEQ ID No. 1 in the sequence listing, and the amino acid sequence of the heavy chain in the anti-Claudin 18.2 antibody IMAB362 is preferably shown in SEQ ID No. 2 in the sequence listing; the amino acid sequence of the light chain in the anti-Trop2 antibody RS7 is preferably shown in SEQ ID No. 3 in the sequence listing, and the amino acid sequence of the heavy chain in the anti-Trop2 antibody RS7 is preferably shown in SEQ ID No. 4 in the sequence listing; the anti-HER2 antibody Trastuzumab variant has at least 70%, 75%, 80%, 85%, 90%, 95%, 98% or 99% homology compared with the anti-HER2 antibody Trastuzumab; the anti-B7-H3 antibody P2E5 variant has at least 70%, 75%, 80%, 85%, 90%, 95%, 98% or 99% homology compared with the anti-B7-H3 antibody P2E5; the anti-Trop2 antibody RS7 variant has at least 70%, 75%, 80%, 85%, 90%, 95%, 98% or 99% homology compared with the anti-Trop2 antibody RS7; the anti-Claudin 18.2 antibody IMAB362 variant has at least 70%, 75%, 80%, 85%, 90%, 95%, 98% or 99% homology compared with the anti-Claudin 18.2 antibody IMAB362;
and/or, the cytotoxic drug is a topoisomerase inhibitor containing a hydroxyl group, and preferably a topoisomerase I inhibitor containing a hydroxyl group, further preferably camptothecin or derivatives thereof, and further more preferably 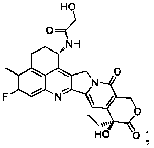
and/or, when the R¹ is C₁-C₆ alkyl substituted by -NR¹⁻¹R¹⁻², the C₁-C₆ alkyl is C₁-C₄ alkyl, preferably methyl, ethyl, *n*-propyl, isopropyl, *n*-butyl, isobutyl or *tert-butyl,* most preferably ethyl; the R¹⁻¹ and R¹⁻² are each independently preferably C₁-C₄ alkyl, more preferably methyl, ethyl, *n-*propyl, isopropyl, *n*-butyl, isobutyl or *tert-butyl,* most preferably methyl;
and/or, when the R¹ is C₁-C₆ alkyl substituted by R¹⁻³S(O)₂-, the C₁-C₆ alkyl is C₁-C₄ alkyl, preferably methyl, ethyl, *n*-propyl, isopropyl, *n*-butyl, isobutyl or *tert-butyl,* more preferably ethyl; the R¹⁻³ is preferably C₁-C₄ alkyl, more preferably methyl, ethyl, *n*-propyl, isopropyl, *n*-butyl, isobutyl or *tert-butyl,* most preferably methyl;
and/or, when the R¹ is C₁-C₆ alkyl, the C₁-C₆ alkyl is C₁-C₄ alkyl, more preferably methyl, ethyl, *n*-propyl, isopropyl, *n*-butyl, isobutyl or *tert-butyl,* most preferably methyl or ethyl;
and/or, the m is 4-8, preferably 7-8, for example, 7.3, 7.4, 7.5, 7.6, 7.7, 7.8, 8.0;
and/or, the n is preferably 8-12.

3. The antibody drug conjugate as defined in claim 1 or 2, wherein,
the L is valine residue or alanine residue, and p is preferably 2; the (L)p is further preferably 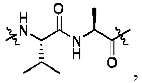 wherein the amino-end of the (L)p is connected to the carbonyl-end in the formula III;
and/or, the R¹ is C₁-C₆ alkyl substituted by -NR¹⁻¹R¹⁻², C₁-C₆ alkyl substituted by R¹⁻³S(O)₂-, or C₁-C₆ alkyl, preferably C₁-C₆ alkyl substituted by -NR¹⁻¹R¹⁻² or C₁-C₆ alkyl substituted by R¹⁻³S(O)₂-, more preferably C₁-C₆ alkyl substituted by R¹⁻³S(O)₂-; when R¹ is C₁-C₆ alkyl, the C₁-C₆ alkyl is preferably methyl or ethyl; the C₁-C₆ alkyl substituted by R¹⁻³S(O)₂- is preferably 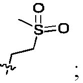 the C₁-C₆ alkyl substituted by -NR¹⁻¹R¹⁻² is preferably 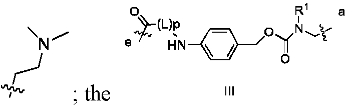 is preferably 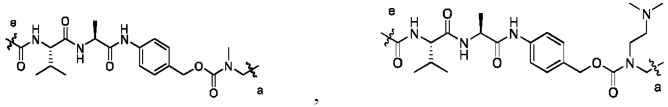 or 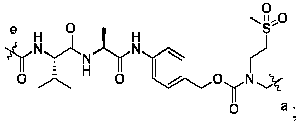
and/or, the L₃ is 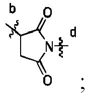
and/or, when the structure of L₁ is as shown in formula I, the L₂ is preferably 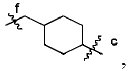 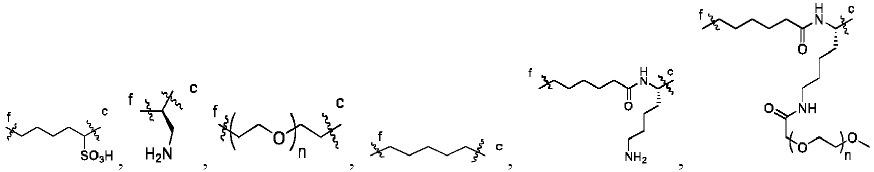 or 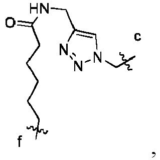 preferably 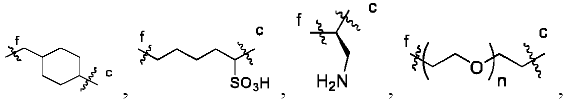 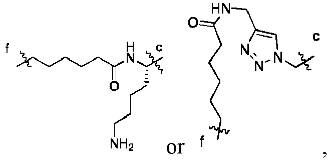 more preferably 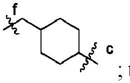 the L₃ is preferably 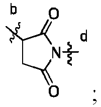
and/or, when the structure of L₁ is as shown in formula II, the L₂ is 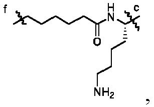 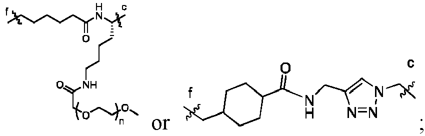 the L₃ is preferably 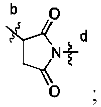
and/or, when the structure of L₁ is as shown in formula III, the L₂ is 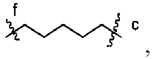 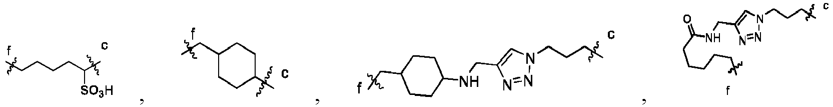 or 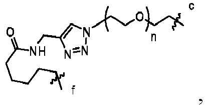 preferably 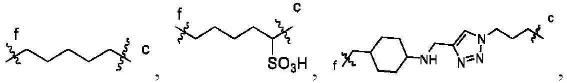 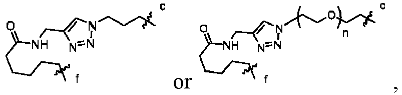 more preferably 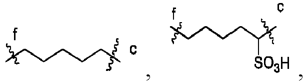 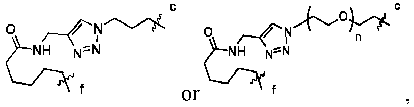 further more preferably 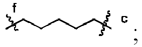 the L₃ is preferably 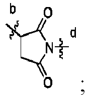
and/or, when the structure of L₁ is as shown in formula IV, the L₂ is 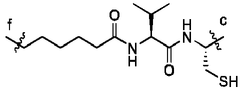 or 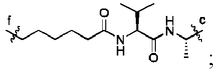 the L₃ is preferably

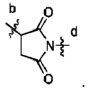

4. The antibody drug conjugate as defined in claim 1, wherein,
the Ab is anti-HER2 antibody Trastuzumab, anti-B7-H3 antibody P2E5 or variant thereof, or anti-Claudin 18.2 antibody IMAB362 or variant thereof; the D is a cytotoxic drug; the m is 2-8;
the structure of the L₁ is as shown in formula I, II, III or IV, 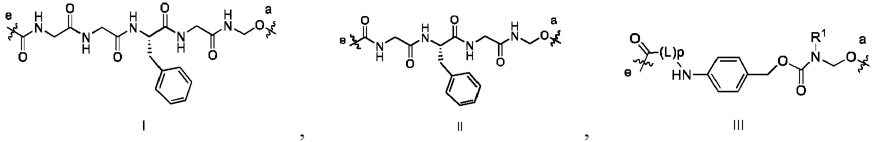 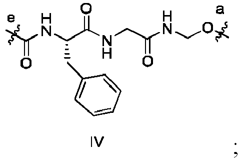
the L₂ is 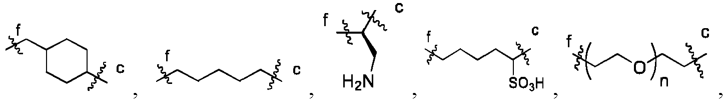 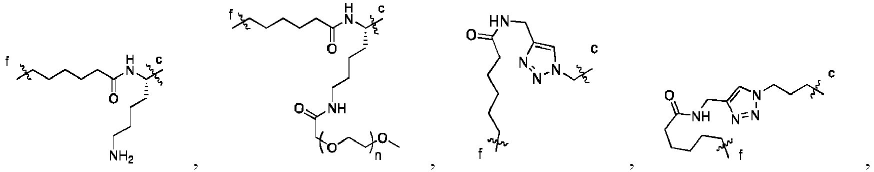 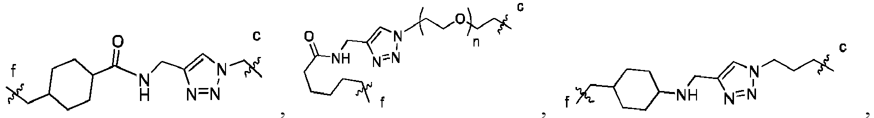 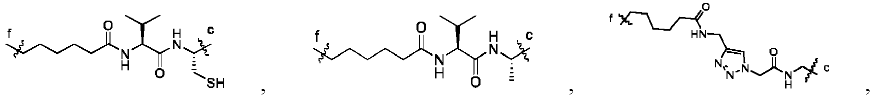 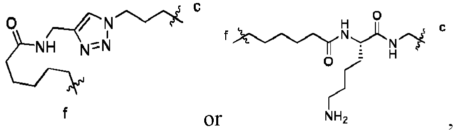 the n is independently 8-12;
the L₃ is 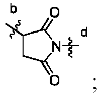
the L is independently valine residue or alanine residue; the p is 2 to 4;
the R¹ is C₁-C₆ alkyl substituted by -NR¹⁻¹R¹⁻², C₁-C₆ alkyl substituted by R¹⁻³S(O)₂-, or C₁-C₆ alkyl;
the R¹⁻¹, R¹⁻² and R¹⁻³ are each independently C₁-C₆ alkyl;
wherein, the amino acid sequence of the light chain in the anti-HER2 antibody Trastuzumab is preferably shown in SEQ ID No. 5 in the sequence listing, and the amino acid sequence of the heavy chain in the anti-HER2 antibody Trastuzumab is preferably shown in SEQ ID No. 6 in the sequence listing; the amino acid sequence of the light chain in the anti-B7-H3 antibody P2E5 is preferably shown in SEQ ID No. 7 in the sequence listing, and the amino acid sequence of the heavy chain in the anti-B7-H3 antibody P2E5 is preferably shown in SEQ ID No. 8 in the sequence listing; the amino acid sequence of the light chain in the anti-Claudin 18.2 antibody IMAB362 is preferably shown in SEQ ID No. 1 in the sequence listing, and the amino acid sequence of the heavy chain in the anti-Claudin 18.2 antibody IMAB362 is preferably shown in SEQ ID No. 2 in the sequence listing.

5. The antibody drug conjugate as defined in any one of claims 1-4, wherein,
the Ab is anti-HER2 antibody Trastuzumab, anti-B7-H3 antibody P2E5 or variant thereof, or anti-Claudin 18.2 antibody IMAB362 or variant thereof; the D is 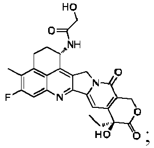 the m is 7-8;
the structure of the L₁ is as shown in formula I or III, 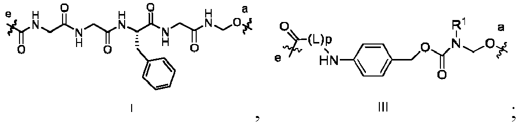
when the structure of the L₁ is as shown in formula I, the L₂ is 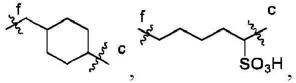 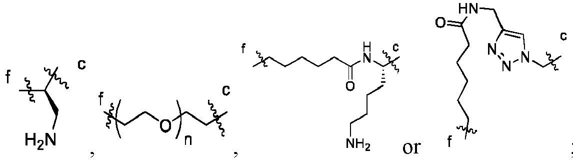 ; the n is independently 8-12;
when the structure of the L₁ is as shown in formula III, the L₂ is 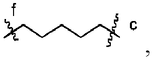 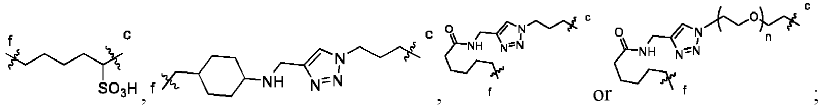 the n is independently 8-12;
the L₃ is 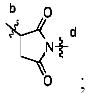
the L is independently valine residue or alanine residue; the p is 2 to 4;
the R¹ is C₁-C₄ alkyl substituted by -NR¹⁻¹R¹⁻², C₁-C₄ alkyl substituted by R¹⁻³S(O)₂-, or C₁-C₄ alkyl; the R¹⁻¹, R¹⁻² and R¹⁻³ are independently C₁-C₄ alkyl;
the amino acid sequence of the light chain in the anti-HER2 antibody Trastuzumab is preferably shown in SEQ ID No. 5 in the sequence listing, and the amino acid sequence of the heavy chain in the anti-HER2 antibody Trastuzumab is preferably shown in SEQ ID No. 6 in the sequence listing; the amino acid sequence of the light chain in the anti-B7-H3 antibody P2E5 is preferably shown in SEQ ID No. 7 in the sequence listing, and the amino acid sequence of the heavy chain in the anti-B7-H3 antibody P2E5 is preferably shown in SEQ ID No. 8 in the sequence listing; the amino acid sequence of the light chain in the anti-Claudin 18.2 antibody IMAB362 is preferably shown in SEQ ID No. 1 in the sequence listing, and the amino acid sequence of the heavy chain in the anti-Claudin 18.2 antibody IMAB362 is preferably shown in SEQ ID No. 2 in the sequence listing.

6. The antibody drug conjugate as defined in claim 1, wherein,
Ab is antibody; D is 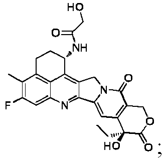
L₁ is 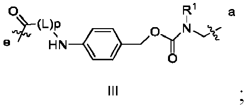 wherein, L is valine residue or alanine residue, p is 2, (L)p is preferably 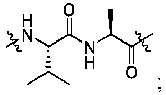 R¹ is C₁-C₆ alkyl substituted by -NR¹⁻¹R¹⁻², C₁-C₆ alkyl substituted by R¹⁻³S(O)₂-, or C₁-C₆ alkyl, preferably C₁-C₆ alkyl substituted by -NR¹⁻¹R¹⁻² or C₁-C₆ alkyl substituted by R¹⁻³ S(O)₂-, more preferably C₁-C₆ alkyl substituted by R¹⁻³S(O)₂-; the R¹⁻¹, R¹⁻² and R¹⁻³ are independently C₁-C₄ alkyl, preferably methyl; the C₁-C₆ alkyl substituted by -NR¹⁻¹R¹⁻² is preferably 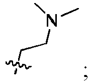 the C₁-C₆ alkyl substituted by R¹⁻³S(O)₂- is preferably 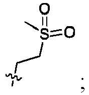 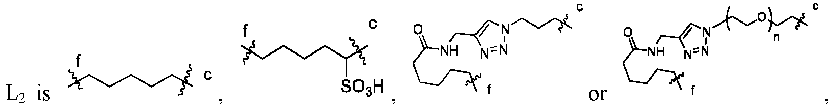 wherein, n is preferably 8, L₂ is preferably

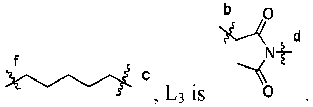

7. The antibody drug conjugate as defined in claim 1, wherein, the antibody drug conjugate is any of the compounds shown below: 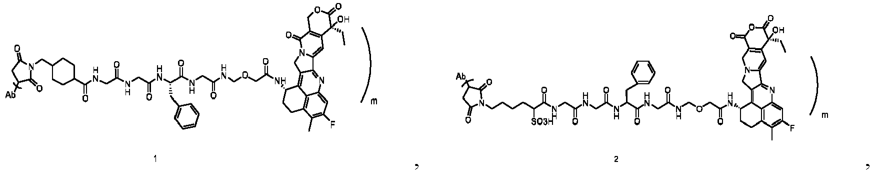 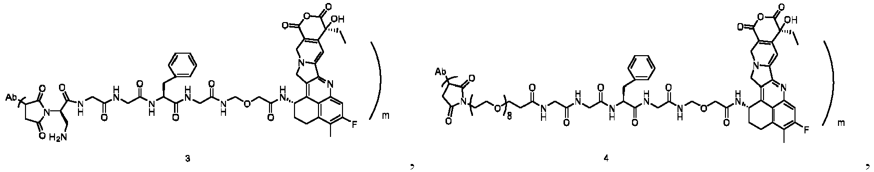 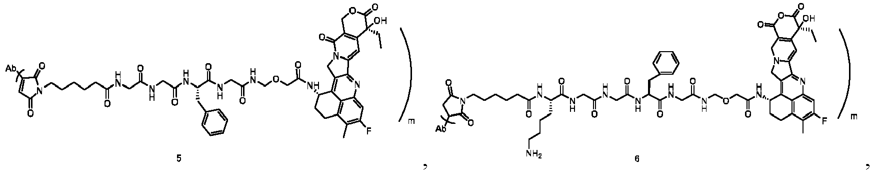 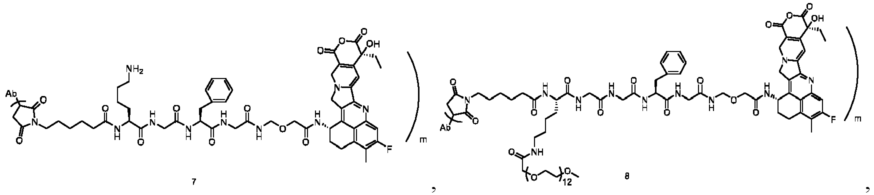 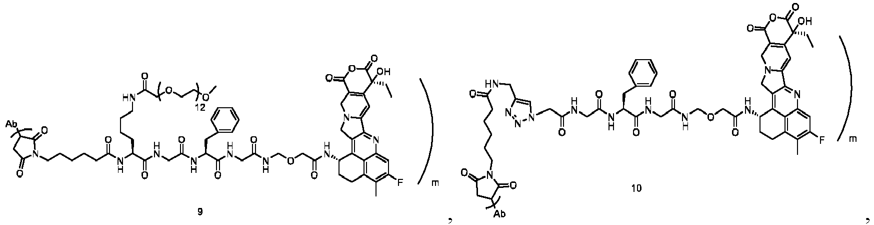 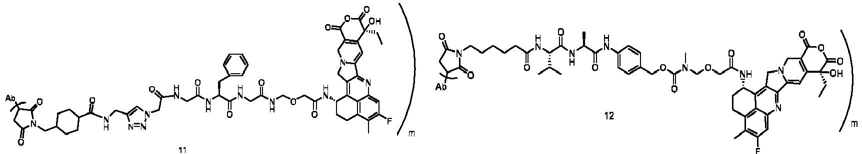 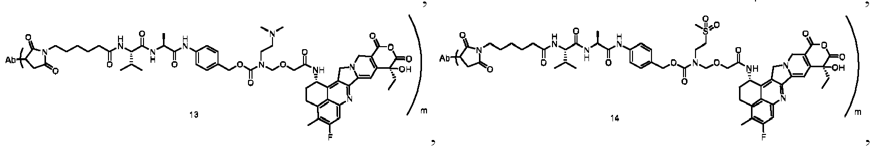 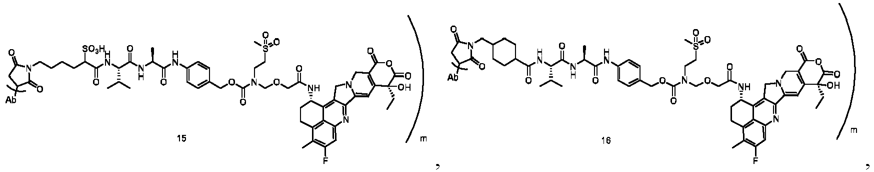 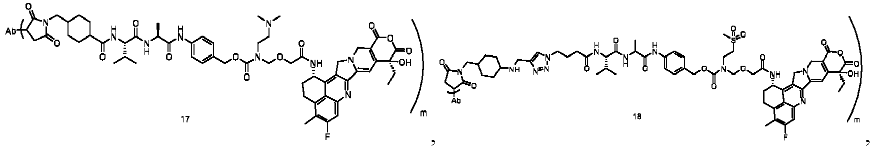 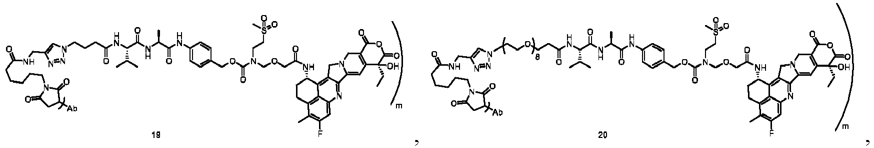 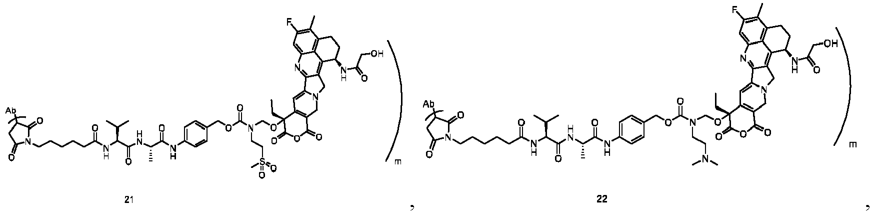 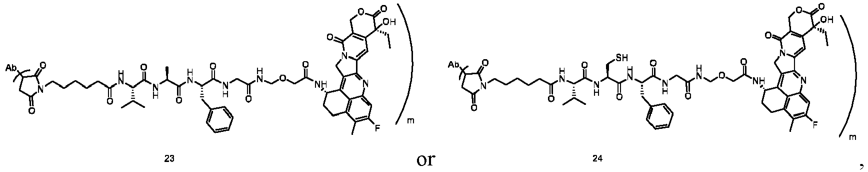
wherein, m is 2-8, preferably 7-8, for example 7.3, 7.4, 7.5, 7.6, 7.7, 7.8 or 8.0;
Ab is anti-HER2 antibody Trastuzumab, anti-B7-H3 antibody P2E5 or anti-Claudin 18.2 antibody IMAB362; the amino acid sequence of the light chain in the Ab is shown in SEQ ID No. 5 in the sequence listing, and the amino acid sequence of the heavy chain in the Ab is shown in SEQ ID No. 6 in the sequence listing; the amino acid sequence of the light chain in the anti-B7-H3 antibody P2E5 is shown in SEQ ID No. 7 in the sequence listing, and the amino acid sequence of the heavy chain in the anti-B7-H3 antibody P2E5 is shown in SEQ ID No. 8 in the sequence listing; the amino acid sequence of the light chain in the anti-Claudin 18.2 antibody IMAB362 is shown in SEQ ID No. 1 in the sequence listing, and the amino acid sequence of the heavy chain in the anti-Claudin 18.2 is shown in SEQ ID No. 2 in the sequence listing.

8. The antibody drug conjugate as defined in claims 1-7, wherein, the antibody drug conjugate is any of the compounds shown below: 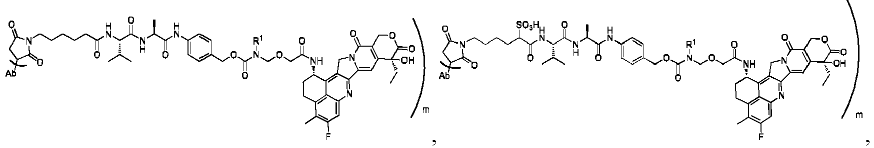 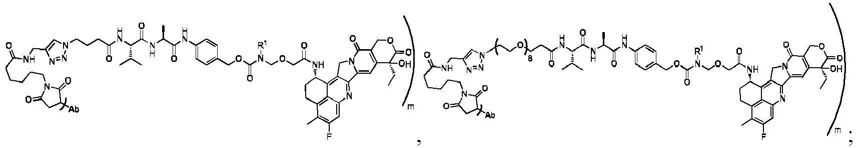 wherein, Ab, m and R¹ are as defined in any one of claims 1-7.

9. The antibody drug conjugate as defined in claim 1, wherein, the antibody drug conjugate is any of the compounds shown below: 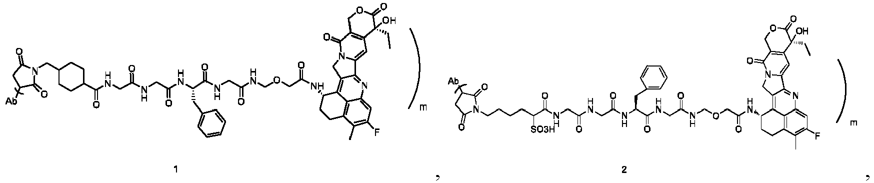 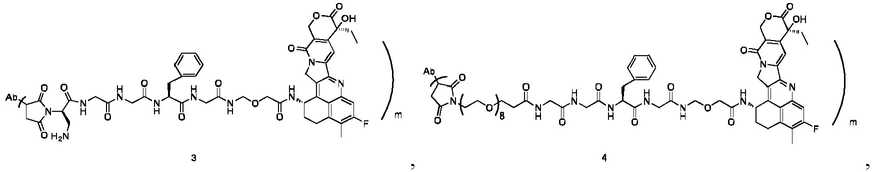 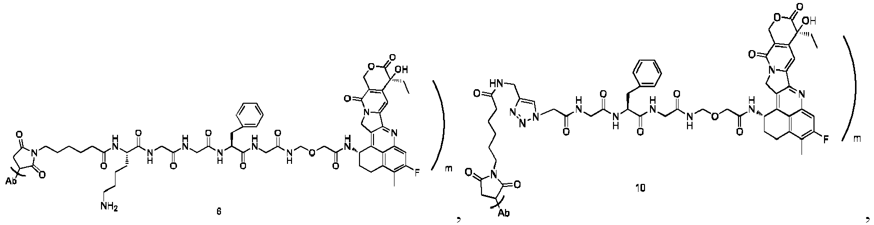 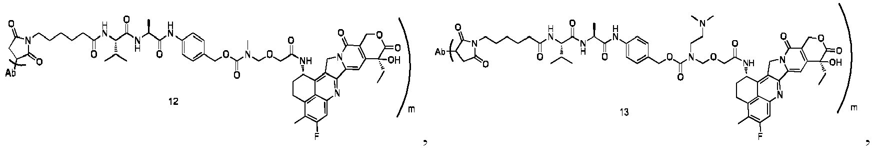 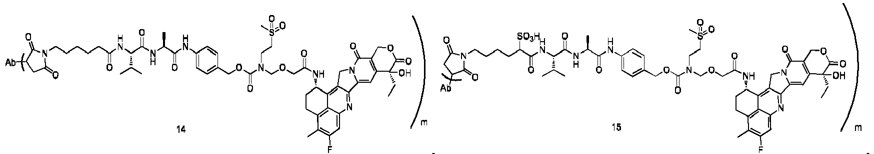 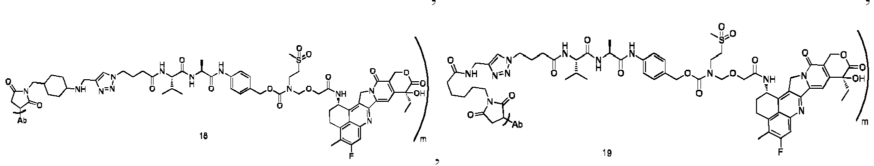 or 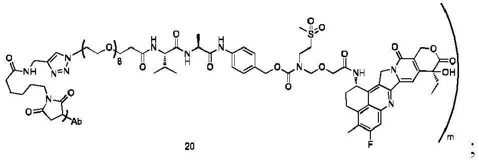 wherein, Ab is anti-HER2 antibody Trastuzumab; or , the amino acid sequence of the light chain in the Ab is shown in SEQ ID No. 5 in the sequence listing, and the amino acid sequence of the heavy chain in the Ab is shown in SEQ ID No. 6 in the sequence listing; wherein, m is 2-8, preferably 7-8, for example 7.3, 7.4, 7.5, 7.6, 7.7, 7.8 or 8.0;
or, the antibody drug conjugate is any of the compounds shown below: 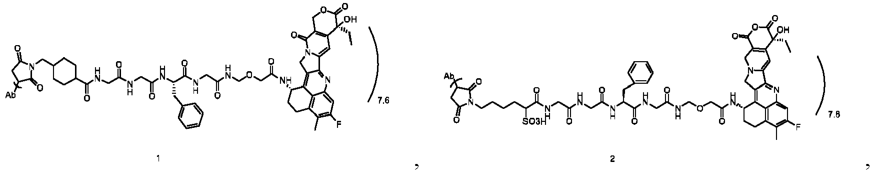 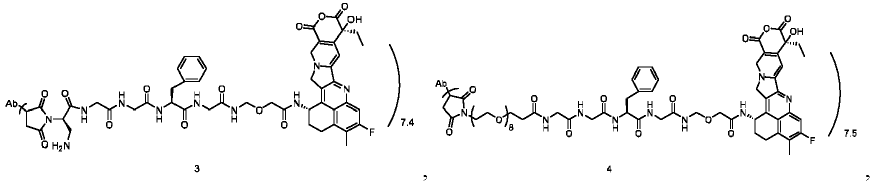 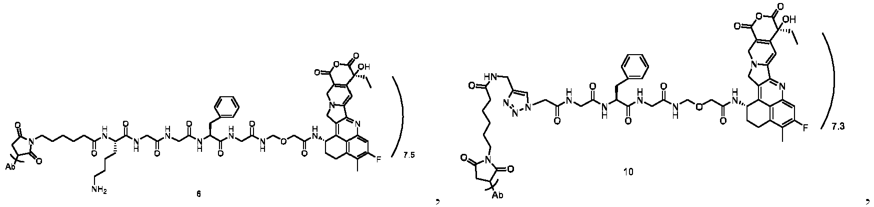 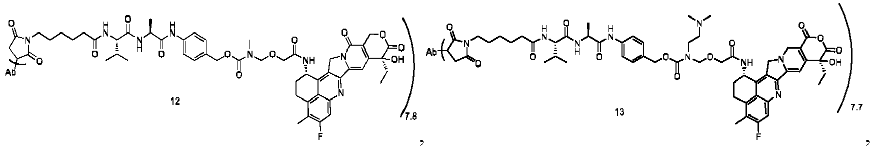 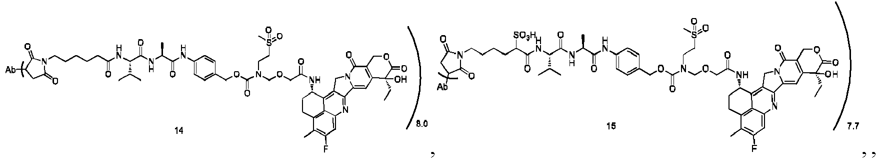 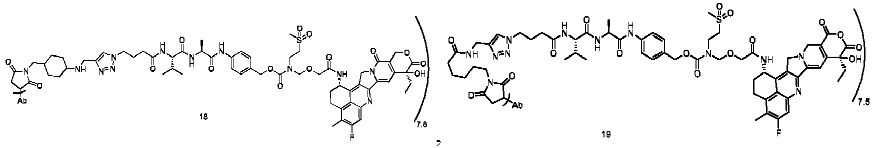 or 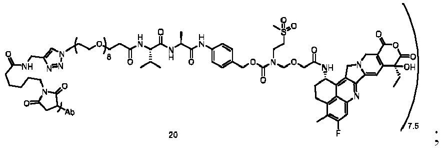 wherein, Ab is anti-HER2 antibody Trastuzumab; or, the amino acid sequence of the light chain in the Ab is shown in SEQ ID No. 5 in the sequence listing, and the amino acid sequence of the heavy chain in the Ab is shown in SEQ ID No. 6 in the sequence listing;
or, the antibody drug conjugate is any of the compounds shown below: 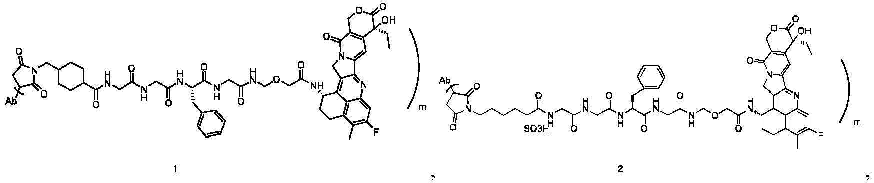 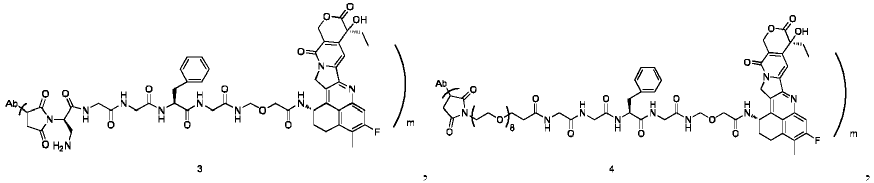 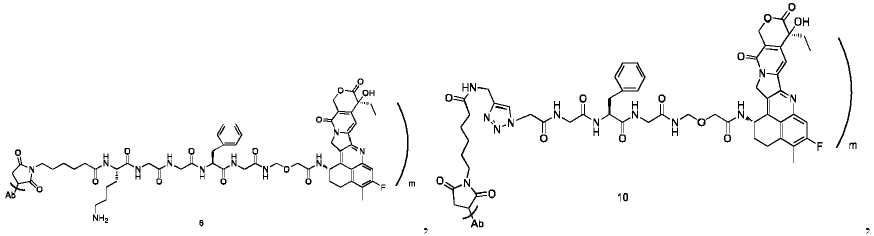 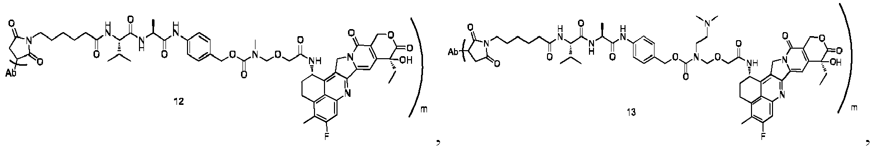 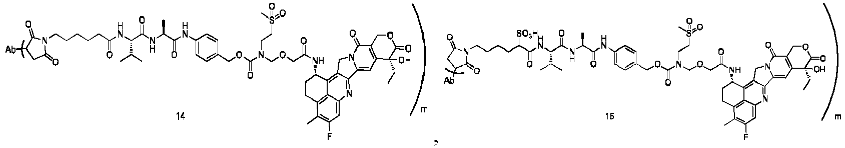 ,or 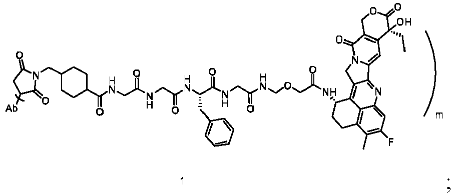 wherein, Ab is anti-B7-H3 antibody P2E5; or, the amino acid sequence of the light chain in the Ab is shown in SEQ ID No. 7 in the sequence listing, and the amino acid sequence of the heavy chain in the Ab is shown in SEQ ID No. 8 in the sequence listing; wherein, m is 2-8, preferably 7-8, for example 7.3, 7.4, 7.5, 7.6, 7.7, 7.8 or 8.0;
or, the antibody drug conjugate is any of the compounds shown below: 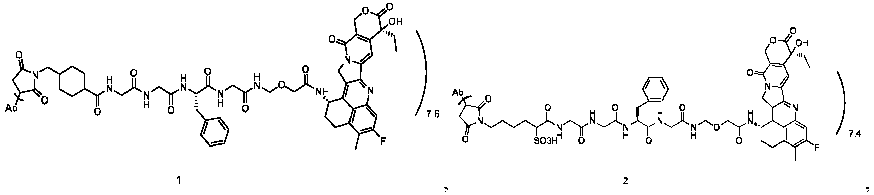 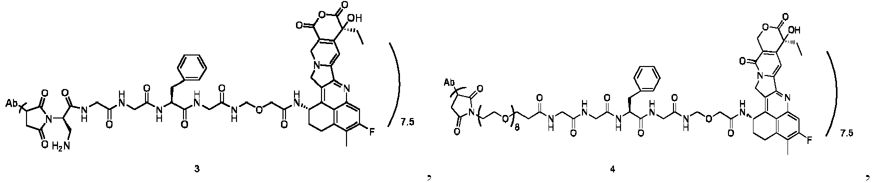 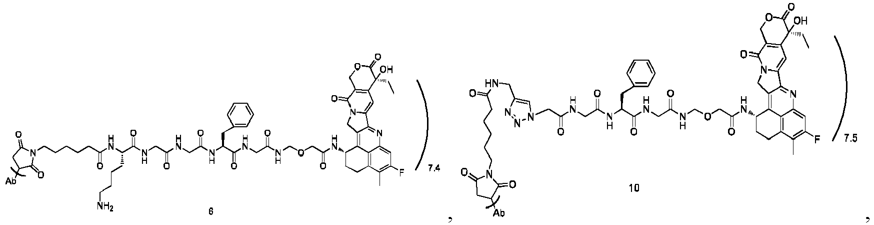 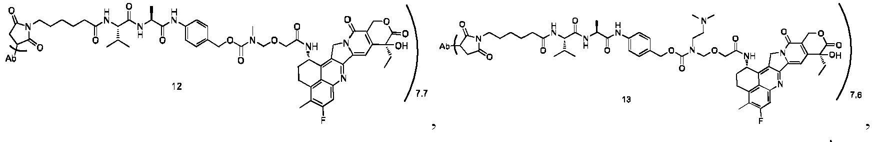 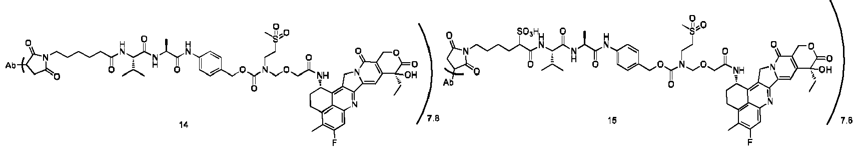 or 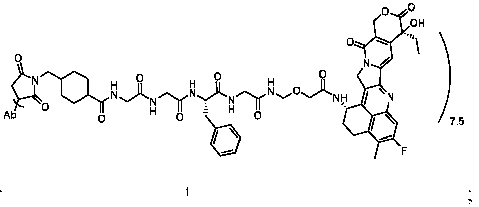 wherein, Ab is anti-B7-H3 antibody P2E5; or, the amino acid sequence of the light chain in the Ab is shown in SEQ ID No. 7 in the sequence listing, and the amino acid sequence of the heavy chain in the Ab is shown in SEQ ID No. 8 in the sequence listing;
or, the antibody drug conjugate is any of the compounds shown below: 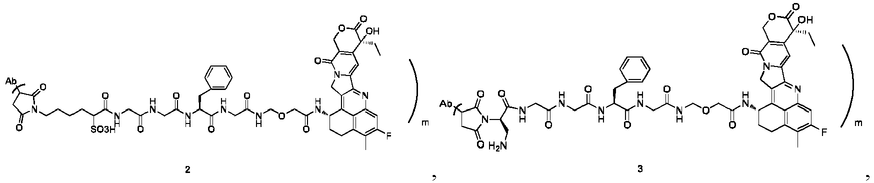 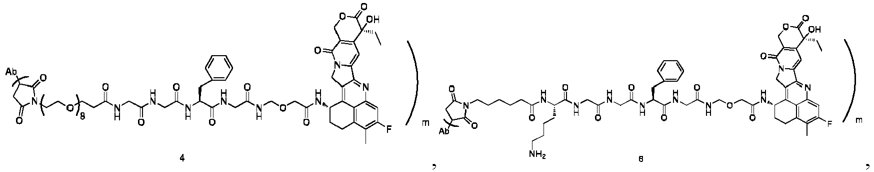 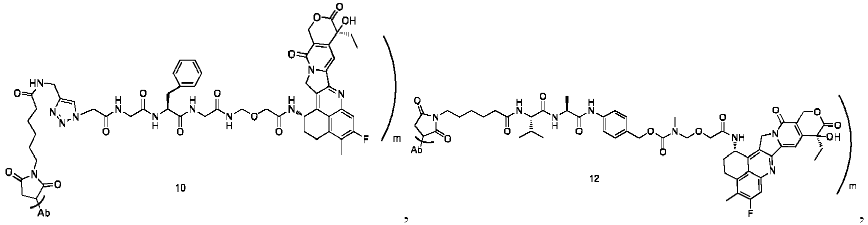 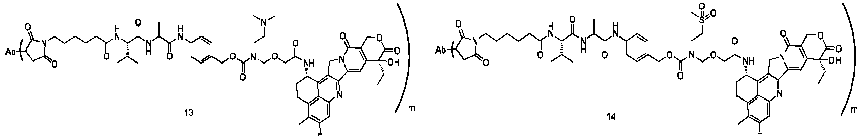 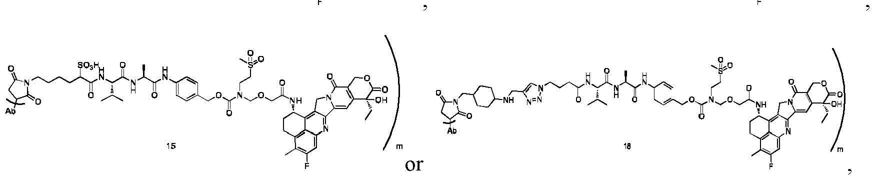 wherein, Ab is anti-Claudin18.2 antibody IMAB362; or, the amino acid sequence of the light chain in the Ab is shown in SEQ ID No. 1 in the sequence listing, and the amino acid sequence of the heavy chain in the Ab is shown in SEQ ID No. 2 in the sequence listing; wherein, m is 2-8, preferably 7-8, for example 7.3, 7.4, 7.5, 7.6, 7.7, 7.8 or 8.0;
or, the antibody drug conjugate is any of the compounds shown below: 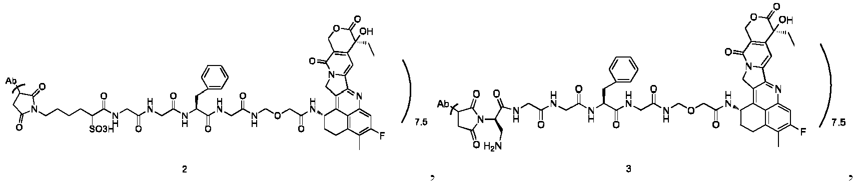 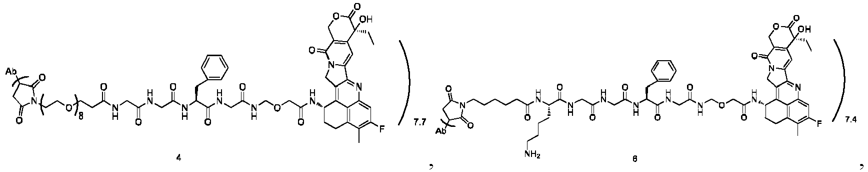 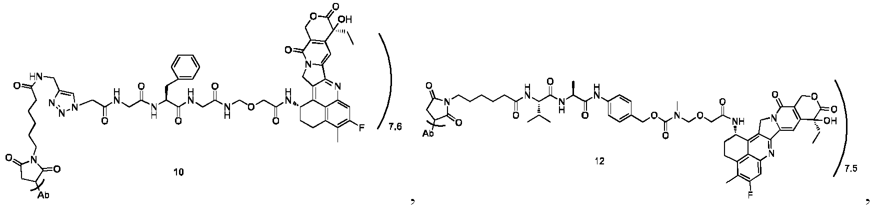 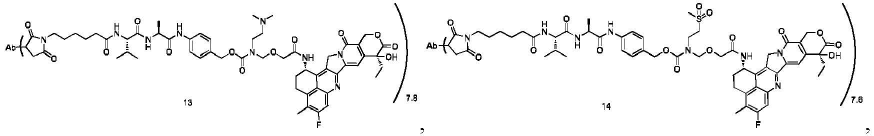 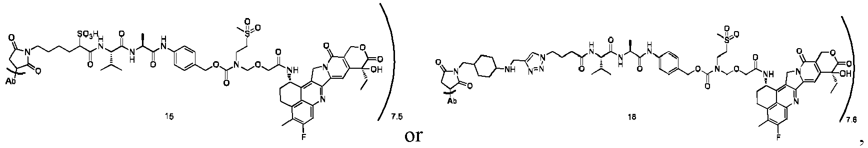 wherein, Ab is anti-Claudin18.2 antibody IMAB362; or, the amino acid sequence of the light chain in the Ab is shown in SEQ ID No. 1 in the sequence listing, and the amino acid sequence of the heavy chain in the Ab is shown in SEQ ID No. 2 in the sequence listing.

10. A linker-drug conjugate, a general structural formula of the linker-drug conjugate is L₄-L₂-L₁-D; wherein L₄ is 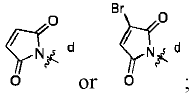 L₂, L₁, and D are as defined in any one of claims 1-9, f-end of the L₂ is connected to d-end of the L₄; when the L₄ is 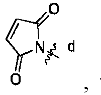 when the L₁ is 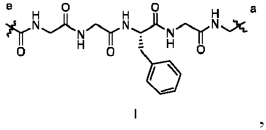 the L₂ is not

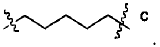

11. The linker-drug conjugate as defined in claim 10, the linker-drug conjugate is any of the compounds shown below: 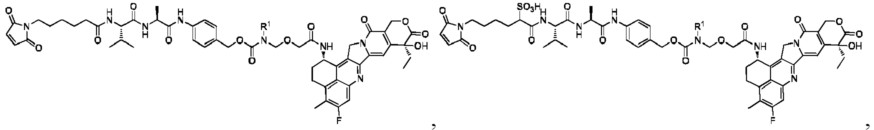 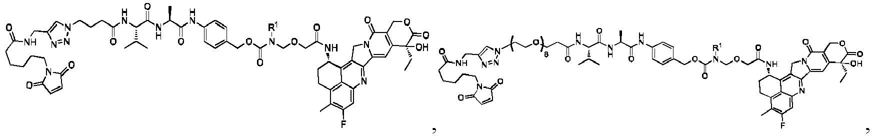 wherein, R¹ is as defined in any one of claims 1-9.

12. The linker-drug conjugate as defined in claim 10, wherein, the linker-drug conjugate is any of the compounds shown below:

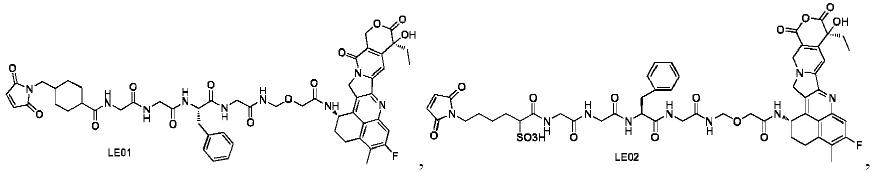

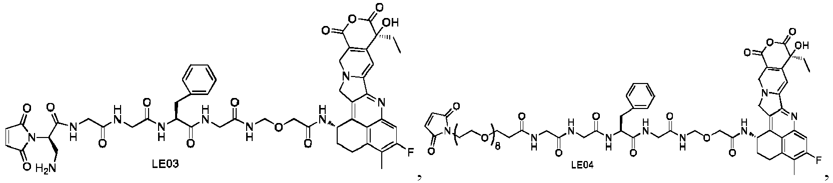

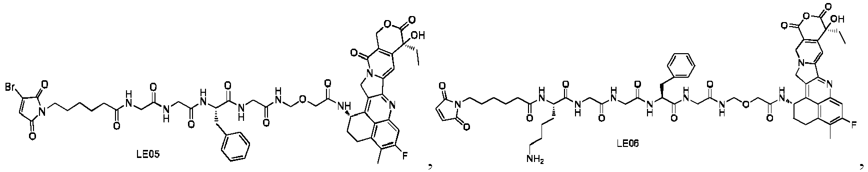

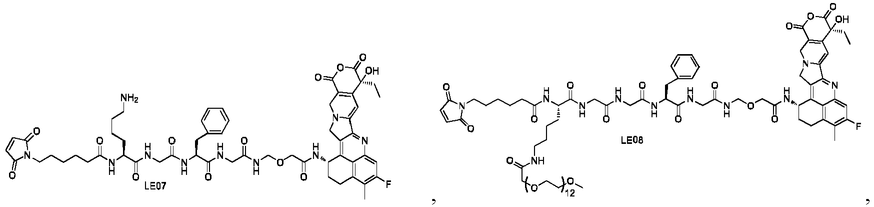

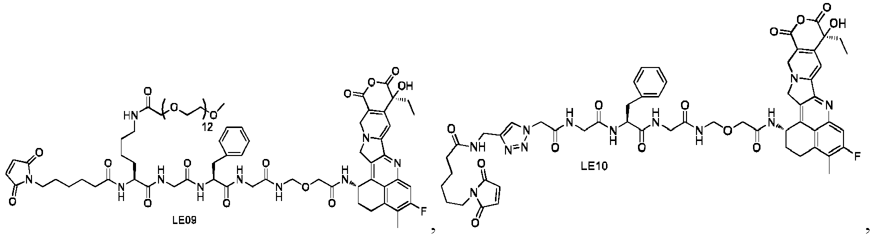

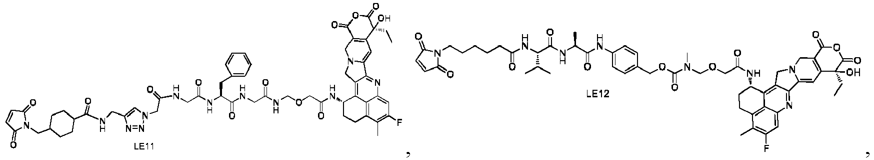

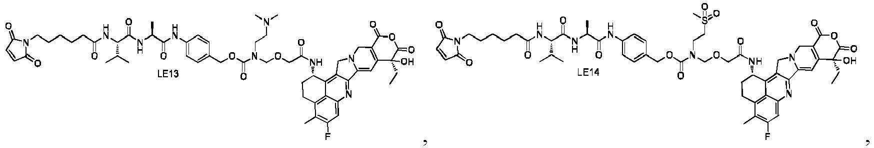

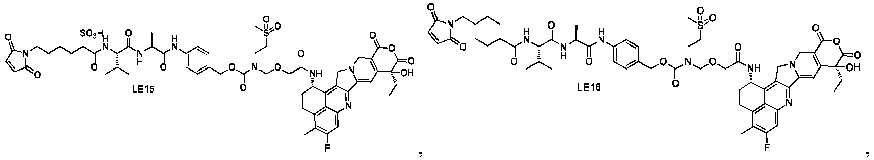

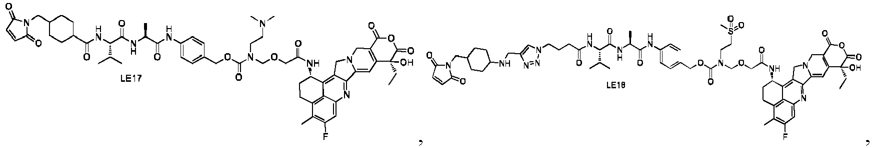

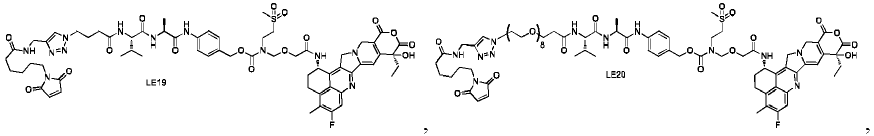

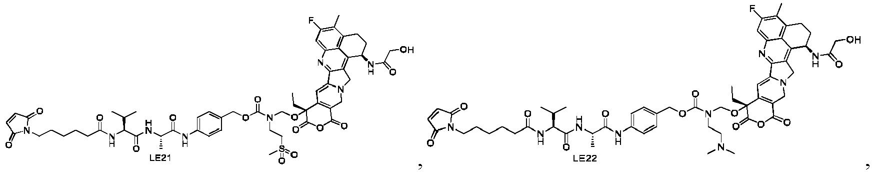

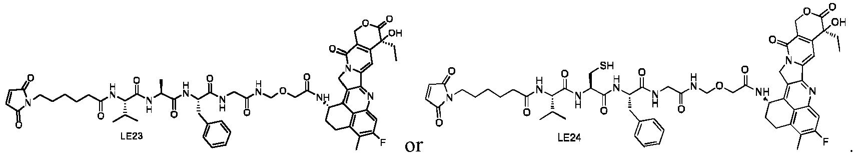

13. A method for preparing the antibody drug conjugate as defined in any one of claims 1-9, the method comprises the following steps, coupling the linker-drug conjugate as defined in any one of claims 10-12 with the antibody as defined in any one of claims 1-9.

14. A pharmaceutical composition comprising the antibody drug conjugate as defined in any one of claims 1-9 and a pharmaceutically acceptable carrier.

15. A use of the antibody drug conjugate as defined in any one of claims 1-9 or the pharmaceutical composition as defined in claim 14 in the preparation of a medicament for the prevention and/or treatment of cancer; preferably, the cancer is gastric cancer, breast cancer, non-small cell lung cancer, urothelial cancer or pancreatic cancer.

16. A compound as shown below, 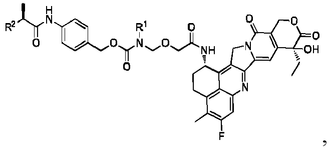
wherein, R¹ is as defined in any one of claims 1-9;
R² is -N₃, -NH₂,

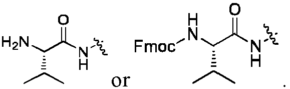

17. The compound as defined in claim 16, the compound is any of the compounds shown below:


SEQUENCE LISTING

<110>  Shanghai Fudan-Zhangjiang Bio-Pharmaceutical Co., Ltd.

<120>  Antibody-Drug Conjugate, Intermediate Thereof, Preparation Method
       Therefor and Application Thereof

<130>  EP137799-SZ030pau

<140>  EP20833007.6
<141>  2020-06-05

<150>  CN201910577909.6
<151>  2019-06-28

<160>  8

<170>  PatentIn version 3.5

<210>  1
<211>  220
<212>  PRT
<213>  Artificial Sequence

<220>
<223>  IMAB362 Light chain sequence

<400>  1

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Phe Thr Phe Gly Ser Gly Thr Lys Leu Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

<210>  2
<211>  448
<212>  PRT
<213>  Artificial Sequence

<220>
<223>  IMAB362 Heavy chain sequence

<400>  2

Gln Val Gln Leu Gln Gln Pro Gly Ala Glu Leu Val Arg Pro Gly Ala
1               5                   10                  15

Ser Val Lys Leu Ser Cys Lys Ala Ser Gly Tyr Thr Phe Thr Ser Tyr
            20                  25                  30

Trp Ile Asn Trp Val Lys Gln Arg Pro Gly Gln Gly Leu Glu Trp Ile
        35                  40                  45

Gly Asn Ile Tyr Pro Ser Asp Ser Tyr Thr Asn Tyr Asn Gln Lys Phe
    50                  55                  60

Lys Asp Lys Ala Thr Leu Thr Val Asp Lys Ser Ser Ser Thr Ala Tyr
65                  70                  75                  80

Met Gln Leu Ser Ser Pro Thr Ser Glu Asp Ser Ala Val Tyr Tyr Cys
                85                  90                  95

Thr Arg Ser Trp Arg Gly Asn Ser Phe Asp Tyr Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Arg Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Glu Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210>  3
<211>  214
<212>  PRT
<213>  Artificial Sequence

<220>
<223>  RS7 Light chain sequence

<400>  3

Asp Ile Gln Leu Thr Gln Ser Pro Ser Ser Leu Ser Ala Ser Val Gly
1               5                   10                  15

Asp Arg Val Ser Ile Thr Cys Lys Ala Ser Gln Asp Val Ser Ile Ala
            20                  25                  30

Val Ala Trp Tyr Gln Gln Lys Pro Gly Lys Ala Pro Lys Leu Leu Ile
        35                  40                  45

Tyr Ser Ala Ser Tyr Arg Tyr Thr Gly Val Pro Asp Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Val Tyr Tyr Cys Gln Gln His Tyr Ile Thr Pro Leu
                85                  90                  95

Thr Phe Gly Ala Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  4
<211>  451
<212>  PRT
<213>  Artificial Sequence

<220>
<223>  RS7 Heavy chain sequence

<400>  4

Gln Val Gln Leu Gln Gln Ser Gly Ser Glu Leu Lys Lys Pro Gly Ala
1               5                   10                  15

Ser Val Lys Val Ser Cys Lys Ala Ser Gly Tyr Thr Phe Thr Asn Tyr
            20                  25                  30

Gly Met Asn Trp Val Lys Gln Ala Pro Gly Gln Gly Leu Lys Trp Met
        35                  40                  45

Gly Trp Ile Asn Thr Tyr Thr Gly Glu Pro Thr Tyr Thr Asp Asp Phe
    50                  55                  60

Lys Gly Arg Phe Ala Phe Ser Leu Asp Thr Ser Val Ser Thr Ala Tyr
65                  70                  75                  80

Leu Gln Ile Ser Ser Leu Lys Ala Asp Asp Thr Ala Val Tyr Phe Cys
                85                  90                  95

Ala Arg Gly Gly Phe Gly Ser Ser Tyr Trp Tyr Phe Asp Val Trp Gly
            100                 105                 110

Gln Gly Ser Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Arg Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  5
<211>  214
<212>  PRT
<213>  Artificial Sequence

<220>
<223>  Trastuzumab Light chain sequence

<400>  5

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  6
<211>  450
<212>  PRT
<213>  Artificial Sequence

<220>
<223>  Trastuzumab Heavy chain sequence

<400>  6

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val

        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  7
<211>  217
<212>  PRT
<213>  Artificial Sequence

<220>
<223>  P2E5 Light chain sequence

<400>  7

Gln Thr Val Val Thr Gln Glu Pro Ser Phe Ser Val Ser Pro Gly Gly
1               5                   10                  15

Thr Val Thr Leu Thr Cys Gly Leu Asn Ser Gly Ser Val Ser Thr Ser
            20                  25                  30

Tyr Phe Pro Ser Trp Tyr Gln Gln Thr Pro Gly Gln Ala Pro Arg Thr
        35                  40                  45

Leu Ile Tyr Asn Thr Asn Thr Arg Ser Ser Gly Val Pro Asp Arg Phe
    50                  55                  60

Ser Gly Ser Ile Leu Gly Asn Lys Ala Ala Leu Thr Ile Thr Gly Ala
65                  70                  75                  80

Gln Ala Asp Asp Glu Ser Asp Tyr Tyr Cys Leu Leu Tyr Met Asp Ser
                85                  90                  95

Gly Pro His Trp Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu

        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210> 8
<211> 449
<212> PRT
<213> Artificial Sequence

<220>
<223> P2E5 Heavy chain sequence

<400> 8

Gln Val Thr Leu Lys Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ser
            20                  25                  30

Tyr Met Thr Trp Val Arg Gln Ala Pro Gly Met Gly Leu Glu Trp Val
        35                  40                  45

Ala Ser Met Lys Pro Asp Gly Ser Val Lys His Tyr Val Asp Ser Val
    50                  55                  60

Lys Gly Arg Phe Thr Ile Ser Arg Asp Asn Thr Lys Asn Ser Leu Asp
65                  70                  75                  80

Leu Gln Met Asn Ser Leu Arg Ala Glu Asp Thr Ala Val Tyr Tyr Cys
                85                  90                  95

Ser Ser Tyr Asp Thr Arg Trp Gly Trp Phe Asp Pro Trp Gly Glu Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr

355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys